(19) Europäisches Patentamt
European Patent Office
Office européen des brevets

(11) EP 4 113 118 A1

(12) EUROPEAN PATENT APPLICATION
published in accordance with Art. 153(4) EPC

(43) Date of publication:
04.01.2023 Bulletin 2023/01

(21) Application number: 21759830.9

(22) Date of filing: 01.03.2021

(51) International Patent Classification (IPC):
*G01N 33/53* (2006.01) *G01N 33/566* (2006.01)

(52) Cooperative Patent Classification (CPC):
G01N 33/53; G01N 33/566

(86) International application number:
PCT/JP2021/007726

(87) International publication number:
WO 2021/172591 (02.09.2021 Gazette 2021/35)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 27.02.2020 JP 2020031457
23.09.2020 JP 2020159008

(71) Applicant: TearExo Inc.
Nada-ku
Kobe-shi, Hyogo 657-8501 (JP)

(72) Inventors:
- TAKEUCHI, Toshifumi
Kobe-shi, Hyogo 657-8501 (JP)
- SUNAYAMA, Hirobumi
Kobe-shi, Hyogo 657-8501 (JP)
- TAKANO, Eri
Kobe-shi, Hyogo 657-8501 (JP)

(74) Representative: Engelhard, Markus
Boehmert & Boehmert
Anwaltspartnerschaft mbB
Pettenkoferstrasse 22
80336 München (DE)

# (54) NANOPARTICLES FOR SENSING USE AND PRODUCTION METHOD FOR SAME

(57) The purpose of the present invention is to provide: an artificial bio sensor that is easy to produce and that exhibits superior specificity; and a method for producing same. Nanoparticles according to the present invention for sensing use comprise a molecular imprint polymer having a molecular imprint space for biological matter. The molecular imprint polymer includes a structural unit derived from functional groups including an interaction group with respect to said biological matter and a signaling substance binding group different from said interaction group. The functional groups are present on the surface of the molecular imprint space. The nanoparticles are useful as an artificial biosensor that is easy to produce and exhibits superior specificity.

EP 4 113 118 A1

## Description

[Technical Field]

The present invention relates to a nanoparticle for sensing use and a manufacturing method thereof.

[Background Art]

Biological materials such as proteins are involved in many vital phenomena such as metabolism, signaling, immunity, and formation of biological structures and provide information that is beneficial in the diagnosis of a disease, etc. In order to further increase the value of utility of such biomolecules, studies for applying a material that recognizes biomolecules to a biosensor are ongoing.

Antibodies which are used as a receptor that recognizes biomolecules in a biosensor contribute significantly to the performance of the biosensor through their excellent specificity and affinity. Meanwhile, antibodies have inherent problems in terms of being vulnerable to external stimulation such as heat and pH, high cost, etc. due to being a biomolecule.

In view of the inherent problems of antibodies, application of molecular imprinting has been attempted in order to artificially acquire the specificity and affinity of antibodies. Molecular imprinting is established as a technology for molding a template of a biomolecule within a polymer. Specifically, a complex of a template biomolecule and a functional monomer is formed and copolymerized with a crosslinker, and then the template is removed to obtain a molecularly imprinted polymer (MIP) with a binding cavity (molecularly imprinted cavity) that is complementary to the template. Various artificial biosensor substrates with a film of such as an MIP formed on the substrate have been reported.

For example, Non Patent Literature 1 reports an artificial biosensor substrate that enables improved detection sensitivity of a biomarker bound to a molecularly imprinted cavity. With such an artificial biosensor substrate, use of an antibody is no longer required for the initial binding by creating a molecularly imprinted cavity. In addition, such an artificial biosensor substrate improves the sensitivity for detecting and quantifying a low concentration of biomolecules in a complex sample, quickly without a label at a high sensitivity, by replacing a secondary labeling antibody with a biosensor that is dependent on capacitance.

Non Patent Literature 2 reports an artificial biosensor substrate, which enables improvement in the specificity and affinity of a molecularly imprinted cavity and reading out binding information from a change in fluorescence. Such an artificial biosensor substrate introduces two types of functional monomers having reversible bonds that are different from each other (disulfide group and oxyimino group) via a covalent bond to a template $\alpha$-fetoprotein (AFP) and forms a film of MIP by molecular imprinting, then cleaves the reversible bonds and removes the AFP, so that two types of functional groups (thiol group and oxyamino group) from the reversible bonds are localized within a molecularly imprinted cavity. The specificity and affinity of the AFP to the molecularly imprinted cavity are improved by introducing a group that interacts with AFP to one of the two types of functional groups, and a fluorescent reporter molecule is introduced to the other functional group to read out information on the binding of AFP to the molecularly imprinted cavity through a change in fluorescence.

[Citation List]

[Non Patent Literature]

[NPL 1] Journal of Visualized Experiments, 132, e57208, doi: 10.3791/57208 (2018)
[NPL 2] Angewandte Chemie International Edition., 55, 13023-13027 (2016)

[Summary of Invention]

[Technical Problem]

While studies are conducted to improve the performance of artificial biosensor substrates with a film of MIP formed thereon as described above, there is still room for further improvement.

The primary objective of the technology in Non Patent Literature 1 is to replace an antibody with a molecularly imprinted cavity, and improve the sensitivity of detection of recognized molecules. However, the recognition ability of a molecularly imprinted cavity is merely achieved based on the complementary shape of the surface of a target molecule. The specificity and affinity thereof are far from satisfactory.

The technology of Non Patent Literature 2 adds a group that interacts with a target molecule within a molecularly imprinted cavity to improve the affinity of the molecularly imprinted cavity and the target molecule by utilizing not only the shape of the cavity, but also the interaction with the group that interacts with the target molecule. Further, non-specific adsorption outside of the cavity is reduced by a molecular imprinting protocol for localizing a group that interacts with the target molecule within the molecularly imprinted cavity. However, in exchange for achieving such an excellent performance, a special process for covalently binding two types of functional monomers in advance to a template is required, which makes the manufacturing method complex.

The inventor attempted to simplify the manufacturing method by eliminating the special process described above in the technology of Non Patent Literature 2. Specifically, the inventor attempted to use a functional monomer having a functional group (group that interacts with a template) that interacts with a template biomolecule in a non-covalent bond form (non-covalent bond or similarly weak covalent bond) to cause adsorption of the functional monomer to the template surface in a polymerization system and localization of the group that interacts with the template in a molecularly imprinted cavity. This was designed so that the functional monomer has both a group that interacts with a template and a signal substance binding group in order to cause localization of a group that introduces a fluorescent reporter molecule (signal substance binding group) in the molecularly imprinted cavity. However, the inventor was confronted with a problem of poor specificity with the resulting artificial biosensor substrate, even after using such a manufacturing method.

In this regard, the objective of the present invention is to provide an artificial biosensor with excellent specificity while being easy to manufacture, and a manufacturing method thereof.

[Solution to Problem]

Diligent studies by the inventor resulted in the discovery that an artificial biosensor with excellent specificity is obtained, despite using a simple manufacturing method without a special processing of a template, by using a monomer having both a group that interacts with a target molecule and a signal substance binding group and forming MIPs in a particulate form. The present invention was completed by conducting additional studies based on this finding.

Specifically, the present invention provides inventions of the embodiments described below.

Item 1.

A nanoparticle for sensing use comprising a molecularly imprinted polymer having a molecularly imprinted cavity for a biological material,

wherein the molecularly imprinted polymer comprises a constituent unit derived from a functional monomer having a functional group comprising a group that interacts with the biological material and a signal substance binding group that is different from the group that interacts with the biological material, and
wherein the functional group is present on a surface of the molecularly imprinted cavity.

Item 2.

The nanoparticle for sensing use of item 1, wherein the functional group is represented by formula (1):

[Chemical Formula 1] $-R^2-L^1-R^1$ (1)

wherein $R^1$ represents the group that interacts with the biological material, $R^2$ represents a linking group comprising the signal substance binding group, and $L^1$ represents a direct bond or a linking group.

Item 3.

The nanoparticle for sensing use of item 1 or 2, wherein the group that interacts with the biological material is selected from the group consisting of a substituted or unsubstituted amino group, a substituted or unsubstituted aromatic group, an amidino group, a guanidino group, a carboxyl group, a sulfo group, a boronyl group, and a ligand of the biomolecule.

Item 4.

The nanoparticle for sensing use of any of items 1 to 3, wherein the signal substance binding group is selected from the group consisting of an amino group, a carboxyl group, a thiol group, a disulfide group-containing group, an

aminooxy group, an aldehyde group, and a hydroxyl group.

Item 5.

The nanoparticle for sensing use of any of items 1 to 4, wherein the functional group is represented by formula (11) or (12):

[Chemical Formula 2]

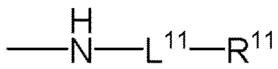

(11)

wherein $R^{11}$ represents a carboxylaryl group or a sulfoaryl group, and $L^{11}$ represents an alkylene group with 1 to 4 carbons, or

[Chemical Formula 3]

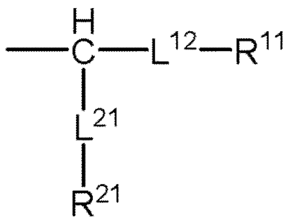

(12)

wherein $R^{11}$ represents a carboxylaryl group or a sulfoaryl group, $L^{12}$ represents a divalent linking group, $R^{21}$ represents an amino group, a carboxyl group, a thiol group, a disulfide group-containing group, an aminooxy group, an aldehyde group, or a hydroxyl group, and $L^{21}$ represents a divalent linking group.

Item 6.

The nanoparticle for sensing use of any of items 1 to 5, comprising a signal group bound to the signal substance binding group.

Item 7.

The nanoparticle for sensing use of any of items 1 to 6, wherein the molecularly imprinted polymer comprises a constituent unit derived from an N-substituted or unsubstituted (meth)acrylamide and/or a biocompatible monomer.

Item 8.

The nanoparticle for sensing use of any of items 1 to 7, wherein the biological material is selected from the group consisting of a protein, a sugar chain, a lipid, and a composite molecule of two or more thereof.

Item 9.

The nanoparticle for sensing use of any of items 1 to 8, wherein the biological material is albumin.

Item 10.

The nanoparticle for sensing use of any of items 1 to 9, which is used for the detection of the biological material. Item 11.

The nanoparticle for sensing use of any of items 1 to 9, wherein the biological material is a substance adhering to or bound to a biological membrane of a membrane structure, a molecule corresponding to an exposed partial structure of the biological membrane, or a fragment of a specific biomolecule, and is used for the detection of the membrane structure or the specific biomolecule via capture of the biomolecule by the group that interacts with the biological material.

Item 12.

The nanoparticle for sensing use of item 11, wherein the membrane structure is a cell, a virus, an extracellular vesicle, or an organelle, and the specific biomolecule is an antibody.

Item 13.

The nanoparticle for sensing use of any of items 1 to 12, wherein the biological material is a biological material derived from an animal or plant, which falls under haram, and is used to verify halal.

Item 14.

A substrate for sensing use, comprising a substrate, and the nanoparticle for sensing use of any of items 1 to 13 immobilized on the substrate.

Item 15.

A reagent for sensing use, comprising the nanoparticle for sensing use of any of items 1 to 13.

Item 16.

A method of manufacturing a nanoparticle for sensing use, comprising:

step 1 of synthesizing a molecularly imprinted polymer by polymerizing monomer components comprising a functional monomer having a functional group comprising a group that interacts with a biological material and a signal substance binding group that is different from the group that interacts with the biological material by using the biological material as a template; and
step 2 of removing the template from the molecularly imprinted polymer.

Item 17.

The method of manufacturing a nanoparticle for sensing use of item 16, comprising, after step 2, step 3 of binding a signal substance to the signal substance binding group in the molecularly imprinted polymer.

[Advantageous Effects of Invention]

The present invention provides an artificial biosensor with excellent specificity while being easy to manufacture, and a manufacturing method thereof.

[Brief Description of Drawings]

[Figure 1] Figure **1** schematically shows an example of the nanoparticle for sensing use of the invention.
[Figure 2] Figure **2** schematically shows another example of the nanoparticle for sensing use of the invention.
[Figure 3] Figure **3** schematically shows an example of an embodiment of use of the nanoparticle for sensing use of the invention.
[Figure 4] Figure **4** schematically shows step 1 in an example of the method of manufacturing a nanoparticle for sensing use of the invention.
[Figure 5] Figure **5** schematically shows steps 2 and 3 in an example of the method of manufacturing a nanoparticle for sensing use of the invention.
[Figure 6] Figure **6** schematically shows an example of the substrate for sensing use of the invention.
[Figure 7] Figure **7** schematically shows another example of the substrate for sensing use of the invention.
[Figure 8] Figure **8** shows results of a protein adsorption test according to Example 2 (corresponding to the substrate for sensing use in Figure **7**), obtained in Test Example 5.
[Figure 9] Figure **9** shows results of a protein adsorption test according to Comparative Example 2 (the comparative substrate for sensing use provided with an MIP film), obtained in Test Example 5.
[Figure 10] Figure **10** shows the correlation between the HSA concentration according to BCP and the relative

fluorescence intensity based on HSA bonds measured in a substrate for sensing use, obtained in Test Example 6.

[Figure 11] Figure **11** shows the correlation between the obtained relative fluorescence intensity and the PSA concentration for a substrate for sensing use with the fluorescent group introduced MIP-NGs of Example 4 immobilized thereon, obtained in Test Example 7.

[Figure 12] Figure **12** shows results of a protein adsorption test using a substrate for sensing use (corresponding to the substrate for sensing use in Figure **7**) with the fluorescent group introduced MIP-NGs of Example 4 immobilized thereon, obtained in Test Example 7.

[Figure 13] Figure **13** shows results of a protein adsorption test using a substrate for sensing use with the fluorescent group introduced NIP-NGs of Comparative Example 3 immobilized thereon, obtained in Test Example 7.

[Figure 14] Figure **14** shows results of a PSA adsorption test in a PSA contaminated beef extract sample using a substrate for sensing use (corresponding to the substrate for sensing use in Figure **7**) with the fluorescent group introduced MIP-NGs of Example 4 immobilized thereon, obtained in Test Example 7.

[Figure 15] Figure **15** shows results of a fluorescence response test in a pork extract sample contaminated-beef extract sample using a substrate for sensing use (corresponding to the substrate for sensing use in Figure 7) with the fluorescent group introduced MIP-NGs of Example 4 immobilized thereon, obtained in Test Example 7.

[Figure 16] Figure **16** shows the change in fluorescence spectrum ($\lambda ex$ = 647 nm) upon addition of PSA with respect to non-immobilized (free) particles of the fluorescent group introduced MIP-NGs of Example 4, obtained in Test Example 8.

[Figure 17] Figure **17** is an experiment on selectivity of non-immobilized (free) particles of the fluorescent group introduced MIP-NGs of Example 4 to PSA (sensing target protein) or transferrin (reference protein), obtained in Test Example 8, and shows the change in relative fluorescence intensity of 667 nm upon addition of each protein at a given concentration.

[Figure 18] Figure **18** shows the change in relative fluorescence intensity upon addition of an Fc domain fragment to an Fc domain sensing (and Fc domain capture mediated IgG sensing) substrate, obtained in Test Example 9.

[Figure 19] Figure **19** shows the change in relative fluorescence intensity upon addition of an Fc domain fragment, whole IgG, or lysosome to an Fc domain sensing (and Fc domain capture mediated IgG sensing) substrate, obtained in Test Example 9.

[Figure 20] Figure **20** shows the relationship between the relative fluorescence intensity and exosome concentration in an HER2 capture mediated exosome substrate for sensing use, obtained in Test Example 10.

[Figure 21] Figure **21** shows the change in relative fluorescence intensity upon addition of an exosome derived from an HER2 overexpressing breast cancer cell line for an HER2 capture mediated exosome substrate for sensing use and a substrate for sensing use with an anti-HSA affibody introduced instead of an anti-HER2 affibody, obtained in Test Example 10.

[Description of Embodiments]

[1. Nanoparticle for sensing use]

The nanoparticle for sensing use of the invention comprises a molecularly imprinted polymer having a molecularly imprinted cavity for a biological material, wherein the molecularly imprinted polymer comprises a constituent unit derived from a functional monomer having a functional group comprising a group that interacts with the biological material and a signal substance binding group that is different from the group that interacts with the biological material, and wherein the functional group is present on a surface of the molecularly imprinted cavity. The nanoparticle for sensing use of the invention is described hereinafter in detail.

Figure **1** schematically shows an example of the nanoparticle for sensing use of the invention. A nanoparticle for sensing use **10** comprises a molecularly imprinted polymer **20** having a molecularly imprinted cavity **21** for a biological material, wherein the molecularly imprinted polymer **20** comprises a constituent unit derived from a functional monomer having a functional group **22** comprising a group that interacts with the biological material (schematically represented by $R^1$ in the drawing) and a signal substance binding group (included in the structure schematically represented by $R^2$ in the drawing) that is different from the group that interacts with the biological material, and wherein the functional group **22** is present on a surface of the molecularly imprinted cavity **21**. The nanoparticle for sensing use **10** has a free signal substance binding group, and can be used as a sensor for recognizing a biological material by introducing a signal group to the signal substance binding group to arrive at the form of a nanoparticle for sensing use **10a**. The nanoparticle for sensing use **10** can be customized by a user by freely selecting a signal group to be introduced to a signal substance binding group.

Figure **2** schematically shows another example of the nanoparticle for sensing use of the invention. The nanoparticle for sensing use **10a** shown in Figure **2** has a signal group **31** introduced to a signal substance binding group of the nanoparticle for sensing use **10** shown in Figure **1** in advance. For this reason, the nanoparticle for sensing use

**10a** itself can be used as a biological material recognizing sensor.

Furthermore, Figure **3** schematically shows an example of an embodiment of use of the nanoparticle for sensing use of the invention. Figure **3** shows the nanoparticle for sensing use **10a** (biological material recognizing sensor) shown in Figure 2 recognizing and capturing a biological material **90**, which is targeted for sensing use.

[1-1. Molecularly imprinted polymer]

[1-1-1. Shape]

The molecularly imprinted polymer **20** is particulate and has a molecularly imprinted cavity (recess) **21** for a biological material (biological material **90** shown in Figure **3**) targeted for sensing use. The number of imprinted cavities **21** provided in a single molecularly imprinted polymer molecule **20** may be one as illustrated or multiple (e.g., 2 to 3, preferably 2). Since the molecularly imprinted cavity **21** is formed by molecular imprinting using a biological material targeted for sensing use as a template, the cavity has a shape that is complementary to the shape of the surface of the biological material.

The particulate shape of the molecularly imprinted polymer **20** fundamentally contributes to expression of excellent specificity of the nanoparticle for sensing use **10a**. Specificity as good as the nanoparticle for sensing use of the invention cannot be expressed with a biological material recognizing sensor substrate prepared by forming a flat molecularly imprinted polymer film (MIP film) on a substrate with the same monomer composition as the molecularly imprinted polymer **20** and introducing a signal group. While the specific mechanism that enables such expression of excellent specificity to be achieved as an effect that is unique to the present invention is not certain, the following reason is conceivable as one idea. It is understood that molecularly imprinted cavities are formed non-uniformly (with large variance in the sizes of opening of molecularly imprinted cavities) due to the propensity of templates being randomly present during film formation (polymerization) using a biological material as a template, whereby the ratio of molecularly imprinted cavities that can result in non-specific adsorption (e.g., cavities without sufficient selectivity due to the size of opening being too small) with respect to all molecularly imprinted cavities would be high, whereas molecularly imprinted cavities can be formed highly uniformly (with low variance in sizes of openings of molecularly imprinted cavities) due to the propensity of a biological material template that is relatively hydrophilic to a polymer being in the vicinity of the interface between the polymer and polymerization reaction solution during polymeric particle formation (polymerization) using a biological material as a template, so that the ratio of molecularly imprinted cavities that can result in non-specific adsorption with respect to all molecularly imprinted cavities would be very low.

Furthermore, the particulate shape of the molecularly imprinted polymer **20** is not limited by the shape of a substrate as the specific shape of a biological material recognizing sensor, so that particles can be formulated as, for example, a liquid reagent for sensing use. Thus, this is also excellent in terms of versatility.

While the mean particle size of the molecularly imprinted polymer **20** varies depending on the size, use, etc. of the biological material targeted for sensing use, the mean particle size can be, for example, 5 to 500 nm in terms of volume average particle size (50% cumulative value of volume particle size distribution) measured by dynamic light scattering (DLS), or 5 to 500 nm in terms of Z-average particle size measured by DLS. More specifically, the mean particle size of the molecularly imprinted polymers 20 when administered in vivo and when used in in vitro imaging that observes inside a cell can be 5 to 100 nm in terms of volume average particle size measured by dynamic light scattering (DLS) or 5 to 100 nm in terms of Z-average particle size measured by DLS. The mean particle size of the molecularly imprinted polymers **20** when used without in vivo administration can be 5 to 500 nm in terms of volume average particle size measured by dynamic light scattering (DLS), or 5 to 500 nm in terms of Z-average particle size measured by DLS. Preferred examples of the mean particle size of the molecularly imprinted polymers **20** include 7 to 50 nm, 8 to 40 nm, 15 to 30 nm, and 20 to 25 nm in terms of volume average particle size, and 7 to 50 nm, 8 to 40 nm, 15 to 30 nm, and 20 to 25 nm in terms of Z-average particle size.

[1-1-2. Functional group disposed within a molecularly imprinted cavity]

The functional group **22** is present in the molecularly imprinted cavity **21** of the molecularly imprinted polymer **20**. The functional group **22** contributes to recognition of a biological material targeted for sensing use and provision of signal information based thereon. In the present invention, as long as the functional group **22** is present in the molecularly imprinted cavity **21**, the presence of the functional group **22** on the surface of the molecularly imprinted polymer **20** other than the molecularly imprinted cavity **21** is permitted, but the molecularly imprinted cavity **21** exerts excellent specificity by suppressing non-specific adsorption by a molecule other than a biological material targeted for sensing use due to the shape molded from a protein targeted for sensing use and the presence of the functional group **22**.

[1-1-3. Structure of a functional group]

As described above, the functional group 22 comprises a group that interacts with a biological material targeted for sensing use (schematically represented by $R^1$ in the drawing) and a signal substance binding group (included in the structure schematically represented by $R^2$ in the drawing). Furthermore, a group that interacts with a biological material and a signal substance binding group are designed as groups that are different from each other.

[1-1-3-1. Function of a group that interacts with a biological material and a signal substance binding group]

A group that interacts with a biological material is a group that can interact with a biological material targeted for sensing use and form a non-covalently formed bond. Figure **3** shows a group that interacts with a biological material ($R^1$) interacting with the surface of the biological substance **90** targeted for sensing use. Non-covalently formed bonds include non-covalent bonds and covalent bonds that are weak to the same extent as a non-covalent bond. Examples of types of non-covalent bonds include hydrogen bonds, bonds due to electrostatic interaction (ionic bonds), hydrophobic bonds, and van der Waals forces. Examples of covalent bonds that are weak to the same extent as a non-covalent bond include covalent bonds that can be cleaved just by changing the pH without adding a cleavage reagent that exhibits a strong oxidizing or reducing action, etc. such as an oxidizing agent or reducing agent. Specific examples thereof, when the biological material is a glycoprotein, include covalent bonds formed between a borate group and a sugar group of a glycoprotein. This facilitates the removal of a biological material used as a template after the synthesis of molecularly imprinted polymer particles in molecular imprinting.

A group that interacts with a biological material is at a position corresponding to the specific amino acid residue described above and/or specific hydrophobic region described above in the biological material on the surface of the molecularly imprinted cavity **21** with a shape that is complementary to the surface shape of the protein. Thus, the molecularly imprinted polymer **20** has an excellent ability to recognize the biological material due to the molecularly imprinted cavity **21** having a shape that is unique to the biological material and a group that interacts with the biological material in the molecularly imprinted cavity **21** being at a position that is unique to the biological material.

A signal substance binding group is a group capable of binding to a signal substance. The signal group **31** is introduced, in the same manner as the nanoparticle for sensing use **10a**, by a signal substance binding group binding to a signal substance. Since the signal group **31** is affected by a change in the environment from a biological material targeted for sensing use non-covalently binding to a group that interacts with a biological material, this results in a change in signals before and after binding to the biological material. Figure **3** shows the signal group **31** exposed in Figure **2** (before recognition of the biological material **90**) subjected to a change in the environment from the biological material **90** targeted for sensing use non-covalently binding to the group that interacts with the biological material ($R^1$). Specifically, the nanoparticle for sensing use **10a** can read out information on binding of a biological material targeted for sensing use from a change in signals.

A change in the environment by which the signal group **31** is affected due to an interaction with a biological material targeted for sensing use is not particularly limited, as long as the change results in a change in signals due to approaching of the biological material targeted for sensing use. Examples of the change in environment include: (i) an electron of the signal group **31** is pulled towards a polar group of a biological material targeted for sensing use due to the polar group approaching the vicinity of the signal group **31**; (ii) a polar group of a biological substance targeted for sensing use approaches the vicinity of the signal group **31** and eliminates a water molecule that was present in the vicinity of the signal group **31** to release an electron of the signal group **31** from being pulled towards the water molecule that was in the vicinity; (iii) a polar group of a biological substance targeted for sensing use approaches the vicinity of the signal group **31** to transfer energy from the signal group **31** to the biological material targeted for sensing use that is bound; (iv) a polar group of a biological substance targeted for sensing use approaches the vicinity of the signal group **31** and blocks the signal group **31**, such that excitation energy can no longer reach the signal group **31**; etc.

[1-1-3-2. Positional relationship between a group that interacts with a biological material and a signal substance binding group]

In the illustrated embodiment, a group that interacts with a biological material is positioned on the tip side of the functional group **22**, and a signal substance binding group is positioned on the base end side of the functional group **22.** Meanwhile, the positional relationship between a group that interacts with a biological material and a signal substance binding group is not limited thereto in the functional group **22**, as long as the biological material targeted for sensing use can non-covalently bind to the group that interacts with the biological material and the signal group **31** is affected by a change in environment upon non-covalent binding. For example, the positional relationship between a group that interacts with a biological material and a signal substance binding group may be reversed from the illustrated embodiment. However, it is preferable that a group that interacts with a biological material is positioned on the tip side of the functional

group **22**, and a signal substance binding group is positioned on the base end side of the functional group **22** as illustrated, from the viewpoint of acquiring a higher recognition ability by facilitating a non-covalent bond between the biological material and the group that interacts with a biological material and/or the viewpoint of acquiring a change in signals at a higher sensitivity by ensuring the environment of the signal group **31** to change upon a non-covalent bond of the biological material with the group that interacts with the biological material.

[1-1-3-3. Specific example of a functional group]

(General formulas)

A preferred example of a functional group is represented by formula (1).

[Chemical Formula 4] $-R^2-L^1-R^1$ (1)

wherein $R^1$ represents a group that interacts with a biological material, $R^2$ represents a linking group comprising a signal substance binding group, and $L^1$ represents a direct bond or a linking group.

(Group $R^1$ that interacts with a biological material)

Group $R^1$ that interacts with a biological material is designed in accordance with the state of the surface of the biological material targeted for sensing use. Examples of the group $R^1$ that interacts with a biological material include groups that can form one or more bonds selected from the group consisting of a hydrogen bond, a bond due to electrostatic interaction (ionic bond), a hydrophobic bond, and a van der Waals bond by an interaction with a specific structure constituting the biological material. Specific examples of the group $R^1$ that interacts with a biological material include [1] a substituted or unsubstituted amino group, [2] a substituted or unsubstituted aromatic group, [3] an amidino group ($-C(=NH)NH_2$), [4] a guanidino group ($-NHC(=NH)NH_2$), [5] a carboxyl group, [6] a sulfo group, [7] a boronyl group ($-B(OH)_2$), and/or, [8] a ligand. Examples of the aromatic group in [2] a substituted or unsubstituted aromatic group include [21] a substituted or unsubstituted nitrogen-containing aromatic group and [22] a substituted or unsubstituted nitrogen-free aromatic group, as well as [25] a substituted or unsubstituted aryl group ([251] a nitrogen-containing aryl group and [252] a nitrogen-free aryl group) and [26] a substituted or unsubstituted arylene group ([261] a nitrogen-containing arylene group and [262] a nitrogen-free arylene group). Group $R^1$ that interacts with a biological material may be a group consisting of any of these groups or a group comprising a combination of two or more selected from these groups.

Specific examples of group $R^1$ that interacts with a biological material, when the biological material is a protein, include groups that can form a hydrogen bond and/or electrostatic interaction by an interaction with a specific amino acid residue constituting the protein, groups that can form a hydrophobic bond with a specific hydrophobic region of the protein, etc. The combinations of an amino acid residue or hydrophobic region, a specific group selected as group $R^1$ that interacts with a biological material, and the type of non-covalently formed bond formed by an interaction thereof are shown in the following table.

[Table 1]

| Site of interaction of protein subjected to analysis | Group $R^1$ that interacts with a biological material | Type of non-covalently formed bond |
|---|---|---|
| Acidic amino acid residue (carboxyl group, etc.) | Basic group (substituted or unsubstituted amino group, substituted or unsubstituted nitrogen-containing aromatic group, amidino group, guanidino group, etc.) | Non-covalent bond (hydrogen bond, electrostatic interaction) |
| Basic amino acid residue (amino group, etc.) | Acidic group (carboxyl group, sulfo group, etc.) | Non-covalent bond (hydrogen bond, electrostatic interaction) |
| Aromatic amino acid residue (phenyl group, indole group, imidazoyl group, etc.) | Substituted or unsubstituted aromatic group (substituted or unsubstituted aryl group, arylene group, etc.) | Non-covalent bond (hydrophobic interaction) |

(continued)

| Site of interaction of protein subjected to analysis | Group $R^1$ that interacts with a biological material | Type of non-covalently formed bond |
|---|---|---|
| Hydrophobic region | Substituted or unsubstituted aromatic group (substituted or unsubstituted aryl group, arylene group, etc.) | Non-covalent bond (hydrophobic interaction) |
| Sugar group (cis-diol group) | Boronyl group | Weak covalent bond (cis-diol boronic ester group) |

Examples of the substituent in [1] a substituted amino group include linear or branched alkyl groups and substituted or unsubstituted aralkyl groups with 1 to 8 carbons; alkenyl groups with 4 to 6 carbons constituting a cyclic secondary amino group; substituted or unsubstituted aryl groups; etc. Specific examples of the substituted or unsubstituted aryl group include the groups mentioned as [251] a substituted or unsubstituted nitrogen-containing aryl group and [252] a substituted or unsubstituted nitrogen-free aryl group described below. Specific examples of [1] a substituted or unsubstituted amino group include amino groups (unsubstituted); secondary amino groups such as N-methylamino group, N-ethylamino group, N-n-propylamino group, N-isopropylamino group, N-n-butylamino group, N-isobutylamino group, N-tert-butylamino group, N-benzylamino group, N-phenylamino group, N-mesylamino group, and N-tosylamino group; secondary amino groups such as N,N-dimethylamino group, N,N-diethylamino group, N,N-dibenzylamino group, N-ethyl-N-methylamino group, N,N-di-n-propylamino group, N,N-diisopropylamino group, N,N-diphenylamino group, N-methyl-N-phenylamino group, N-methyl-N-benzylamino group, and N-mesyl-N-methylamino group; cyclic secondary amino groups such as piperidyl group and pyrrolidyl group; etc.

Examples of the nitrogen-containing aryl group in [251] a substituted or unsubstituted nitrogen-containing aryl group among [2] a substituted or unsubstituted aromatic group include nitrogen-containing aryl groups with 2 to 12 carbons. Examples of the substituent in a substituted nitrogen-containing aryl group include linear or branched alkyl groups with 1 to 8 carbons, etc. Specific examples of [251] a substituted or unsubstituted nitrogen-containing aryl group include pyridyl groups, pyrimidyl groups, pyridazyl groups, pyrazyl groups, imidazolyl groups, triazolyl groups, methylpyridyl groups (2-methylpyridyl group, 3-methylpyridyl group, 4-methylpyridyl group, etc.), dimethylpyridyl groups (2,6-dimethylpyridyl group, etc.), methylethylpyridyl groups (2-methyl-6-ethylpyridyl group, etc.), methylimidazolyl groups (1-methylimidazolyl group, etc.), dimethylimidazolyl groups (1,2-dimethylimidazolyl group, etc.), ethylimidazolyl groups (1-ethylimidazolyl group, etc.), propylimidazolyl groups (1-n-propylimidazolyl group, etc.), butylimidazolyl groups (1-n-butylimidazolyl group, etc.), pentylimidazolyl groups (1-n-pentylimidazolyl group, etc.), hexylimidazolyl groups (1-n-hexylimidazolyl group, etc.), etc.

Examples of the nitrogen-free aryl group in [252] a substituted or unsubstituted nitrogen-free aryl among [2] a substituted or unsubstituted aromatic group include phenyl groups, naphthyl groups (1-naphthyl group, 2-naphthyl group, etc.), etc. with 6 to 16 carbons. Examples of the substituent in a substituted aryl group include linear or branched alkyl groups, nitro groups, halogen groups (fluoro group, chloro group, bromo group, etc.), etc. with 1 to 8 carbons. Specific examples of [252] a substituted or unsubstituted aryl group include phenyl groups, naphthyl groups, tolyl groups (o-tolyl group, m-tolyl group, p-tolyl group, etc.), ethylphenyl groups (4-ethylphenyl group, 3-ethylphenyl group, 2-ethylphenyl group, etc.), propylphenyl groups (4-n-propylphenyl group, etc.), isopropylphenyl groups (4-isopropylphenyl group, 2-isopropylphenyl group, etc.), butylphenyl groups (4-n-butylphenyl group, 4-isobutylphenyl group, 4-t-butylphenyl group, 3-t-butylphenyl group, 2-t-butylphenyl group, etc.), pentylphenyl groups (4-n-pentylphenyl group, 4-isopentylphenyl group, 2-neopentylphenyl group, 4-t-pentylphenyl group, etc.), hexylphenyl groups (4-n-hexylphenyl group, etc.), 4-(2-ethylbutyl)phenyl groups, 4-n-heptylphenyl groups, 4-n-octylphenyl groups, 4-(2-ethylhexyl)phenyl groups, 4-t-octylphenyl groups, 4-ethyl-1-naphthyl groups, 6-n-butyl-2-naphthyl groups, dimethylphenyl groups (2,4-dimethylphenyl group, 2,5-dimethylphenyl group, 3,4-dimethylphenyl group, 3,5-dimethylphenyl group, 2,6-dimethylphenyl group, etc.), trimethylphenyl groups (2,3,5-trimethylphenyl group, 2,3,6-trimethylphenyl group, 3,4,5-trimethylphenyl group, etc.), diethylphenyl groups (2,4-diethylphenyl group, 2,6-diethylphenyl group, etc.), 2,5-diisopropylphenyl groups, 2,6-diisobutylphenyl groups, di-t-butylphenyl groups (2,4-di-t-butylphenyl group, 2,5-di-t-butylphenyl group, etc.), 4,6-di-t-butyl-2-methylphenyl groups, 5-t-butyl-2-methylphenyl groups, 4-t-butyl-2,6-dimethylphenyl groups, fluorophenyl groups (4-fluorophenyl group, 3-fluorophenyl group, 2-fluorophenyl group, etc.), chlorophenyl groups (4-chlorophenyl group, 3-chlorophenyl group, 2-chlorophenyl group, etc.), bromophenyl groups (4-bromophenyl group, 2-bromophenyl group, etc.), chloronaphthyl groups (4-chloro-1-naphthyl group, 4-chloro-2-naphthyl group, etc.), 6-bromo-2-naphthyl groups, dichlorophenyl groups (2,3-dichlorophenyl group, 2,4-dichlorophenyl group, 2,5-dichlorophenyl group, 3,4-dichlorophenyl group, 3,5-dichlorophenyl group, etc.), 2,5-dibromophenyl groups, 2,4,6-trichlorophenyl groups, dichloronaphthyl groups (2,4-dichloro-1-naphthyl group, 1,6-dichloro-2-naphthyl group, etc.), chloromethylphenyl groups (2-chloro-4-methylphenyl

group, 2-chloro-5-methylphenyl group, 2-chloro-6-methylphenyl group, 3-chloro-4-methylphenyl group, 2-methyl-3-chlorophenyl group, 2-methyl-4-chlorophenyl group, 3-methyl-4-chlorophenyl group, etc.), trifluoromethylphenyl groups (4-trifluoromethylphenyl group, etc.), nitrophenyl groups (4-nitrophenyl group, etc.), etc.

Examples of a group comprising a combination of two or more selected from the groups of [1] to [7] described above include groups comprising a combination of [1] a substituted or unsubstituted amino group, [251] a nitrogen-containing aryl group, [252] a nitrogen-free aryl group, [3] an amidino group, [4] a guanidino group, [5] a carboxyl group, [6] a sulfo group, or [7] a boronyl group, which is a monovalent group among the groups of [1] to [7] with [26] a substituted or unsubstituted arylene group ([261] a nitrogen-containing arylene group or [262] a nitrogen-free arylene group).

[261] a nitrogen-containing arylene group among [26] a substituted or unsubstituted arylene group is a group prepared by removing one hydrogen bound to carbon constituting an aromatic ring from a group listed as [251] a substituted or unsubstituted nitrogen-containing aryl group described above. [262] a nitrogen-free arylene group among [26] a substituted or unsubstituted arylene group is a group prepared by removing one hydrogen bound to carbon constituting an aromatic ring from a group listed as [252] a substituted or unsubstituted nitrogen-free aryl group described above.

Examples of specific groups comprising a combination of two or more selected from the groups of [1] to [7] described above include an amidinoaryl group which is a combination of [3] an amidino group and [26] a substituted or unsubstituted arylene group, a carboxyaryl group which is a combination of [5] a carboxyl group and [26] a substituted or unsubstituted arylene group, a sulfoaryl group which is a combination of [6] a sulfo group and [26] a substituted or unsubstituted arylene group, a boronylaryl group which is a combination of [7] a boronyl group and [26] a substituted or unsubstituted arylene group, etc. Preferred groups among these groups are shown below.

[Chemical Formula 5]

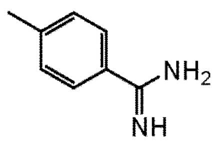
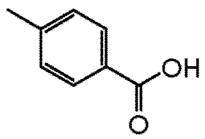
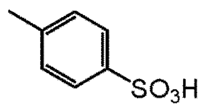

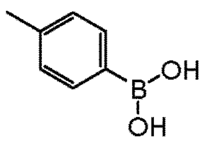
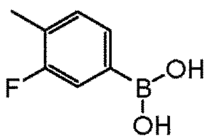
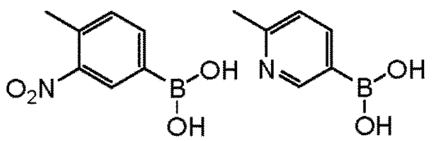

[8] a ligand is not particularly limited, as long as it is a ligand corresponding to an analyte when a biological material targeted for sensing use is the analyte. Examples thereof, when the biological material is an antigen, include antibody mimetics (affibody, etc.), nanobodies, etc. Examples thereof, when the biological material is a sugar chain, include lectin, etc. Examples thereof, when the biological material is a phospholipid, include phospholipid binding proteins (evectin 2, annexin A2, annexin V, and Tim4), etc. Examples thereof, when the biological material is an enzyme, include enzyme substrates and enzyme inhibitors.

(Linking group $R^2$ comprising a signal substrate binding group)

The signal substance binding group in linking group $R^2$ comprising a signal substrate binding group is designed in accordance with a binding group (binding group **32** in Figure **5** described below) of a signal substance (signal substance **30** in Figure **5** described below) from which the signal group **31** to be introduced is derived. Specific examples of the signal substance binding group include amino groups (including monovalent amino groups and divalent amino groups), carboxyl groups, thiol groups, disulfide group-containing groups (monovalent groups), aminooxy groups, aldehyde groups, and hydroxyl groups.

Linking group $R^2$ is an unbranched or branched divalent group.

If linking group $R^2$ is unbranched, a signal substance binding group itself constitutes linking group $R^2$ (i.e., linking group $R^2$ consists of a signal substance binding group). Examples of such an unbranched linking group $R^2$, i.e., a signal substance binding group, include the divalent amino group represented by formula (2).

[Chemical Formula 6] $-NR^3-$ (2)

wherein $R^3$ represents hydrogen or a linear or branched alkyl group with 1 to 8 carbons, and preferably hydrogen.

If linking group $R^2$ is branched, a signal substance binding group is a monovalent group that is present at a

branch. Examples of such a linking group $R^2$ includes the divalent group represented by formula (3).

[Chemical Formula 7]

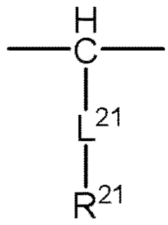

(3)

wherein $R^{21}$ represents an amino group (monovalent), a carboxyl group, a thiol group, a disulfide group-containing group (monovalent group), an aminooxy group, an aldehyde group, or a hydroxyl group, and preferably a thiol group or a disulfide group-containing group. Examples of amino groups (monovalent) include the groups listed as [1] a substituted or unsubstituted amino group, which is an example of group $R^1$ that interacts with a biological material described above.

A disulfide group-containing group (monovalent group) is represented by formula (4)

[Chemical Formula 8]

$$-S-S-R^{211} \quad (4)$$

wherein $R^{211}$ represents a substituted or unsubstituted alkyl group. Examples of the alkyl group in a substituted or unsubstituted alkyl group include linear or branched alkyl groups with 1 to 8 carbons. As the substituent in a substituted alkyl group, those skilled in the art can appropriately select any functional group, which does not interfere with an interaction between group $R^1$ that interacts with a biological material and a template biological material or a reaction between a disulfide bond and a signal substance and guarantees water solubility of a compound as a whole. Specific examples include hydroxyl groups, amino groups (specific examples include the groups listed as [1] a substituted or unsubstituted amino group, which is an example of group $R^1$ that interacts with a biological material described above), carboxy groups, alkoxy groups (e.g., an alkoxy group with 1 to 4, preferably 1 to 3, more preferably 1 to 2, and still more preferably 1 carbon), polyethylene glycol groups (examples of the number of ethylene oxide added include 1 to 6), alkyl groups (examples include alkyl group with 1 to 4 carbons), amide groups, sulfo groups, guanidino groups, amidino groups, etc. Examples of the position of a substituent in a substituted alkyl group include the end of a branch of linking group $R^2$.

In formula (3), $L^{21}$ represents a direct bond or a linking group. Examples of said linking group include alkylene groups, carbonyl groups, ester bonds (-COO- and -OCO-), amide bonds (-CONH- and -NHCO-), and groups consisting of any combination thereof. The linking group is preferably an alkylene group. Examples of the alkylene group include an alkylene group with 1 to 4, preferably 1 to 3, more preferably 1 to 2, and still more preferably 1 carbon.

(Direct bond or linking group $L^1$)

A direct bond or linking group $L^1$ is interposed between group $R^1$ that interacts with a biological material and linking group $R^2$ comprising a signal substance binding group. When the nanoparticle for sensing use **10a** recognizes the biological material **90** targeted for sensing use, $L^1$ is more preferably a linking group from the viewpoint of more effectively suppressing interference to the signal group **31** introduced to a signal substance binding group of linking group $R^2$ and a bond of a biological material to group $R^1$ that interacts with the biological material.

Examples of such a linking group include alkylene groups, carbonyl groups, ester bonds (-COO- and -OCO-), amide bonds (-CONH- and -NHCO-), and groups consisting of any combination thereof. Examples of the alkylene groups include an alkylene groups with 1 to 4 carbons.

(More specific examples)

Examples of specific structures of the functional group **22** include formulas (11) and (12).

[Chemical Formula 9]

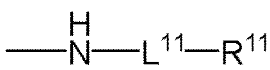

(11)

wherein $R^{11}$ corresponds to the group $R^1$ that interacts with a biological material of formula (1), the divalent amino group (-NH-) corresponds to the linking group $R^2$ comprising a signal substance binding group (this itself constitutes a signal substance binding group) of formula (1), and $L^{11}$ corresponds to the direct bond or linking group $L^1$ of formula (1).

In formula (11), $R^{11}$ indicates a carboxyaryl group or a sulfoaryl group, which is specifically the same as a group described above as a carboxyaryl group that is a combination of [5] a carboxyl group and [26] a substituted or unsubstituted arylene group, or a sulfoaryl group that is a combination of [6] a sulfo group and [26] a substituted or unsubstituted arylene group. In formula (11), $L^{11}$ represents an alkylene group with 1 to 4 carbons. A change in signals can be obtained with higher sensitivity, when the nanoparticle for sensing use **10a** recognizes the biological material **90** targeted for sensing use, with a closer distance between $R^{11}$, which is group $R^1$ that interacts with a biological material, and divalent amino group (-NH-), which is a signal substance binding group. From this viewpoint, $L^{11}$ is an alkylene group with preferably 1 to 3, more preferably 1 to 2, and still more preferably 1 carbon.

[Chemical Formula 10]

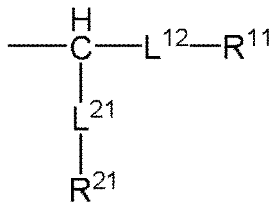

(12)

wherein $R^{11}$ corresponds to the group $R^1$ that interacts with a biological material of formula (1), the divalent group (-CH($L^{21}$-$R^{21}$)-) corresponds to the linking group $R^2$ comprising a signal substance binding group of formula (1), and $L^{12}$ corresponds to the direct bond or linking group $L^1$ of formula (1).

In formula (12), $R^{11}$ indicates a carboxyaryl group or a sulfoaryl group, which is specifically the same as a group described above as a carboxyaryl group that is a combination of [5] a carboxyl group and [26] a substituted or unsubstituted arylene group, or a sulfoaryl group that is a combination of [6] a sulfo group and [26] a substituted or unsubstituted arylene group. In formula (12), the divalent group (-CH($L^{21}$-$R^{21}$)-) is the same as a group described above as formula (3). In formula (12), $L^{12}$ represents a divalent linking group. Examples of the divalent linking group include alkylene groups, carbonyl groups, ester bonds (-COO- and - OCO-), amide bonds (-CONH- and -NHCO-), and groups consisting of any combination thereof. The divalent linking group is preferably an amide bond.

Additional specific examples of groups represented by formulas (11) and (12) are represented by formulas (11a) and (11b) and formulas (12a) and (12b), respectively.

[Chemical Formula 11]

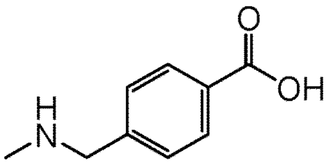

(11a)

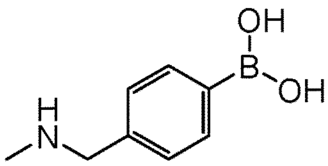

(11b)

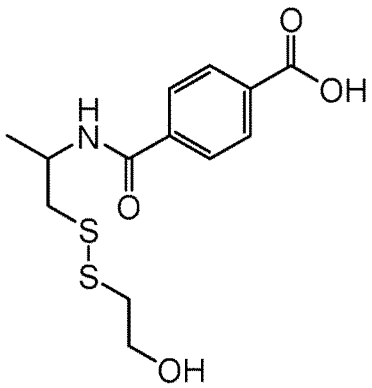

(12a)

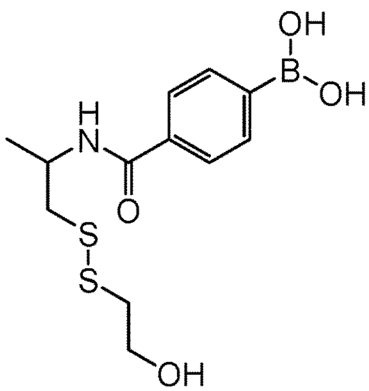

(12b)

Only one type or a plurality of types of these functional groups **22** may be present, and one or a plurality may be present per nanoparticle for sensing use. Only one type or a plurality of types of these functional groups **22** may be

present, and one or a plurality may be present per molecularly imprinted cavity.

[1-1-4. Constituent monomer of molecularly imprinted polymer]

A polymer constituting the molecularly imprinted polymer **20** comprises at least a constituent unit derived from a functional monomer having the functional group **22**. A polymer constituting the molecularly imprinted polymer **20** can comprise a constituent unit derived from a functional monomer as well as a constituent unit derived from an N-substituted or unsubstituted acrylamide and/or biocompatible monomer. Preferably, a polymer constituting the molecularly imprinted polymer **20** comprises a constituent unit derived from a functional monomer as well as a constituent unit derived from an N-substituted or unsubstituted (meth)acrylamide and biocompatible monomer. A polymer constituting the molecularly imprinted polymer **20** may comprise a constituent unit derived from a monomer that is different from a functional monomer, an N-substituted or unsubstituted acrylamide, or a biocompatible monomer.

(Functional monomer)

A functional monomer having the functional group **22** is not particularly limited, as long as it is a monomer comprising a functional group and an ethylenic unsaturated group. The functional monomer is represented by formula (5).

[Chemical Formula 12] $X\text{-}L^5\text{-}R^2\text{-}L^1\text{-}R^1$ (5)

wherein X represents an ethylenic unsaturated group, $L^5$ represents a linking group, and $-R^2\text{-}L^1\text{-}R^1$ represents a functional group. Examples of the ethylenic unsaturated group include acrylic groups and methacrylic groups. Examples of the linking group include alkylene groups, carbonyl groups, ester bonds (-COO- and -OCO-), amide bonds (-CONH- and -NHCO-), and groups consisting of any combination thereof. Preferred examples include groups with any number of alkylene groups and amide bonds bound thereto. A functional group is the same as the groups described in detail in "1-1-3. Structure of a functional group".

Specific examples of the functional monomer represented by formula (5) include functional monomers represented by formulas (51a), (52a), (51b), and (52b).

[Chemical Formula 13]

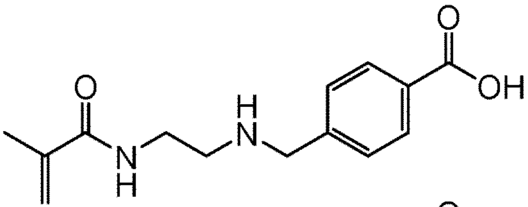

(51a)

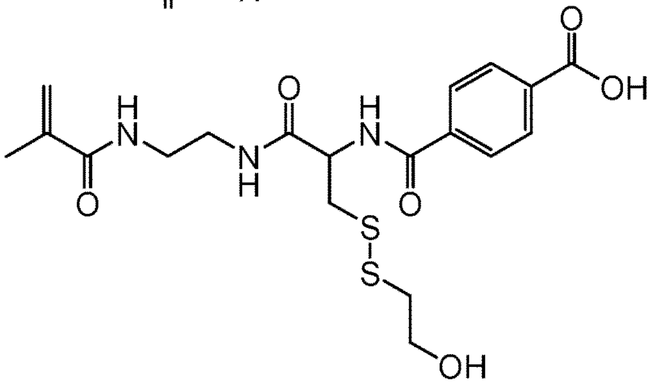

(52a)

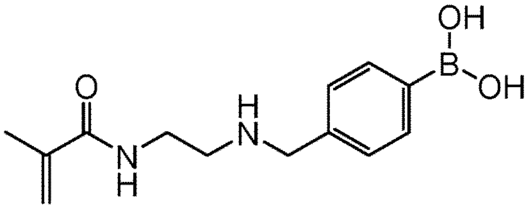

(51b)

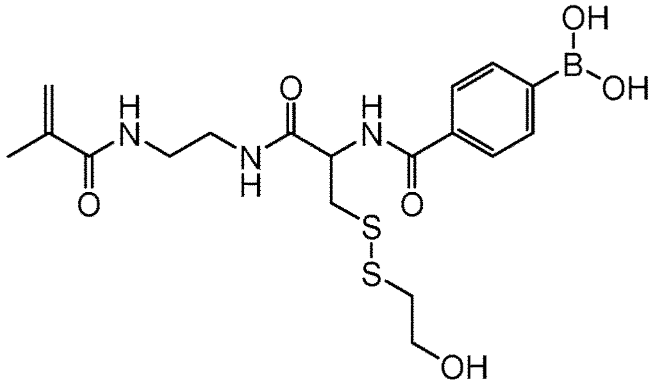

(52b)

(N-substituted or unsubstituted acrylamide)

With regard to N-substituted or unsubstituted (meth)acrylamide, N-substituted (meth)acrylamide is represented by formula (6).

[Chemical Formula 14]

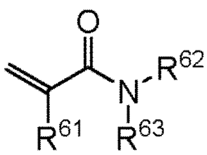

(6)

wherein $R^{61}$ represents hydrogen or a methyl group, preferably hydrogen, $R^{62}$ and $R^{63}$ each independently represent hydrogen; a linear or branched alkyl group with 1 to 6 carbons; a linear or branched hydroxyalkyl group with 1 to 6 carbons; or a linear or branched aminoalkyl group with 1 to 6 carbons, which may be the same or different from each other (with a proviso that $R^{62}$ and $R^{63}$ are not both hydrogen) ; and $R^{62}$ and $R^{63}$ may form a 5- to 7-membered saturated ring comprising an oxygen atom with a nitrogen atom which carries them.

Examples of a linear or branched alkyl group with 1 to 6 carbons include methyl groups, ethyl groups, n-propyl

groups, isopropyl groups, n-butyl groups, sec-butyl groups, tert-butyl groups, n-pentyl groups, n-hexyl groups, etc. Examples of a linear or branched hydroxyalkyl group with 1 to 6 carbons include hydroxymethyl groups, 1-hydroxyethyl groups, 2-hydroxyethyl groups, 1-hydroxy-n-propyl groups, 2-hydroxy-n-propyl groups, 3-hydroxy-n-propyl groups, 1-hydroxyisopropyl groups, 2-hydroxyisopropyl groups, 1-hydroxy-n-butyl groups, 1-hydroxy-n-pentyl groups, 1-hydroxy-n-hexyl groups, etc. Examples of a linear or branched aminoalkyl group with 1 to 6 carbons include aminomethyl groups, 1-aminoethyl groups, 2-aminoethyl groups, 1-amino-n-propyl groups, 2-amino-n-propyl groups, 3-amino-n-propyl groups, 1-aminoisopropyl groups, 2-aminoisopropyl groups, 1-amino-n-butyl groups, 1-amino-n-pentyl groups, 1-amino-n-hexyl groups, etc. Examples of a 5- to 7-membered saturated ring comprising an oxygen atom with a nitrogen atom include morpholine groups, etc.

Such N-substituted or unsubstituted (meth)acrylamide is preferably N-substituted (meth)acrylamide, more preferably N-monosubstituted (meth)acrylamide, still more preferably N-alkyl(meth)acrylamide (alkyl group is the linear or branched alkyl group with 1 to 6 carbons described above), even more preferably N-isopropyl(meth)acrylamide, and especially preferably N-isopropylacrylamide.

(Biocompatible monomer)

Biocompatibility refers to a property of not inducing adhesion of a biological material. Non-specific adsorption can be further suppressed by including a component derived from a polymeric biocompatible monomer constituting the molecularly imprinted polymer **20**. A biocompatible monomer is preferably a hydrophilic monomer and more preferably a zwitterionic monomer.

A zwitterionic monomer comprises both an anionic group derived from an acidic functional group (e.g., phosphoric acid group, sulfuric acid group, carboxyl group, etc.) and a cationic group derived from a basic functional group (e.g., primary amino group, secondary amino group, tertiary amino group, quaternary ammonium group, etc.) in one molecule. Examples of the types of zwitterionic monomer include phosphobetaine, sulfobetaine, carboxybetaine, etc.

Examples of phoshobetaine include molecules having a phosphorylcholine group on a side chain. Preferably, phosphobetaine is 2-methacryloyloxyethyl phoshphorylcholine (MPC), etc. Examples of sufobetaine include N,N-dimethyl-N-(3-sulfopropyl)-3'-methacryloylaminopropanaminium inner salt (SPB), N,N-dimethyl-N-(4-sulfobutyl)-3'-methacryloylaminopropanaminium inner salt (SBB), etc. Examples of carboxybetaine include N,N-dimethyl-N-(1-carboxymethyl)-2'-methacryloyloxyethanaminium inner salt (CMB), N,N-dimethyl-N-(2-carboxyethyl)-2'-methacryloyloxyethanaminium inner salt (CEB), etc.

Such biocompatible monomers are preferably phosphobetaine, more preferably a molecule having a phosphorylcholine group on a side chain, and still more preferably 2-methacryloyloxyethyl phoshphorylcholine (MPC).

[1-2. Signal group]

The signal group **31** functions to provide information on a bond between the biological material **90** targeted for sensing use and group $R^1$ that interacts with a biological material in the molecularly imprinted cavity **21**. The signal group **31** is not particularly limited, as long as a signal changes by being subjected to a change in the environment from the biological material **90** targeted for sensing use binding to group $R^1$ that interacts with a biological material. Embodiments of changes in signals include a change in signal intensity and a change in spectrum (e.g., peak shift).

Examples of the signal group **31** include a fluorescent group, radioactive element-containing group, magnetic group, etc. A fluorescent group is preferred as the signal group **31** from the viewpoint of ease of detection, etc. A fluorescent group is preferably a group of a non-biological material system. Examples thereof include fluorescein dyes, cyanine dyes such as an indocyanine dye, fluorescent dyes such as a rhodamine dye, etc. Examples of a radioactive element-containing group include saccharides, amino acids, and nucleic acids labeled with a radioisotope such as $^{18}F$, MRI probes labeled with $^{19}F$, etc. Examples of a magnetic group include groups having a magnetic substance such as ferrichrome.

[1-3. Other structures]

As described in [1-1-2. Functional group disposed in a molecularly imprinted cavity], the presence of the functional group **22** on the surface of the molecularly imprinted polymer **20** other than the molecularly imprinted cavity **21** is permitted in the present invention, as long as the functional group **22** is present in the molecularly imprinted cavity **21**. A capping modification group may be bound to the functional group **22** that is present on the surface of the molecularly imprinted polymer **20** other than the molecularly imprinted cavity **21** in the nanoparticle for sensing use of the invention when the functional group **22** is present on the surface of the molecularly imprinted polymer **20** other than the molecularly imprinted cavity **21**.

A capping modification group is a group introduced by a compound that inhibits the function of a functional

monomer. Examples of the compound that inhibits the function of a functional monomer (capping compound) include non-signal substances, which are compounds used for the suppression of adsorption of a biological material, etc. (e.g., compound having an oligo ethylene glycol chain), compounds having a sugar chain structure, etc. Examples of binding positions of a capping modification group include group $R^1$ that interacts with a biological material and/or signal substance binding group of the functional group **22**. Since such a capping modification group blocks the functional group **22** from the outside, a higher effect of suppressing non-specific bonds and background signal can be achieved.

[1-4. Biological material targeted for sensing use]

The target of sensing of the nanoparticle for sensing use of the invention is a biological material. As used herein, a biological material refers to a molecular species classified as a molecule that can be a base material constituting an organism. A biological material is generally a molecule having a biological function or biological significance, comprising carbon and hydrogen, as well as nitrogen, oxygen, phosphorous, and/or sulfur, etc. as constituent elements. Specific examples of biological materials include proteins (including peptides; the same applies hereinafter), sugar chains, lipids (e.g., phospholipids, etc.), composite molecules of two or more thereof, composite molecules comprising a protein and a cofactor (examples thereof include coenzymes and prosthetic groups, specifically organic molecules other than proteins and metal ions), etc. In the present invention, a biological material can be a naturally occurring or artificial material. Figure **3** shows human serum albumin (HSA) as an exemplary biological material **90** targeted for sensing use for convenience, but the biological material **90** is intended to be any biological material targeted for sensing use by the present invention.

Preferred examples of biological materials include proteins. In the following descriptions, proteins encompass normal proteins without any sugar, lipid, or cofactor, and composite proteins comprising at least one of a sugar, lipid, and cofactor (e.g., glycoproteins, lipoproteins, metal proteins, etc.) A protein may be a naturally occurring protein or artificial protein. A naturally occurring protein is a protein produced as a result of natural selection. Examples of artificial proteins include naturally occurring proteins with a part of an amino acid sequence that has been artificially altered, naturally occurring proteins with a modification group that has been artificially introduced or removed, etc.

Examples of the biological material **90** include, from the viewpoint of in vivo form, free molecules secreted in the bodily fluid (e.g., free proteins, free sugar chains, etc.), substances adhering or bound to a biological membrane of a membrane structure (e.g., cell, virus, extracellular vesicle (exosome, etc.), organelle, etc.) (e.g., membrane protein and membrane sugar chain), and molecules corresponding to an exposed partial structure of a biological membrane (e.g., phospholipid such as phosphatidylserine, etc.) The biological material **90** may also be a fragment of a specific biomolecule. Examples of such a fragment include molecules obtained by cutting out a specific region (e.g., domain) from a specific biomolecule (e.g., antibody, etc.) and molecules obtained by artificially preparing only such a region. Other examples of the biological material **90** include, from the viewpoint of the function thereof, plasma proteins, biomarkers, domains, enzymes, hormones, etc. Examples of plasma proteins include albumin, $\gamma$-globulin, fibrinogen, etc. Examples of biomarkers include renal function markers, liver function markers, inflammation markers, tumor markers, etc. More specific examples thereof include urinary albumin, aspartate aminotransferase (AST), alanine aminotransferase (ALT), transferrin, ceruloplasmin, lactate dehydrogenase (LDH), alkaline phosphatase (ALP), $\gamma$-glutamyl transpeptidase ($\gamma$-GTP), C reactive protein (CRP), $\alpha$-fetoprotein, (AFP), various antigens (e.g., carbohydrate antigen 19-9 (CA19-9), carcinoembryonic antigen (CEA), prostate-specific antigen (PSA), exosome-specific antigen (CD63, CD9, CD81, CD37, CD53, CD82, CD13, CD11, CD86, ICAM-1, Rab5, Annexin V, LAMP1, etc.), etc. Examples of the biological material **90**, from the viewpoint of the organism of origin, include human derived biological materials, non-human organism derived biological materials, etc. Non-human organisms are not particularly limited. Examples thereof include any organism such as vertebrates such as fish, amphibians, reptiles, and mammals, invertebrates such as cnidarian and crustaceans, and plants such as spermatophytes/gymnosperms and algae. A non-human organism is preferably a mammal. Examples thereof include mice, rats, monkeys, dogs, cats, cows, horses, pigs, hamsters, rabbits, goats, etc. Examples of the biological material **90**, from the viewpoint of Islamic practice, include biological materials derived from animals and plants, which falls under haram.

[1-5. Application]

The nanoparticle for sensing use of the invention can be used in an application for detecting the biological materials described above. If the biological material is a substance adhering or bound to a biological membrane of a membrane structure (e.g., cell, virus, extracellular vesicle, organelle, etc.) (e.g., membrane protein or membrane sugar chain), or a molecule corresponding to an exposed partial structure of a biological membrane (e.g., phospholipid such as phosphatidylserine, etc.), or a fragment of a specific biomolecule (e.g., specific region such as a domain), the nanoparticle for sensing use of the invention can be used in an application for detecting a specific biomolecule (e.g., antibody, etc.) having a membrane structure (e.g., cell, virus, extracellular vesicle (e.g., exosome, etc.), organelle, etc.) or the

fragment (e.g., specific region such as a domain) having the biological material as a partial structure, via the biological material described above.

More specific applications of the nanoparticle for sensing use of the invention vary depending on the type of biological material. Meanwhile, if the nanoparticle for sensing use of the invention is designed for sensing use of a biomarker such as a renal function marker, liver function marker, inflammation marker, or tumor marker, the application can be for a diagnostic application based on renal function, liver function, presence/absence or extent of inflammation, presence/absence or extent of tumor, etc.

Since the nanoparticle for sensing use of the invention can identify the same species of biological materials among different species of organisms in view of its excellent specificity, a more specific application of the nanoparticle for sensing use of the invention, when the biological material is a biological material derived from an animal or plant which falls under haram, is an application for verifying halal from checking the presence/absence of contamination by a biological material derived from an animal or plant (e.g., pig serum albumin, etc.), which falls under haram, in a food product.

[2. Method of manufacturing a nanoparticle for sensing use] [2-1. Manufacturing method 1]

The present invention also provides a method of manufacturing the nanoparticle for sensing use of the invention described above in [1. Nanoparticle for sensing use]. The method of manufacturing a nanoparticle for sensing use of the invention comprises: step 1 for synthesizing a molecularly imprinted polymer by polymerizing monomer components comprising a functional monomer having a functional group comprising a group that interacts with a biological material and a signal substance binding group that is different from the group that interacts with a biological material by using the biological material as a template; and step 2 for removing the template from the molecularly imprinted polymer. The method of manufacturing a nanoparticle for sensing use of the invention can comprise, after step 2, step 3 for binding a signal substance to the signal substance binding group in the molecularly imprinted polymer. In the manufacturing method of the invention, an N-substituted or unsubstituted (meth)acrylamide and/or biocompatible monomer may be further comprised in the monomer components in step 1.

In manufacturing method 1, it is not necessary to apply a surface modification by a covalent bond (e.g., processing performed to cause localization of a group that interacts with a biological material and a signal substance binding group in a molecularly imprinted cavity), etc. to a biological material used as a template prior to step 1. Manufacturing method 1 can be applied to any of the nanoparticle for sensing uses of the invention described above in [1. Nanoparticle for sensing use], but is preferably applied when the group that interacts with a biological material is selected from the group consisting of a substituted or unsubstituted amino group, a substituted or unsubstituted aromatic group, an amidino group, a guanidino group, a carboxyl group, a sulfo group, and a boronyl group.

Figure **4** schematically shows step 1 in an example of the method of manufacturing a nanoparticle for sensing use of the invention. Further, Figure **5** schematically shows steps 2 and 3 in an example of the method of manufacturing a nanoparticle for sensing use of the invention. The method of manufacturing a nanoparticle for sensing use of the invention is described in detail hereinafter with reference to these drawings.

[2-1-1. Step 1]

As shown in Figure **4**, step 1 synthesizes a molecularly imprinted polymer by polymerizing monomer components comprising a functional monomer **M22** having the functional group **22** comprising group $R^1$ that interacts with a biological material **90'** and a signal substance binding group that is different from group $R^1$ that interacts with a biological material by using the biological material **90'** as a template. In the example of Figure **4**, a monomer component comprises the functional monomer **M22** as well as N-substituted or unsubstituted (meth)acrylamide **M23** and biocompatible monomer **M24**.

The biological material **90'** used as a template is the same species of biological material as the biological material **90** targeted for sensing use. For example, if the biological material **90** targeted for sensing use is HSA, HSA can also be used as the biological material **90'** used as a template. Thus, examples of the biological material **90'** used as a template include those mentioned above in "1-4. Biological material targeted for sensing use".

The concentration of the biological material **90'** used as a template in a polymerization reaction solution is not particularly limited. The concentration is, for example, 0.5 to 5 μmol/L, preferably 1 to 3 μmol/L, and more preferably 1.5 to 2.5 μmol/L.

The functional monomer **M22** is the same as a monomer described above in "(Functional monomer)" of "1-1-4. Constituent monomer of a molecularly imprinted polymer".

The concentration of the functional monomer **M22** in a polymerization reaction solution is not particularly limited. The concentration is, for example, 1 to 6 mmol/L, preferably 2 to 4 mmol/L, and more preferably 2.5 to 3.5 mmol/L. As the amount of the functional monomer **M22** used relative to the biological material **90'** used as the template, the amount

of the functional monomer **M22** used per 1 μmol of the biological material **90'** used as a template is preferably 0.5 to 4 mmol, more preferably 1 to 2 mmol, and still more preferably 1.3 to 1.7 mmol.

The N-substituted or unsubstituted (meth)acrylamide **M23** is the same as that described above in "(N-substituted or unsubstituted (meth)acrylamide)" of "1-1-4. Constituent monomer of a molecularly imprinted polymer".

The concentration of the N-substituted or unsubstituted (meth)acrylamide **M23** in a polymerization reaction solution is not particularly limited. The concentration is, for example, 20 to 60 mmol/L, preferably 30 to 50 mmol/L, and more preferably 35 to 40 mmol/L. As the amount of the N-substituted or unsubstituted (meth)acrylamide **M23** used relative to the biological material **90'** used as the template, the amount of the N-substituted or unsubstituted (meth)acrylamide **M23** used per 1 μmol of the biological material **90'** used as a template is preferably 5 to 40 mmol, more preferably 10 to 33 mmol, and still more preferably 15 to 20 mmol. As the amount of the N-substituted or unsubstituted (meth)acrylamide **M23** used relative to the functional monomer **M22**, the amount of the N-substituted or unsubstituted (meth) acrylamide **M23** used per 1 mol of the functional monomer **M22** is preferably 5 to 30 mol, more preferably 8 to 20 mol, and still more preferably 10 to 15 mol.

The biocompatible monomer **M24** is the same as a monomer described above in "(Biocompatible monomer)" of "1-1-4. Constituent monomer of a molecularly imprinted polymer".

The concentration of the biocompatible monomer **M24** in a polymerization reaction solution is not particularly limited. The concentration is, for example, 0.5 to 5 mmol/L, preferably 1 to 3.5 mmol/L, and more preferably 1.5 to 2.5 mmol/L. As the amount of the biocompatible monomer **M24** used relative to the biological material **90'** used as the template, the amount of the biocompatible monomer **M24** used per 1 μmol of the biological material **90'** used as a template is preferably 0.3 to 3.5 mmol, more preferably 0.5 to 2 mmol, and still more preferably 0.8 to 1.2 mmol. As the amount of the biocompatible monomer **M24** used relative to the functional monomer **M22,** the amount of the biocompatible monomer **M24** used per 1 mol of the functional monomer **M22** is preferably 0.1 to 1.5 mol, more preferably 0.2 to 1 mol, and still more preferably 0.4 to 0.8 mol.

A crosslinker, an initiator, and a solvent are included in a polymerization reaction solution when appropriate.

Examples of a crosslinker include compounds with two or more ethylenic unsaturated groups bound by a linker group. Specific examples of a crosslinker include a compound represented by formula (7).

[Chemical Formula 15] W-Z-W (7)

wherein W represents ethylenic unsaturated groups that may be the same or different from each other, and Z represents a linker group. Examples of ethylenic unsaturated groups includes acrylic groups and methacrylic groups. Examples of a linker group include alkylene groups, amino groups (-NH-), ether groups, carbonyl groups, ester bonds (-COO- and -OCO-), amide bonds ((-CONH- and -NHCO-)), sulfoxide groups (-SO-), sulfonyl groups ($-SO_2-$), and groups prepared from binding two or more thereof with 1 to 6 and preferably 2 to 6 carbons. If two or more of the groups described above bind to constitute the linker group described above, the number of bonds is preferably 5 or less or 4 or less and more preferably 3 or less or 2. More specific examples of a crosslinker include low molecular weight crosslinkers such as N,N'-methylenebisacrylamide and ethylene glycol dimethacrylate. A crosslinker is preferably N,N'-methylenebisacrylamide.

The concentration of a crosslinker in a polymerization reaction solution is not particularly limited. The concentration is, for example, 0.5 to 5 mmol/L, preferably 1 to 3.5 mmol/L, and more preferably 1.5 to 2.5 mmol/L.

Examples of an initiator include peroxides such as ammonium persulfate and potassium persulfate and azo polymerization initiators such as azobisisobutyronitrile and 2,2'-azobis(2-methylpropionamidine) dihydrochloride. An initiator is preferably an azo polymerization initiator and more preferably 2,2'-azobis(2-methylpropionamidine) dihydrochloride.

The concentration of an initiator in a polymerization reaction solution is not particularly limited. The concentration is, for example, 1 to 15 mmol/L, preferably 3 to 12 mmol/L, and more preferably 5 to 10 mmol/L.

As a solvent, an aqueous solvent such as a buffer is used. A solvent is preferably a phosphate buffer. A solvent preferably comprises NaCl. The concentration of NaCl is, for example, 100 to 200 mM, preferably 120 to 160 mM. As the pH of a solvent, a pH that does not result in denaturation of the template 90' is selected as appropriate. The pH is, for example, 6 to 8, preferably 6.8 to 7.8, and more preferably 7.2 to 7.6.

In the polymerization reaction system of step 1, a functional monomer **M22**-template **90'** complex is formed by specific interaction of the functional monomer **M22** among the monomer components with a given site of the template **90'** via group $R^1$ that interacts with a template, as shown in Figure **4.** With progression in a polymerization reaction while such a complex is formed, a molecularly imprinted cavity (recess) that is complementary to the shape of the surface of the template **90'** can be formed, and the functional group **22** can be made to be present on the surface of the molecularly imprinted cavity (recess). Further, a molecularly imprinted cavity is efficiently formed because the template **90'** is facilitated to be in the vicinity of the polymer interface.

A specific polymerization method for obtaining molecularly imprinted polymer nanoparticles is, for example,

emulsifier-free precipitation polymerization, dispersion polymerization, emulsion polymerization, seed emulsion polymerization, etc. and is preferably emulsifier-free precipitation polymerization.

In step 1, it is preferable to subject a polymerization reaction solution to a low temperature condition of 1 to 15°C, preferably 1 to 10°C, and more preferably 2 to 6°C, in order to sufficiently form a functional monomer **M22**-template **90'** complex, prior to subjecting the solution to a temperature condition for progressing a polymerization reaction. Examples of the period of time during which the solution is subjected to a low temperature condition is 20 to 30 hours. The temperature condition for progressing a polymerization reaction is, for example, 40 to 76°C, preferably 50 to 74°C, and more preferably 60 to 72°C. Examples of the period of time a solution is subjected to a temperature condition for progressing a polymerization reaction include 10 to 15 hours. Even if a polymerization reaction is performed under heating conditions, a template retains its shape to the extent that heating does not result in denaturation that would eliminate the specificity of a molecularly imprinted cavity, so that specificity of a molecularly imprinted cavity can be efficiently acquired.

In this manner, the molecularly imprinted polymer **20** portion is synthesized to obtain a complex of the molecularly imprinted polymer **20** and template **90'**. The complex of the molecularly imprinted polymer **20** and template **90'** is formed by a non-covalently formed bond (including non-covalent bonds and covalent bonds that are week to the extent of a non-covalent bond) between the group $R^1$ that interacts with a template and template **90'**.

[2-1-2. Step 2]

Step 2 removes the template **90'** from the complex of the molecularly imprinted polymer **20** and template **90'**, as shown in Figure **5**. Since a bond between the group $R^1$ that interacts with the template and template **90'** is a non-covalently formed weak bond in the complex of the molecularly imprinted polymer **20** and the template **90'**, the template **90'** can be readily detached and removed without adding a cleaving reagent exhibiting a strong oxidating or reducing action, etc. such as an oxidant or reducing agent.

In this regard, when a functional monomer having a reversible bond (covalent bond of a disulfide group, oxyimino group, etc.) is introduced to a template in advance, and a nanoparticle is formed by molecular imprinting polymerization, then the reversible bond is cleaved with a cleaving reagent to remove the template to cause localization of a functional group derived from the reversible bond (group that functions as a signal substance binding group and/or group that interacts with a template such as a thiol group or oxyamino group) within a molecularly imprinted cavity, the template and molecularly imprinted cavity are covalently bound, so that the template cannot be readily removed even with a cleaving reagent, unlike the manufacturing method of the invention. In such a case, to ensure removal of the template, it is necessarily to not only introduce a functional monomer to the template in advance, but also to have such a template carried by a carrier such as silica, and remove the template with the carrier when removing the template after molecular imprinting polymerization. Meanwhile, the manufacturing method of the invention does not require a step of introducing a functional monomer to the template **90'** or a step of having the template **90'** carried by a carrier prior to molecular imprinting polymerization, in addition to removal of the template **90'** from a complex of the molecularly imprinted polymer **20** and template **90'** being easy. Thus, the manufacturing method of the invention is also excellent in terms of ease of manufacture.

Removal of the template **90'** can be appropriately determined by those skilled in the art based on the form of bond between group $R^1$ that interacts with the template and template **90'**. The template **90'** can be preferably removed by subjecting the reaction solution that has undergone step 1 to chromatography (preferably, anion exchange chromatography), and also using the mobile phase used at this time as an eluate of template molecule. The removed template **90'** is separated from the molecularly imprinted polymer **20** by chromatography, and the molecularly imprinted polymer **20** is purified.

A mobile phase is preferably a Tris HCl buffer. The mobile phase also preferably comprises NaCl. The concentration of NaCl may be the same as the concentration in the solvent used in step 1. The concentration is, for example, 100 to 200 mM, and preferably 120 to 160 mM. The pH of a mobile phase may be the same as the pH of the solvent used in step 1. The pH is, for example, 6 to 8, preferably 6.8 to 7.8, and more preferably 7.2 to 7.6.

The nanoparticle for sensing use **10** is synthesized in this manner.

[2-1-3. Step 3]

Step 3 binds the signal substance **30** to a signal substance binding group in the molecularly imprinted polymer 20 as shown in Figure **5**.

The signal substance **30** comprises the signal group **31** and the binding group **32**. The signal group **31** is the same as a group described above in "1-2. Signal group". The binding group **32** is a group that can be covalently bound to a signal substance binding group. Specifically, the binding group can be appropriately determined by those skilled in the art in accordance with the type of signal substance binding group (amino group (including a monovalent amino group

and divalent amino group), a carboxyl group, a thiol group, a disulfide group-containing group (monovalent group), an aminooxy group, an aldehyde group, or a hydroxyl group).

As the specific conditions for introducing the signal substance **30** to the molecularly imprinted polymer **20**, those skilled in the art can appropriately determine, based on the type of binding group **32** and the type of signal substance binding group, conditions under which the groups can form a covalent bond through a chemical reaction.

The nanoparticle for sensing use **10a** is synthesized in this manner.

[2-1-4. Other steps]

When obtaining a nanoparticle for sensing use with a capping modification group described above in "1-3. Other structures", the following steps can be performed on the synthesized nanoparticle for sensing use **10**. In such a case, it is preferably to introduce a signal group after introducing a capping modification group.

First, a very small amount of biological material (same type of biological material as the biological material **90** targeted for sensing use and the biological material **90'** used as a template) is added to the nanoparticle for sensing use **10**, and the molecularly imprinted cavity **21** is protected by blocking with the biological material (protection step). Next, a compound that inhibits the function of a functional monomer (capping compound), is reacted to bind a capping modification group to the group $R^1$ that interacts with a biological material and/or signal substance binding group of the functional group **22** that is on the surface of the molecularly imprinted polymer **20** other than the molecularly imprinted cavity **21** (capping modification step). Examples of the compound that inhibits the function of a functional monomer (capping compound) includes non-signal substances, which are compounds that are generally used in the suppression of adsorption of a biological material (e.g., compounds having an oligo ethylene glycol chain), compounds having a sugar chain structure, etc. Next, the bound biological material is removed to reproduce the molecularly imprinted cavity **21** (deprotection step).

[2-2. Manufacturing Method 2]

A method of manufacturing the nanoparticle for sensing use of the invention described above in "1. Nanoparticle for sensing use" is not limited to the method described above in "2-1. Manufacturing Method 1". The method of manufacturing a nanoparticle for sensing use of the invention can comprise: step 11 for preparing a template for molecular imprinting, with a surface of a biological material modified by a polymerizable functional group via a reversible linking group; step 12 for synthesizing a molecularly imprinted polymer for a part of the surface of the template by using the polymerizable functional group as a substrate; step 13 for removing the template from the molecularly imprinted polymer by cleaving the reversible linking group; and step 14 for introducing a molecule having a functional group comprising a group for binding a group that interacts with the biological material and a signal substance binding group via a reversible linking group. Manufacturing method 2 can further comprise, in addition to the step of performing steps 11 to 14 described above, a step for binding a molecule which provides a group that interacts with the biological material to the group for binding a group that interacts with the biological material, and a step of binding a signal substance to the signal substance binding group.

Manufacturing method 2 can be applied to any of the nanoparticle for sensing uses of the invention described above in "1. Nanoparticle for sensing use", but is preferably applied when the group that interacts with a biological material is a ligand of the biomolecule.

[3. Substrate for sensing use and reagent for sensing use]

Examples of applications of the nanoparticle for sensing use of the invention described above in "1. Nanoparticle for sensing use" include substrate for sensing uses and reagent for sensing uses.

[3-1. Substrate for sensing use]

A substrate for sensing use comprises a substrate and a nanoparticle for sensing use immobilized on the substrate.

Figure **6** schematically shows an example of a substrate for sensing use. Figure **7** schematically shows another example of a substrate for sensing use. A substrate for sensing use **1** shown in Figure **6** comprises a substrate **40** and a nanoparticle for sensing use **10** immobilized on the substrate **40**. A substrate for sensing use **1a** shown in Figure **7** comprises a substrate **40** and a nanoparticle for sensing use **10a** immobilized on the substrate **40**.

Examples of the material of the substrate **40** include materials selected from the group consisting of metal, glass, and resin. Examples of metal include gold, silver, copper, aluminum, tungsten, molybdenum, etc. Examples of resin include polymethacrylate, polyacrylate, polystyrene, ABS (acrylonitrile-butadiene-styrene copolymer), polycarbonate, polyester, polyethylene, polypropylene, nylon, polyurethane, silicone resin, fluoropolymer, methylpentyl resin, phenol resin, melamine resin, epoxy resin, vinyl chloride resin, etc.

The substrate **40** may be formed by combining a plurality of materials selected from the materials described above. For example, the substrate **40** may be a substrate with a metal film provided on the surface of glass or resin.

A substrate compatible with the sensing means of the signal group **31** can be used as the substrate **40**. Examples of the substrate **40** include a surface plasmon (SPR) substrate, surface-enhanced Raman spectroscopy (SERS) substrate, etc.

The substrate for sensing use **1** immobilized on the substrate **40** is the same as those described as the nanoparticle for sensing use **10** described above in "1. Nanoparticle for sensing use". In the illustrated embodiment, the orientation of the nanoparticle for sensing uses **10** is uniform for convenience, but the orientation of the nanoparticle for sensing uses **10** may be random.

The substrate for sensing use **1** shown in Figure **6** is obtained by immobilizing the nanoparticle for sensing use **10** described above in "1. Nanoparticle for sensing use" on the surface of the substrate **40**.

A method of immobilizing the nanoparticle for sensing use **10** on the surface of the substrate **40** is not particularly limited. Those skilled in the art can appropriately determine a general method for immobilizing a polymeric nanoparticle on a substrate surface. Such a method can be performed by, for example, a molecular film formation step for forming a molecular film having, on the surface, a binding functional group on the surface of the substrate **40** and a step of binding the nanoparticle for sensing use **10** to the binding functional group of the formed molecular film surface. Examples of a functional group on the surface of the nanoparticle for sensing use **10** that binds to a binding functional group on a molecular film surface include groups that interacts with a biological material and/or signal substance binding groups derived from a functional monomer remaining on the surface and/or functional groups derived from an initiator remaining at the end of a polymer constituting the nanoparticle for sensing use **10**, etc.

The illustrated embodiment shows an example of forming, on the surface of the substrate **40,** a molecular film having a carboxyl group as a binding functional group on the surface, and forming an amide bond between the carboxyl group and an amino group on the surface of the nanoparticle for sensing use **10** to cause binding of the nanoparticle for sensing use **10.**

The substrate for sensing use **1a** shown in Figure **7** can be obtained by introducing a signal group to the substrate for sensing use **1** described above. The method of introducing a signal group is the same as the method described above in "2-3. Step 3". The substrate for sensing use **1a** shown in Figure **7** can also be obtained in the same manner as the method of manufacturing the substrate for sensing use **1** described above by using the nanoparticle for sensing use **10a** instead of the nanoparticle for sensing use **10** as a nanoparticle for sensing use immobilized on the substrate **40.**

A substrate for sensing use can be used in an application for detecting the biological material specified in section 1-4. If the biological material is a substance adhering or bound to a biological membrane of a membranous structure (e.g., cell, virus, extracellular vesicle, organelle, etc.) (e.g., membrane protein or membrane sugar chain), a molecule corresponding to an exposed partial structure of a biological membrane (e.g., phospholipid such as phosphatidylserine, etc.), or a fragment of a specific biomolecule (e.g., specific region such as a domain), a substrate for sensing use can be used in an application for detecting a membranous structure (e.g., cell, virus, extracellular vesicle (e.g., exosome, etc.), organelle, etc.) having the biological material or a specific biomolecule (e.g., antibody, etc.) having the fragment (e.g., specific region such as a domain) as a partial structure via the biological material described above. More specific applications vary depending on the type of biological material targeted for sensing use, but for substrate for sensing uses designed for sensing use of a biomarker such as a renal function marker, liver function marker, inflammation marker, or tumor marker, the application can be for a diagnostic application based on renal function, liver function, presence/absence or extent of inflammation, presence/absence or extent of tumor, etc. More specific examples of applications of the substrate for sensing use of the invention, when the biological material is a biological material derived from an animal or plant which falls under haram, include an application for verifying halal from checking the presence/absence of contamination by a biological material derived from an animal or plant (e.g., pig serum albumin, etc.), which falls under haram, in a food product.

[3-2. Reagent for sensing use]

A reagent for sensing use comprises a nanoparticle for sensing use. A nanoparticle for sensing use in a reagent for sensing use is not immobilized on a bulk base material such as a substrate.

A reagent for sensing use is formulated as a liquid or solid (e.g., powder). A reagent for sensing use comprises other pharmaceutically acceptable components. As other components, a solid and/or liquid that does not affect the stability or recognition capability of the reagent for sensing use is appropriately selected. Examples thereof include water, salts such as NaCl, buffer, stabilizers, antioxidants, preservatives, pH modulators, surfactants, excipients, binding agents, etc.

A reagent for sensing use can be obtained by formulation based on a common formulation method using the nanoparticle for sensing use **10** or nanoparticle for sensing use **10a** and the other components described above.

Examples of applications of a reagent for sensing use include the same applications mentioned as the applications for a substrate for sensing use described above. Other examples of applications of a reagent for sensing use include in vitro imaging and in vivo imaging, which utilize a characteristic of a sensing particle not being immobilized on a substrate. Examples of in vitro imaging include a method of preparing the nanoparticle for sensing use **10a,** which targets a biological material in a cell or on the surface for sensing use, and adding the nanoparticle to the cell for imaging of kinetics of the biological material **90** in the cell or the surface through signal detection means such as a fluorescence microscope. Examples of in vivo imaging include a method of preparing the nanoparticle for sensing use **10a,** which targets a biological material within the body for sensing use, and administering the nanoparticle to a circulatory organ such as blood of an animal, e.g., laboratory animal, for imaging of pharmacokinetics of the biological material **90** through signal detection means.

[Examples]

While the present invention is described hereinafter in detail based on the Examples, the present invention is not limited thereto.

**[Test Example 1: Synthesis of a nanoparticle for sensing use-1]**

This Test Example synthesized a nanoparticle for sensing use human serum albumin (HSA) (hereinafter, also referred to as "MIP-NGs"), which corresponds to the nanoparticle for sensing use **10** in Figure **1.** As a control, a nanoparticle was also synthesized in the same manner, except for not performing molecular imprinting (hereinafter, also referred to as "NIP-NGs").

1-1. Synthesis of a molecularly imprinted polymer (Step 1)

Emulsifier-free precipitation polymerization was performed for 12 hours at 70°C under a nitrogen atmosphere after preparing a mixture of components listed in the following table (prepolymer solution) in a 50 mL Schlenk flask, and incubating the mixture for 24 hours at 4°C to allow thorough interaction between a functional monomer and HSA. Meanwhile, emulsifier-free precipitation polymerization was performed for control NIP-NGs under the same condition except for not including HSA in a prepolymer solution.

[Table 2]

| | | **Example 1 MIP-NGs** | **Comparative Example 1 NIP-NGs** |
|---|---|---|---|
| **Template protein** | **Human serum albumin (HSA)** | 6.70 mg (0.101 μmol) | - |
| **Functional monomer** | **Functional** monomer **represented by formula (51a)** | 39.3 mg (0.15 mmol) | 39.3 mg (0.15 mmol) |
| **N-substituted or unsubstituted (**meth) **acrylamide** | N-Isopropylacrylamide (NIPAm) | 204 mg (1.81 mmol) | 6.70 mg (0.101 μmol) |
| **Biocompatible monomer** | 2-Metacryloyloxyethyl phosphorylcholine (MPC) | 29.5 mg (0.100 mmol) | 29.5 mg (0.100 mmol) |
| **Crosslinker** | N,N' -Methylenbisacrylamide (MBAA) | 16.5 mg (0.108 mmol) | 16.5 mg (0.108 mmol) |

(continued)

| | | Example 1 MIP-NGs | Comparative Example 1 NIP-NGs |
|---|---|---|---|
| **Initiator** | 2-2'-Azobis(2-methyl propion amide) dihydrochloride (V-50) | 109 mg (0.400 mmol) | 109 mg (0.400 mmol) |
| **Solvent** | 10mM PBS buffer pH 7.4 **(containing 140 mM NaCl)** | 50 mL | 50 mL |

[Chemical Formula 16]

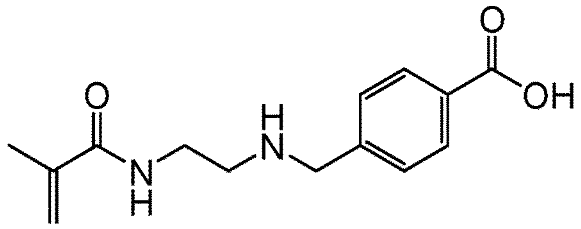

(51a)

The functional monomer represented by formula 51a has a benzene ring, a carboxyl group, and a secondary amino group. It is understood that a benzene ring interacts with a hydrophobic pocket of HSA, and a carboxyl group interacts with a positively charged site represented by a lysine residue of HSA in MIP-NGs. Furthermore, a secondary amino group is used for introduction of a fluorescent molecule in the post-imprinting modification (step 3) described below.

1-2. Removal of template (Step 2)

Particles were purified by using anion exchange chromatography in the following manner. In this step, a strong interaction between an anion exchange chromatography carrier and HSA was used to remove a template from a molecularly imprinted polymer.

The solution after a polymerization reaction was subjected to three ultrafiltrations (25°C, 7500 × g, 20 min) by using an Amicon Ultra-4 10 kDa dialysis tube. Unreacted monomers were removed, and the solvent was exchanged from PBS to pH 7.4 10 mM Tris HCl buffer containing 140 mM NaCl to obtain unpurified samples.

1.0 g of anion exchange chromatography packing material DEAE-Sephadex was added to 35 mL of Tris HCl buffer containing 140 mM NaCl (pH of 7.4) and allowed to swell for 24 hours, and filled in a polypropylene Big LibraTube® to a height of about 5.5 cm. 1 mL of the unpurified sample was added thereto. 10 mM Tris HCl buffer containing 140 mM NaCl (pH of 7.4) was used as a mobile phase, and the eluate was fractionated to 1.5 mL each. Fluorescence from a functional monomer (functional group) of a fractionated fraction (400 nm at an excitation wavelength of 280 nm) and fluorescence from HSA (340 nm at an excitation wavelength of 280 nm) were measured with Hitachi Technologies F-2500 to confirm that HSA has been removed. $2^{nd}$ to $7^{th}$ fractions were all mixed, and the solvent was replaced with PBS using a 10 kDa dialysis tube. The solution was concentrated to 5 mL. A purified sample solutions of MIP-NGs or NIP-NGs were obtained thereby.

1-3. Measurement of particle size

The particle sizes of the resulting MIP-NGs and NIP-NGs were evaluated by DLS measurement (Malvern ZETASIZER NANO-ZS MAL500735) in the following manner.

500 μL of purified sample solution was added to a 1.5 mL microtube and dried overnight at 90°C. The concentration of solids was calculated from the mass before and after drying based on the following equation (wherein $w_0$ represents the weight of 1.5 mL microtube (g), $w_1$ represents the weight before drying (g), and $w_2$ represents the weight after drying (g)). The results are provided in the following table.

[Numeral 1]

$$\text{Concentration of solids (\%)} = \frac{w_2 - w_0}{w_1 - w_0} \times 100$$

[Table 3]

| | Volume average particles size | Concentration of solids |
|---|---|---|
| **Example 1 MIP-NGs** | 8.07 nm | 0.0429 wt% |
| **Comparative Example 1 NIP-NGs** | 7.90 nm | 0.166 wt% |

As shown in Table 3, the presence of nanometer order particles was confirmed for MIP-NGs and NIP-NGs.

**[Test Example 2: Preparation of substrate for sensing use-1]**

In this Test Example, the MIP-NGs (Example 1) and NIP-NGs (Comparative Example 1) obtained in the Test Example 1 were immobilized on a substrate in the following manner. Specifically, the MIP-NGs obtained in Test Example 1 were immobilized on a substrate to prepare a substrate for sensing use human serum albumin (HSA) corresponding to the substrate for sensing use **1** in Figure **6.** NIP-NGs were also immobilized on a substrate in the same manner.

2-1. Immobilization of a particle on a substrate

An SPR gold substrate (SIA Kit Au, GE Healthcare) was washed with ethanol and pure water, then immersed in a 1 mM ethanol solution of 11-Mercaptoundecanoic acid and incubated for 24 hours at 25°C. The substrate after the reaction was washed with ethanol and pure water, and dried with nitrogen gas.

By using an SPR-based molecular interaction analyzer (Biacore 3000; GE Healthcare), a 1:1 mixture of 0.1 M N-Hydroxysuccinimide (NHS) and 0.4 M 1-(3-dimethylaminopropyl)-3-ethylcarbodiimide hydrochloride (EDC) was injected for 7 minutes at a flow rate of 20 μL/min to form a molecular film, then the purified sample (466 μg/mL) obtained in step 2 of Test Example 1 was injected for 7 minutes at a flow rate of 20 μL/min for immobilization of a particle on a substrate. In this immobilization, a particle is immobilized on a substrate by a reaction between an NHS ester on the molecular film surface and a functional group on the particle surface (the functional group can be an amino group derived from a functional monomer and/or an amidino group derived from an initiator remaining at the end of a polymer constituting a particle). Subsequently, 1 M of 2-amino ethanol (pH 8.5) was injected for 7 minutes at a flow rate of 20 μL/min to block unreacted NHS ester on the surface of the molecular film.

2-2. Protein adsorption test

Human serum albumin (HSA; Mw: 66.5 kDa, pI: 4.6), cytochrome c (Cyt c; Mw: 11.7 kDa, pI: 10.3), or transferrin (Trf; 80 kDa, pI: 6.1) dissolved in PBS (10 mM, pH 7.4, containing 140 mM NaCl) was injected to particles immobilized on a substrate under the following conditions to conduct an adsorption experiment.

[Table 4]

| | |
|---|---|
| **Flow rate** | 20 μL/min |
| **Injection volume** | 100 μL |
| **Temperature** | 25°C |
| **Data point** | 6 min after injection |
| **Running buffer** | PBS |
| **Proteins for adsorption test (concentration)** | HSA, Cyt c, Trf (0, 1.5, 3, 6.02, 12.0, 24.1, 48.1, 96.2, 192 nM) |

The binding constant was calculated by curve fitting from the resulting adsorption isotherm. Fitting was performed based on the following equation (wherein K represents a binding constant, G represents the protein concentration, D represents the amount of maximum change in SPR signal intensity, and H represents an independent variable) by using

DeltaGraph 5.4.5v (Nihon Poladigital). The results are provided in the following table.

[Numeral 2]

$$y = \frac{D}{2KG}\left\{(1 + K \times G + K \times H) - ((1 + K \times G + K \times H)^2 - 4 \times K \times K \times H \times G)^{1/2}\right\}$$

[Table 5]

| | Binding constant$M^{-1}$ (vs HSA) |
|---|---|
| **Example 1 MIP-NGs** | $4.98 \times 10^7$ |
| **Comparative Example 1 NIP-NGs** | $6.00 \times 10^6$ |

[Table 6]

| | **Protein** | Binding constant $\mathbf{M^{-1}}$ |
|---|---|---|
| **Example 1 MIP-NGs** | **HSA (target of sensing)** | $4.98 \times 10^7$ |
| | Cyt c | $6.28 \times 10^6$ |
| | Trf | $5.63 \times 10^6$ |

As shown in Table 5, the binding constant is significantly greater for MIP-NGs relative to NIP-NGs. Thus, MIP-NGs were confirmed to exhibit high affinity to HSA due to an effect of molecular imprinting. Since the binding constant for HSA is significantly greater among the three types of proteins shown in Table 6, it is inferred that MIP-NGs have a binding cavity that is specific to HSA.

**[Test Example 3: Preparation of a comparative substrate for sensing use provided with an MIP film]**

A comparative substrate for HSA sensing use was prepared by forming a flat MIP film on the substrate by using the same monomer as the monomer used in Test Example 1 for a comparative test in Test Example 5 described below.

3-1. Preparation of a polymerizable functional group immobilized substrate

A gold-coated glass substrate (5 × 10 mm) was washed with ethanol and pure water, then treated with UV-$O_3$ for 30 minutes. The substrate after treatment was immersed in a 1 mM ethanol solution of 11-mercapto-1-undecanol and incubated for 24 hours at 25°C. Subsequently, the substrate was washed with ethanol and pure water and dried with nitrogen gas. The resulting substrate was immersed in a $CH_2Cl_2$ solution with 10 mM methacryloyl chloride and 10 mM triethylamine dissolved therein, and incubated for 24 hours at 25°C. The substrate was then washed with ethanol and pure water and dried with nitrogen gas, whereby a polymerizable functional group immobilized substrate was obtained.

3-2. Synthesis of an MIP film

A prepolymer solution was prepared by mixing the components shown in the following table. A polymerizable functional group immobilized substrate was covered with a silicon rubber sheet (thickness: 0.5 mm) provided with 3 mm radius pores. The prepolymer solution was added dropwise to the exposed portion of the gold substrate surface within the pores, which was covered with a cover glass from above. This was set on a photoreaction system (KeyChem-Lumino, YMC Co., Ltd.) to perform a photopolymerization reaction ($\lambda_{em}$ = 365 nm, 4°C, 10 min), whereby an MIP film (Comparative Example 2) was obtained.

[Table 7]

| | | Comparative Example 2 MIP film |
|---|---|---|
| **Template protein** | **Human serum albumin (HSA)** | 0.134 mg (0.02 μmol) |
| **Functional monomer** | **Functional monomer represented by formula (51a)** | 0.79mg (3.0 μmol) |
| **N-substituted or unsubstituted (meth) acrylamide** | N -Isopropylacrylamide (NIPAm) | 4.08 mg (36 μmol) |
| **Biocompatible monomer** | 2-Metacryloyloxyethyl phosphorylcholine (MPC) | 0.59 mg (2 μmol) |
| **Crosslinker** | N,N'-Methylenbisacrylamide (MBAA) | 0.33 mg (2.1 μmol) |
| **Initiator** | **Photo-initiator represented by formula (8)** | 3.07 mg (8.0 μmol) |
| **Solvent** | 10mM PBS buffer pH 7.4 **(containing 140 mM NaCl)** | 1 mL |

[Chemical Formula 17]

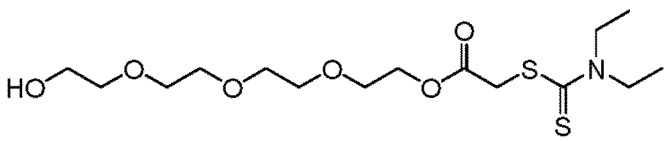

(8)

3-3. Template removal

A substrate with an MIP film (Comparative Example 2) provided thereon was washed with pure water, then immersed in an aqueous 1M NaCl solution and 0.5 wt% of aqueous SDS solution (2 hours each) to remove HSA.

3-4. Introduction of fluorescence

10 μL of 50 μg/mL ATTO647N NHS solution (in 0.5% DMSO comprising 10 mM PBS) was added dropwise to the MIP film portion of the washed substrate, and was incubated for 1 hour at room temperature. The substrate was then washed with pure water.

A comparative MIP film substrate for HSA sensing was obtained thereby. The comparative MIP film substrate for HSA sensing has an molecularly imprinted cavity for HSA (recess) on the MIP film surface provided on the substrate, and is provided with at least a functional group derived from a functional monomer and a fluorescent group within the molecularly imprinted cavity (recess). The fluorescent group would have an increase in fluorescence from a change in environment upon adsorption of a target of sensing to the imprinted cavity (recess).

**[Test Example 4: Synthesis of a nanoparticle for sensing use-2]**

This Example synthesized a nanoparticle for HSA sensing use corresponding to the nanoparticle for sensing use **10a** in Figure **2.**

Introduction of a signal substance (fluorescent substance) (Step 3)

5 μL of a 0.5 wt% DMSO solution comprising 10 mM PBS of 10 mg/mL fluorescent substance (ATTO647N NHS, excitation wavelength of 646 nm, fluorescence wavelength of 664 nm) was added to 1 mL of the purified sample (466 μg/mL) of MIP-NGs (Example 1) obtained in step 2 of Test Example 1, and the mixture was incubated for 2 hours at 25°C. Subsequently, MIP-NGs introduced with a fluorescent group were obtained by subjecting the mixture to ultrafiltration (25°C, 7500 × g, 20 minutes, 3 times) using Amicon Ultra-4 10 kDa and washing MIP-NGs with PBS.

**[Test Example 5: Preparation of substrate for sensing use-2]**

This Test Example prepared a substrate for HSA sensing use corresponding to the substrate for sensing use

**1a** in Figure **7.**

5-1. Immobilization of fluorescent group introduced MIP-NGs on a substrate

A gold-coated glass substrate (5 mm × 10 mm) was washed with ethanol and pure water, then immersed in a 1 mM ethanol solution of 11-Mercaptoundecanoic acid, and incubated for 24 hours at 25°C. The substrate after the reaction was washed with ethanol and pure water and dried with nitrogen gas.

100 μL of aqueous solution comprising 0.2 M EDC and 50 mM NHS was added dropwise, and the substrate was incubated for 1 hour at room temperature. After washing with pure water, 100 μL of the fluorescent group introduced MIP-NGs (47 μg/mL) obtained in Test Example 4 was added dropwise, and reacted for 2 hours at room temperature for immobilization. After washing with pure water, 100 μL of 1 M 2-amino ethanol (pH 8.5) was added dropwise and allowed to react for 30 minutes for blocking, whereby a substrate for HSA sensing use with fluorescent group introduced MIP-NGs immobilized on the substrate was obtained.

5-2. Protein adsorption test-1

A protein adsorption test was conducted in the same manner as Test Example 2 for the resulting fluorescent group introduced MIP-NG immobilized substrate for HSA sensing use. The results are provided in the following table.

[Table 8]

| | Protein | Binding constant $M^{-1}$ |
|---|---|---|
| **Example 2 Fluorescence Introduced MIP-NGs** | **HSA (Sensing target)** | $3.17 \times 10^8$ |
| | Cyt c | $3.00 \times 10^7$ |
| | Trf | $7.10 \times 10^6$ |

As shown in Table 8, the binding constant for HSA improved further by introducing a fluorescent group. It is inferred that this is due to not only the introduced fluorescent group not inhibiting binding between HSA targeted for sensing use and a group that interacts with a biological material in a molecularly imprinted cavity, but also the hydrophobicity of the introduced fluorescent group adding an interaction between a hydrophobic fluorescent group and a hydrophobic region of HSA to an interaction between a benzene ring in the group that interacts with a biological material and the hydrophobic region of HSA.

5-3. Protein adsorption test-2

A protein adsorption test was conducted based on a selectivity factor for the resulting fluorescent group introduced MIP-NG immobilized substrate for HSA sensing use and the comparative substrate for HSA sensing use provided with an MIP film obtained in Test Example 3. The features of each of the compared substrate for HSA sensing use are described in the following Table.

[Table 9]

| | **Shape of MIP immobilized on substrate** | **Constituent monomer** | **Template, sensing target** | **Fluorescent group** |
|---|---|---|---|---|
| **Example 2** | **Particulate** | **Functional monomer represented by formula (51a)** NIPAm MPC | HSA | ATTO647N |
| **Comparative Example 2** | **Flat membrane** | | | |

For a substrate for sensing use, human serum albumin (HSA; Mw: 66.5 kDa, pI: 4.6), cytochrome c (Cyt c; Mw: 11.7 kDa, pI: 10.3), or transferrin (Trf; 80 kDa, pI: 6.1) dissolved in PBS (10 mM, pH 7.4, containing 140 mM NaCl) was incubated for 10 minutes at 25°C, and measurement was taken under the following conditions by using a fluorescence microscope equipped with an automatic SIC dispenser.

(Measurement conditions for a fluorescence microscope with an automatic SIC dispenser)

*Fluorescence microscope

Filter: Cy5
Objective lens: ×5
Exposure time: 0.1 seconds
Quantity of light: 12%
Light source: mercury lamp

*Automatic SIC dispenser sequence

1. Tip attachment
2. Sample suction (150 μL)
3. Incubation (25°C, 10 minutes)
4. Keep measurement position (measurement port)

*Repeated the procedure of 2→4 for each protein

The ratio of change in fluorescence intensity before and after adding each protein to a substrate for sensing use was divided by the ratio of change in fluorescence intensity of HSA, which is targeted for sensing use, to calculate the selectivity factor. Specifically, the selectivity factor is represented by the following equation, wherein $F_0$ represents the fluorescence intensity of the substrate surface in the buffer described above, $F_{sample}$ represents the fluorescence intensity of the substrate surface in a sample comprising each protein, and $F_{HSA}$ represents the fluorescence intensity of the substrate surface in a sample comprising HSA, which is targeted for sensing use.

[Numeral 3]

$$\text{Selectivity factor} = \frac{(F_{sample}-F_0)/F_0}{(F_{HSA}-F_0)/F_0}$$

Figure **8** shows the results for the fluorescent group introduced MIP-NG (particle) immobilized substrate for HSA sensing use (Example 2). Figure **9** shows results for the comparative HSA sensing MIP film substrate provided with an MIP film obtained in Test Example 3 (Comparative Example 2).

A value of selectivity factor less than 1 indicates the presence of a capability to recognize HSA. As shown in Figures **8** and **9,** a capability to recognize HSA was found in each substrate for sensing use. However, as shown in Figure **9,** it was observed that a large change in fluorescence was exhibited for not only HSA targeted for sensing use, but also Cyt c and Trf, which are not targeted for sensing use, for a comparative substrate for HSA sensing use provided with an MIP film (Comparative Example 2). Thus, said substrate was found to have poor specificity. In contrast, high specificity for HSA, which is targeted for sensing use, was found for a substrate for HSA sensing use with a particulate MIP immobilized on the substrate (Example 2) as shown in Figure **8.**

**[Test Example 6: Measurement of HSA in a serum sample]**

This Test Example measured HSA in a serum sample by using the substrate for HSA sensing use (Example 2) corresponding to the substrate for sensing use **1a** in Figure **7** prepared in Test Example 5.

Eight human serum sample specimens approved by the ethics committee of Kobe University Graduate School of Medicine with an ID number of 180128 were used. First, the HSA concentration of each of these samples was checked by measurement with bromocresol purple (BCP method). The checked HSA concentrations are shown in the following table.

[Table 10]

| Specimen number | 1 | 2 | 3 | 4 | 5 | 6 | 7 | 8 |
|---|---|---|---|---|---|---|---|---|
| HSA concentration (g/dL) | 3.6 | 4.2 | 4 | 3 | 4.8 | 4.2 | 4.4 | 4.2 |

Next, each specimen was diluted 1000-fold with a buffer (PBS (10 mM, pH 7.4, containing 140 mM NaCl)). The relative fluorescence intensity was found in the same manner as Protein adsorption test-2 in Test Example 5 by using the substrate for HSA sensing use corresponding to the substrate for sensing use **1a** in Figure **7** prepared in Test Example 5.

Figure **10** shows the correlation between the obtained relative fluorescence intensity and the concentration obtained by the BCP method. The relative fluorescence intensity is represented by $F-F_0/F_0$ (wherein $F_0$ represents the fluorescence intensity of the substrate surface in the buffer described above, and F represents the fluorescence intensity of the substrate surface in a specimen). As shown in Figure **10,** a high correlation was found between the HSA concentration according to the BCP method and the relative fluorescence intensity based on HSA bond measured by a substrate for HSA sensing use. This indicates that a substrate for HSA sensing use can also detect and quantify HSA from human serum.

**[Test Example 7: Verification of halal using a substrate for PSA sensing use ]**

This Example prepared a substrate for PSA sensing use with a particle immobilized on the substrate, with pig serum albumin (PSA) as a target of sensing, and verified halal using this substrate based on use of PSA as a marker for pig derived component contamination in halal food.

7-1. Synthesis of sensing nanoparaticle-3

First, a nanoparticle for PSA sensing use corresponding to the nanoparticle for sensing use **10** in Figure **1** was synthesized. Specifically, MIP-NGs were synthesized in the same manner as Test Example 1 by using a mixture of components listed in the following table (prepolymer solution), except for using PSA as a template protein (Example 3).

[Table 11]

| | | Example 3 MIP-NGs | Comparative Example 1 NIP-NGS |
|---|---|---|---|
| **Template protein** | **Pig serum albumin (PSA)** | 6.70 mg (0.101 μmol) | - |
| **Functional monomer** | **Functional monomer represented by formula (51a)** | 39.3 mg (0.15 mmol) | 39.3 mg (0.15 mmol) |
| **N-substituted or unsubstituted (meth) acrylamide** | N -Isopropylacrylamide (NIPAm) | 204 mg (1.81 mmol) | 6.70 mg (0.101 μmol) |
| **Biocompatible monomer** | 2-Metacryloyloxyethyl phosphorylcholine (MPC) | 29.5 mg (0.100 mmol) | 29.5 mg (0.100 mmol) |
| **Crosslinker** | N,N' -Methylenbisacrylamide (MBAA) | 1.6.5 mg (0.108 mmol) | 16.5 mg (0.108 mmol) |
| **Initiator** | 2-2'-Azobis(2-methyl propion amide) dihydrochloride (V-50) | 109 mg (0.400 mmol) | 109 mg (0.400 mmol) |
| **Solvent** | 10mM PBS buffer pH 7.4 **(containing 140 mM NaCl)** | 50 mL | 50 mL |

7-2. Measurement of particle size

When the particle size was measured for the obtained MIP-NGs and NIP-NGs in the same manner as Test Example 1, the presence of nanometer order particles was confirmed.

[Table 12]

| | **Volume average particle size** |
|---|---|
| **Example 3 MIP-NGs** | 24.55 nm |
| **Comparative Example 1 NIP-NGs** | 20.7 nm |

7-3. Synthesis of a nanoparticle for sensing use-4

Next, a nanoparticle for PSA sensing use corresponding to the nanoparticle for sensing use **10a** in Figure **2** was synthesized. Specifically, a fluorescent substance ATTO647N NHS was introduced to the MIP-NGs of Example 3 in the same manner as Test Example 4 to obtain fluorescent group introduced MIP-NGs (Example 4).

7-4. Sensing substate-3

Next, the fluorescent group introduced MIP-NGs of Example 4 were immobilized on a substrate as follows.

A gold-coated glass SPR substrate (5 mm × 10 mm) was washed with ethanol and pure water, then treated with a UV-$O_3$ cleaner for 20 minutes, immersed in a 1 mM ethanol solution of 11-amino-1-undecaneoctanethiol hydrochloride, and incubated for 24 hours at 25°C. The substrate after the reaction was washed with ethanol and pure water and dried with nitrogen gas. This resulted in a substrate with a molecular film formed on the surface. Furthermore, an aqueous solution (100 μL) with EDC (0.4 M) and NHS (0.1 M) dissolved therein was added dropwise to the molecular film on the substrate, and left standing for 1 hour. 100 μL (300 μg/mL) of the MIP-NGs introduced with a fluorescent group of Example 3 was added dropwise thereto and reacted for 2 hours for immobilization of the particles to the substrate. In this immobilization, a particle is immobilized on a substrate by a reaction between an amino group on the molecular film surface and a functional group on the particle surface (carboxyl group derived from a functional monomer). 100 μL of aqueous 10 mM sulfo-NHS acetate solution was added dropwise and reacted for 30 minutes for blocking of the amino group on the surface.

7-5. Measurement of PSA in a sample

A fluorescent substance was introduced to the NIP-NGs in Comparative Example 1 in the same manner described above to obtain fluorescence introduced NIP-NGs (Comparative Example 3). The fluorescent group introduced NIP-NGs of Comparative Example 3 were immobilized on a substrate in the same manner to prepare a comparative substrate for sensing use. A substrate for PSA sensing use with the fluorescent group introduced MIP-NGs of Example 4 immobilized thereon and a substrate for sensing use with the fluorescent group introduced NIP-NGs of Comparative Example 3 immobilized thereon were used to measure the concentration of PSA contained in a sample with a PSA concentration in a buffer (PBS (10 mM, pH 7.4, containing 140 mM NaCl)) of 0 to 20 nM in the same manner as Test Example 6. The detection limit was 12 ng/mL.

Figure **11** shows the correlation between the obtained relative fluorescence intensity and the PSA concentration for a substrate for PSA sensing use with the fluorescent group introduced MIP-NGs of Example 4 immobilized thereon. The relative fluorescence intensity is the same as Test Example 6. As shown in Figure **11,** a high correlation was found between the PSA concentration and relative fluorescence intensity based on a PSA bond measured by the substrate for PSA sensing use. This indicates that not only human serum albumin HSA, but also pig serum albumin PSA can be detected and quantified by changing the template of an imprinted recess. Meanwhile, fluorescence was measured in the same manner for a substrate for sensing use with the fluorescent group introduced NIP-NGs of Comparative Example 3 immobilized thereon, but a change in fluorescence was hardly observed. Furthermore, the following table shows the results of calculating the apparent binding constant from the obtained ratio of change in fluorescence.

[Table 13]

| | Binding constant$M^{-1}$ (vs PSA) |
|---|---|
| **Example 4 MIP-NGs** | $1.87 \times 10^8$ |

(continued)

| | Binding constantM$^{-1}$ (vs PSA) |
|---|---|
| **Comparative Example 3 NIP-NGs** | $9.00\times10^7$ |

As shown in Table 13, the MIP-NGs (Example 4) had a significantly greater binding constant relative to the NIP-NGs (Comparative Example 3). Thus, MIP-NGs were confirmed to exhibit high affinity to PSA due to an effect of molecular imprinting.

7-6. Specificity to PSA

Samples were prepared by dissolving pig serum albumin (PSA), human serum albumin (HSA), bovine serum albumin (BSA), lysozyme (LyZ) or transferrin (Trf) in a buffer (PBS (10 mM, pH 7.4, containing 140 mM NaCl)) at a concentration of 40 nM. These samples were measured in the same manner as Test Example 6 by using a substrate for sensing use with the fluorescent group introduced MIP-NGs of Example 4 immobilized thereon or a substrate for sensing use with the fluorescent group introduced NIP-NGs of Comparative Example 3 immobilized thereon. The selectivity factor is a ratio of the change in fluorescence in a sample to the change in fluorescence in 40 nM PSA, and is represented by the following equation, wherein $F_0$ represents the fluorescence intensity of the substrate surface in the buffer described above, $F_{sample}$ represents the fluorescence intensity of the substrate surface in a sample, and $F_{PSA}$ represents the fluorescence intensity of the substrate surface in 40 nM PSA.

[Numeral 4]

$$\text{Selectivity factor} = \frac{(F_{sample}-F_0)/F_0}{(F_{PSA}-F_0)/F_0}$$

Figure **12** shows results for a substrate for sensing use with the fluorescent group introduced MIP-NGs of Example 4 immobilized thereon, and Figure **13** shows results for a substrate for sensing use with the fluorescent group introduced NIP-NGs of Comparative Example 3 immobilized thereon.

A value of selectivity factor less than 1 indicates the presence of a capability to recognize PSA. As shown in Figures **12** and **13,** a capability to recognize PSA was found in each substrate for sensing use. However, as shown in Figure **13,** it was observed that a large change in fluorescence was exhibited for not only PSA targeted for sensing use, but also proteins that are not targeted for sensing use, for a substrate for sensing use with the fluorescent group introduced NIP-NGs of Comparative Example 3 immobilized thereon. Thus, the substrate was found to have poor specificity. In contrast, high specificity for PSA, which is targeted for sensing use, was found for a substrate for sensing use with the fluorescent group introduced MIP-NGs of Example 4 immobilized thereon, as shown in Figure **12.**

7-7. Halal verification-1

1 g of thinly sliced beef was added to 4 mL of phosphate buffer (pH 7.4), and the beef was homogenized for 1 minute at 10,000 rpm by using a homogenizer. The supernatant obtained by centrifugation (16,000 × g, 30 min, 4°C) was filtered three times with a 0.2 pm filter, and diluted 500-fold with a phosphate buffer (pH 7.4) to obtain a beef extract sample.

PSA was added at various concentrations to the resulting beef extraction sample to prepare PSA contaminated beef extract samples. Separately, PSA was added to a phosphate buffer (pH 7.4) at the same concentration to prepare a PSA sample. The change in fluorescence was measured for the PSA contaminated beef extract samples and the PSA sample by using a substrate for sensing use with the fluorescent group introduced MIP-NGs of Example 4 immobilized thereon, and the change in relative fluorescence intensity was deduced in the same manner described above. The results are shown in Figure **14.**

As shown in Figure **14,** it was found that a substrate for sensing use with the fluorescent group introduced MIP-NGs of Example 4 immobilized thereon can quantify PSA contaminating a beef extract sample (in 500× beef extract) in the same manner as PSA in a phosphate buffer (in PBS buffer).

7-8. Verification of halal-2

A beef extract sample with a pork extract sample contamination was prepared. First, a beef extract sample diluted 500-fold was obtained in the same manner as above. Further, a pork extract sample diluted 500-fold was obtained in the same manner, except for using pork instead of beef. The two extract samples were mixed, so that the beef extract sample content and pork extract sample contamination rate reach the ratios shown in the following table, to obtain beef extract samples contaminated with a pork extract sample. The ratios in the table indicate the amount corresponding to the amount of protein (weight based) in the extract samples.

[Table 14]

| | **Pork extract sample contamination rate (wt%)** | **Beef extract sample content (wt%)** |
|---|---|---|
| entry 1 (negative control) | 0 | 100 |
| entry 2 | 0.1 | 99.9 |
| entry 3 | 1 | 99 |

The change in fluorescence for the beef extract samples contaminated with a pork extract sample was measured by using a substrate for sensing use with the fluorescent group introduced MIP-NGs of Example 4 immobilized thereon to deduce the relative fluorescence intensity in the same manner described above. The results are shown in Figure **15.** In Figure **15,** the horizontal axis indicates the pork extract sample contamination rate, and the vertical axis indicates the relative fluorescence intensity.

As is apparent from Figure **15,** a change in fluorescence was hardly observed in a 100% beef extract sample negative control with no pork extract sample contamination in the same manner as PBS buffer. In contrast, PSA contained in a pork extract sample was able to be detected from a beef extract sample contaminated with a pork extract sample. In particular, PSA was able to be detected even at the pork extract sample contamination rate of 0.1 wt%, so that a substrate for sensing use with the fluorescent group introduced MIP-NGs of Example 4 immobilized thereon was found to be suitable for verification of halal at a high sensitivity.

**[Test Example 8: Immune response using** a **PSA sensing particle (unimmobilized)** ]

This Test Example used a particle targeting pig serum albumin (PSA) for sensing use in a free state without being immobilized on a substrate to test immune responses to each of PSA and transferrin.

A particle targeting PSA for sensing use (fluorescent group introduced MIP-NGs; Example 4) was prepared by the same method as "Synthesis of a sensing nanoparaticle-3" and "Synthesis of a nanoparticle for sensing use-4" in Test Example 7.

A protein solution (in 10 mM phosphate buffer (pH 7.4) comprising 140 mM NaCl) was added to 80 μg/mL fluorescent group introduced MIP-NGs so that the final concentration of protein (PSA or transferrin) would be 5, 10, 50, 100, 500, or 1000 μg/mL (final quantity of solution: 500 μL), and the mixture was incubated for 15 minutes at 25°C. The fluorescence spectrum of the solution was then measured with a fluorometer (F-2500, Hitachi Technologies) (excitation wavelength λex: 647 nm, λem: 658 to 690 nm). The results are shown in Figure **16.** The relative fluorescence intensities of fluorescent wavelength of 667 nm with respect to the protein concentration of each of PSA and transferrin (PSA: 5, 10, 50, 100, 500, and 1000 μg/mL, transferrin: 10, 50, 100, 500, and 1000 μg/mL) was plotted. The results are shown in Figure **17.**

When a PSA solution was added to the fluorescent group introduced MIP-NGs of Example 4 in a free state, an increase in the fluorescence intensity was observed with an increase in the concentration, as shown in Figure **16.** Since the same phenomenon was observed when the fluorescent group introduced MIP-NGs of Example 4 were immobilized on a substrate (Test Example 7), the increase in fluorescence intensity shown in Figure **16** is suggested to be a change in fluorescence intensity associated with a binding of PSA to the fluorescent group introduced MIP-NGs of Example 4. Further, as shown in Figure **17,** a greater change in fluorescence intensity was exhibited when PSA was added compared to the change in fluorescence when a reference protein transferrin was added. This shows that the fluorescent group introduced MIP-NGs of Example 4 can selectively adsorb to PSA, and binding information thereof can be transmitted as a fluorescence signal, even in a free state without being immobilized on a substrate.

**[Example 9: Synthesis of a nanoparticle for sensing use-5]**

This Test Example synthesized an Fc domain sensing (and Fc domain capture mediated IgG sensing) nanoparticle, which corresponds to the nanoparticle for sensing use **10a** in Figure 2.

9-1. Synthesis of functional monomer

The functional monomer represented by formula 51b was designed and synthesized in accordance with the following scheme. The functional monomer represented by formula 51b has a boronylaryl group and a divalent amino group. In MIP-NGs, a boronylaryl group interacts with a sugar chain of an Fc domain of IgG, and a secondary amino group is used for introducing a fluorescent molecule in post-imprinting modification.

[Chemical Formula 18]

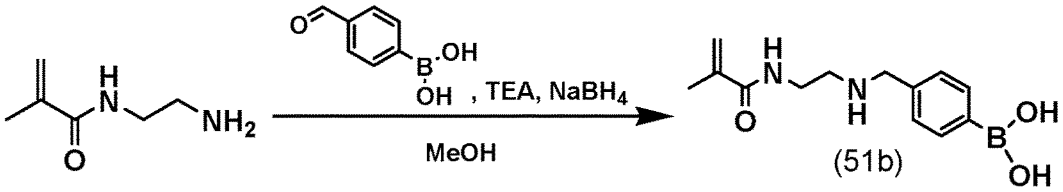


(51b)

Ethylenediamine monomethacrylamide hydrochloride (ca. 1.57 mmol), 4-formylphenylboronic acid (215 mg, 1.5 mmol) , and triethylamine (210 $\mu$L, 1.5 mmol) were dissolved in methanol (10 mL) and stirred at room temperature (0.5 h). The reaction was tracked with TLC (MeOH$\rightarrow$UV@254 nm and ninhydrin), and UV absorption and manifestation of ninhydrin stained spot (Rf: 0.55, purple) were confirmed. Thus, it was determined that a Schiff base was formed, and $NaBH_4$ (120 mg, 9.0 mmol) was added thereto. After the addition, the mixture was stirred overnight (8 h) at room temperature. Since a spot of the compound of interest (Rf: 0.25, yellow) was observed from TLC (MeOH$\rightarrow$UV@254 nm and ninhydrin), the reaction was discontinued, and the solvent was subjected to vacuum evaporation with a rotary evaporator. The residue was purified using an auto column (silica: universal premium, MeOH/DCM = 70/30$\rightarrow$100/0, GR) to obtain the compound of interest.

Yield: 175 mg (0.668 mmol, 42.5 %)

$^1$H-NMR (500 MHz, $D_2O$): $\delta$ = 7.62 (d, 2H, phenyl), 7.30 (d, 2H, phenyl), 5.69 (s, 1H, vinyl), 5.44 (s, 1H, vinyl), 3.97 (s, 2H, N-$CH_2$-), 3.47 (t, 2H, -$CH_2$-), 2.99 (br, 2H, -$CH_2$-), 1.89 (s, 3H, -Me)

9-2. Preparation of Fc domain

10 mg of IgG was dissolved in 10 mM phosphate buffer (pH 7.4, 140 mM NaCl) (5mL) to prepare an antibody solution. 0.02 M L-cysteine, 0.002 M EDTA, and 0.1 mg/mL papain were dissolved in 10 mM phosphate buffer (pH 7.4, 140 mM NaCl) to prepare an enzyme solution. The same amounts of the antibody solution and enzyme solution were mixed and reacted for 24 hours at 37°C. The solvent was then replaced with 20 mM phosphate buffer (pH 7.0) by using a 10 kDa ultrafiltration film (7500 g, 20 min × 3) on the reaction solution. Subsequently, unreacted IgG was separated using a 100 kDa ultrafiltration film (Amicon Ultra) (7500 g, 20 min × 3). The resulting solution (1 mL) was purified using a Protein A column (Hitrap™ Protein A HP, Cytiva). 20 mM phosphate buffer (pH 7.0) was used as a binding buffer, and 0.1 M citrate buffer (pH 3.0) was used as an elution buffer for purification in a syringe (flow rate: about 1 mL/min). The solvent was replaced with a 10 mM carbonate buffer (pH 9.2) by using a 10 kDa ultrafiltration film (7500 g, 20 min × 3) on the solution after purification. Purification of the Fc domain was confirmed by SDS-PAGE.

9-3. Synthesis of a nanoparticle for sensing use-5

A mixture (prepolymer solution) of the components listed in the following table was subjected to emulsifier-free precipitation polymerization for 12 hours at 50°C to synthesize a polymeric nanogel (step 1). A label monomer was also used to facilitate the purification of particles for the monomer contained in the prepolymer solution. The solution after polymerization (2 mL) was replaced with a 10 mM phosphate buffer (pH 7.4, 140 mM NaCl) through an ultrafiltration film (10 kDa, 7500 × g, 20 min × 3). The resulting solution (2 mL) was passed through size-exclusion chromatography (Sephadex G-100) to remove unreacted monomers. The resulting solution (1 mL) and aqueous 40 mg/mL SDS solution (1 mL) were mixed and incubated for 5 minutes. The mixture was then added to an anion exchange resin (DEAE-Sephadex, 10 cm, 1.5 cm i.d.), and eluted by using a 10 mM Tris-HCl buffer (pH 7.4, 140 mM NaCl) as the eluate (step

2). The resultant was then purified by passing it through a desalination column (PD-10) (eluate: 10 mM phosphate buffer (pH 7.4, 140 mM NaCl)) to remove SDS.

[Table 15]

| | | Example 5 MIP-NGs | Comparative Example 4 NIP-NGs |
|---|---|---|---|
| **Template protein** | **Fc domain (IgG fragment)** | 2.50 mg (0.050 μmol) | - |
| **N-substituted or unsubstituted (melth) acrylamide** | N -Isopropylacrylamide (NIPAm) | 102 mg (0.90 mmol) | |
| **Biocompatible monomer** | 2-Metacryloyloxyethyl phosphorylcholine (MPC) | 3.69 mg (0.0125 mmol) | |
| **Crosslinker** | N, N' -Methylenbisacrylamide (MBAA) | 7.71 mg (0.050 mmol) | |
| **Label monomer** | Methacryloxyethyl Thiocarbamoyl Rhodamine B (MTRB) | 0.42 mg (0.625 μmol) | |
| **Functional monomer** | **Functional monomer represented by formula (51b)** | 15.73 mg (0.060 mmol) | |
| **Initiator** | 2-2'-Azobis(2-methyl propion amide) dihydrochloride (V-50) | 54.2 mg (0.20 mmol) | |
| **Solvent** | 10 mM Carbonate buffer 2% DMSO (pH 9.2) | 25 mL | |

When the particle sizes of the resulting MIP-NGs (Example 5) and NIP-NGs (Comparative Example 4) were measured in the same manner as Test Example 1, the presence of nanometer order particles was confirmed. Specifically, the particle size was 21 nm for the MIP-NGs (Example 5), and 18 nm for the NIP-NGs (Comparative Example 4) in terms of Z-average particle size. As for Z-potential, the MIP-NGs (Example 5) was found to be positively charged at 19 mV, and the NIP-NGs (Comparative Example 4) was found to be positively charged at 2.9 mV. It is understood that these charges originate from a secondary amino group (and polymerization initiator) of a functional monomer. This suggests that particles with a functional monomer site incorporated therein were synthesized. In view of the results of fluorescence measurement on the particle solution before and after purification ($\lambda_{ex}$ = 280 nm), fluorescence from tryptophan contained in the Fc region near 340 nm observed prior to purification was eliminated, which confirmed that most Fc domain fragments was able to be washed and removed.

Furthermore, 5 μL of ATTO647N NHS (10 mg/mL) DMSO solution was added to purified MIP-NGs (0.5 mg/mL, 1 mL) and incubated for 2 hours at 25°C (step 3). The post-reaction solution was subjected to ultrafiltration (10 kDa, 7500 × g, 20 min × 3) to remove unreacted fluorescent molecules and obtain the fluorescent group introduced MIP-NGs (Example 5). Introduction of fluorescence was confirmed from observation of a fluorescent peak near 670 nm, which was not observed before introduction, from measurement of fluorescence (excitation wavelength: 647 nm) before and after the step to introduce a fluorescent molecule (ATTO647N). The same step was performed for NIP-NGs to obtain the fluorescent group introduced NIP-NGs (Comparative Example 4)

9-4. Substrate for sensing use-4

A gold sputtered glass substrate (4.3 × 9.8 mm) was washed with pure water and EtOH, and then UV-$O_3$ (20 minutes) . The washed substrate was immersed in a 1 mM EtOH solution of 11-mercaptoundecanoic acid to introduce a carboxy group to the surface by utilizing self-assembling monomolecular film formation (25°C, 24 hours). After washing the substrate with EtOH, the substrate was immersed in an aqueous solution with 0.2 M EDC and 0.05 M NHS dissolved therein to activate the carboxy group on the surface (25°C, 1 hour). 50 μL of a solution of the fluorescent group introduced MIP-NGs (Example 5), or the fluorescent group introduced NIP-NGs (Comparative Example 4) (in PBS, 0.5mg/mL) was added to the substrate after the reaction to immobilize nanoparticles by amine coupling (25°C, 1 hour). An aqueous 1 M amino ethanol solution (50 μL) was then added dropwise to unreacted active ester for inactivation (25°C, 0.5 hours). Lastly, protein free blocking buffer (50 μL) was added dropwise for blocking of the surface (25°C, 0.5 hours). The fluorescence intensity on the surface before and after immobilization was measured. Since an increase in fluorescence

intensity was observed on the substrate after immobilization, immobilization of particles was confirmed.

9-5. Measurement of fluorescence

The ability to detect fluorescence of a protein of a prepared substrate for sensing use was studied by using a fluorescence microscope equipped with an automatic liquid handling robot. The measurement sequence conditions were the following.

Fluorescence microscope

Filter: Cy5 Objective lens: ×5, Exposure time: 0.1 seconds
Quantity of light: 12%, Light source: mercury lamp
ROI: 3 × 5 = 15 locations at the center of the substrate

*Sequence of automatic dispenser

1. Tip attachment
2. Sample suction: 150 μL
3. Reaction: 5 minutes (25°C)
4. Keep measurement position, Repeat 2→4

Figure **18** shows the change in relative fluorescence intensity upon addition of an Fc domain fragment (0 to 1600 nM), which is a template protein, to the prepared substrate for sensing use. Since a greater change in fluorescence was exhibited in an MIP-NG (Example 5) immobilized substrate relative to an NIP-NG (Comparative Example 4) immobilized substrate, it was suggested that a binding cavity of an Fc domain is formed by molecular imprinting in the MIP-NGs (Example 5), and a functional monomer is incorporated into the cavity, and a bond of an Fc domain fragment was able to be converted into a fluorescent signal by applying a fluorescent label by a post-imprinting modification. The apparent dissociation constant ($K_d$) was calculated to be $9.8 \times 10^{-9}$ M from the resulting ratio of change in fluorescence.

The protein selectivity of the prepared substrate for sensing use was tested by using an Fc domain fragment and a whole IgG and Lysozyme (Lyz) as reference proteins. Figure **19** shows fluorescent responses upon addition of each reference protein (100 nm), with the change in relative fluorescence intensity upon addition of an Fc domain fragment (100 nm) as 1. The greatest change in fluorescence was exhibited upon addition of an Fc domain fragment in the MIP-NGs (Example 5). Since about 70% of whole IgG having an Fc domain fragment as a partial structure thereof also responded, it was suggested that an Fc site of IgG can be recognized. Specifically, it is demonstrated that the MIP-NGs (Example 5) prepared by using an Fc domain fragment as a template can also detect IgG via capture of an Fc domain. Meanwhile, whole IgG and Lyz both exhibited a greater fluorescent response than an Fc domain fragment in the NIP-NGs (Comparative Example 4). It is inferred therefrom that a functional monomer residue and a fluorescent molecule introduced thereto that are randomly present on the surface of NIP-NGs (Comparative Example 4) were bound non-specifically. The pI of whole IgG is up to 8.5 and that of Lyz is 11.2, which are positively charged in a neutral solution. Thus, it is understood therefrom that electrostatic interaction with a boronylaryl group of a functional monomer, a hydrogen bond with an amide bond, etc. contributes to a bond.

**[Test Example 10]**

This Test Example prepared a particle having a molecularly imprinted recess targeting HER2 (biological material) for sensing use and having a functional group having an anti-HER2 affibody that interacts with HER2 (group that interacts with a biological material) and a signal substance ATTO647N in the molecularly imprinted recess as an HER2 capture mediated exosome (exosome expressed by HER2 overexpressing breast cancer cell line SK-BR-3 cells) nanoparticle for sensing use.

10-1. Modification of template (step 11)

A template HSA modified with a methacryloyl group (polymerizable functional group) via a disulfide bond (reversible linking group) was prepared in the following manner.

10-1-1. Reagent

*Albumin, from Human Serum (HSA; human serum albumin) (Wako Pure Chemical Industries)
*Compound (5) synthesized by the following method

[Chemical Formula 19]

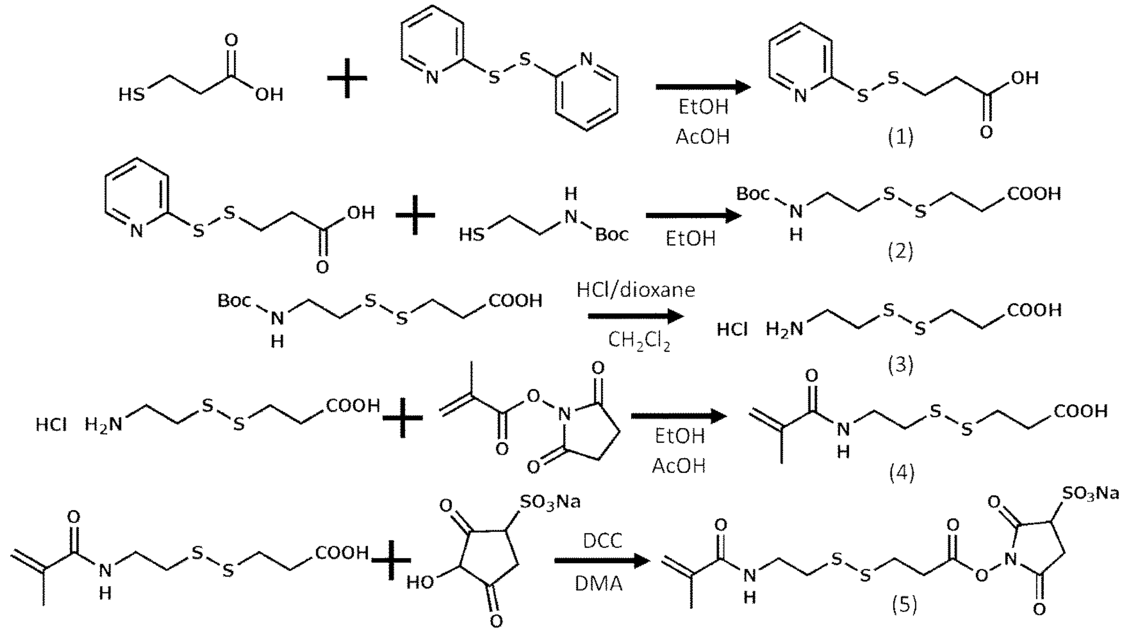


3-mercaptopropionic acid (0.900 g, 8.50 mmol) was dissolved in 20 mL of ethanol, and 1.6 mL of acetic acid was added. 2,2-dipyridyl disulfide (3.75 g ,17 mmol) dissolved in 30 ml of ethanol was added dropwise thereto while stirring at room temperature. The mixture was allowed to react for 3 hours at 25°C to obtain Compound (1).

Purified compound (1) (1.43 g, 3.97 mmol) was dissolved in 15 mL of ethanol, and then 2-(Boc-amino) ethanethiol (1.69 mL, 9.99 mmol, 2 eq) dissolved in 20 mL of ethanol was added. The mixture was allowed to react for 3 hours at room temperature to obtain Compound (2).

4N HCl dioxane (6.60 mL, 25.5 mmol, 5 eq) dissolved in 10 mL of $CH_2Cl_2$, was added dropwise to purified compound (2) (1.43 g, 5.10 mmol) dissolved in 5mL of $CH_2Cl_2$ while stirring and cooling with ice. The reaction was then allowed to take place overnight at room temperature to obtain compound (3).

Purified compound (3) (531.6 mg, 2.45 mmol) was dispersed in 5 mL of $CH_2Cl_2$, and DIEA (1.28 mL, 7.35 mmol, 3 eq) was added. 3.5 mL of $CH_2Cl_2$ with N-succinimidyl methacrylate (670 mg, 3.68 mmol, 1.5 eq) dissolved therein was added dropwise thereto under a nitrogen atmosphere and reacted overnight at room temperature to obtain compound (4).

Purified compound (4) (290 mg, 1.16 mmol, 1.1 eq), sulfo-NHS (230 mg, 1.1 mmol), and DCC (304.5 mg, 1.54 mmol, 1.4 eq) were dissolved in 5 mL of DMA and reacted for 24 hours at room temperature. After the reaction, the solution was cooled to 4°C, and the precipitate was filtered and removed. The filtrate was then subjected to vacuum evaporation to removed DMA. The resulting white solid was dissolved in AcOEt and re-precipitated by adding hexane thereto to obtain compound (5).
($^1$H-NMR chart 7 (500 MHz,$CD_3OD$): $\delta$ = 8.1 (t, 1H, -CO-NH-C-) , 5.7-5.3 (s, 2H, CH3-C = $CH_2$), 4.0-3.8 (d, 1H, -CO-CH-$SO_3$Na), 3.4-3.3 (m, 2H, $NaSO_3$-CH-$CH_2$-) , 3.3-2.7 (m, 8H, -NH-$CH_2$-$CH_2$-S-S-$CH_2$-$CH_2$-) , 1.85 (s, 3H, $CH_3$-C = $CH_2$))

10-1-2. Experimental procedure

2 mL of 10 mM phosphate buffer (pH 7.4) with HSA (100 mg, 2.23 μmol) dissolved therein and 1 mL of 10 mM phosphate buffer (pH 7.4) with compound (5) (20 mg, 44.6 μmol, 20 eq) dissolved therein were mixed and reacted

overnight at 4°C. The mixture was then washed three times with Amicon Ultra-4 10 kDa (4°C, 7500 G, 20 min) to obtain a template HSA modified with a methacryloyl group (polymerizable function group) via a disulfide bond (reversible linking group).

10-2. Synthesis of molecularly imprinted polymer (step 12)

Molecularly imprinted polymer particles were synthesized in the following manner.
Specifically, particulate MIP-NGs of molecularly imprinted polymer were synthesized by 16 hours of emulsifier-free precipitation polymerization at 70°C under a nitrogen atmosphere in a 50 mL Schlenk flask in accordance with the polymerization recipe in the following table.

[Table 16]

| Reagent | Amount used |
|---|---|
| **Methacryloyl group modified HSA** | 18.3 mg (0.30 μmol) |
| N-Isopropylacrylamide (NIPAm) | 610 mg (5.40 mmol) |
| 2-Methacryloyloxyethyl Phosphorylcholine (MPC) | 88.5 mg (0.30 mmol) |
| N,N'-Methylenebisacrylamide (MBAA) | 46.2 mg (0.30 mmol) |
| 2-2'-Azobis(2-methyl propionamide) dihydrochloride (V-50) | 316 mg (1.20 mmol) |
| PBS (10 mM phosphate, 140 mM NaCl, pH7.4) | 50 mL |

10-3. Removal of template (step 13)

A template was removed from a molecularly imprinted polymer.
The solvent was replaced with pure water by ultrafiltration (25°C, 7500 g, 20 minutes, three times) using a dialysis tube of Amicon Ultra-4 (10 kDa) for a PBS solution comprising a complex of particulate MIP-NGs of molecularly imprinted polymer and template HSA. Tris(2-carboxyethyl)phosphine Hydrochloride (TCEP) was added to the complex until reaching 20 mM and reacted overnight at 25°C. The reaction reduced and cleaved the disulfide bond (reversible linking group) between the template HSA and MIP-NGs to expose a thiol group.
Furthermore, TCEP was removed and the solvent was substituted with 10 mM Tris HCl buffer (pH 7.4) containing 140 mM NaCl by sorting using a size exclusion chromatography packing material Sephadex G-100 and ultrafiltration (25°C, 7500 g, 20 minutes, three times) using a dialysis tube of Amicon Ultra-4 (10 kDa). Furthermore, the template HSA was removed by anion exchange chromatography, whereby particulate molecularly imprinted polymer (MIP-NG) were obtained.

10-4. Immobilization of particles on a substrate

The resulting particulate MIP-NGs of molecularly imprinted polymer were immobilized on a substrate in the following manner.
An SPR gold substrate was washed with pure water and ethanol, then immersed in an ethanol solution prepared so that 11-Mercaptoundecanoic acid would be 1 mM, and incubated overnight at 25°C. The substrate after the reaction was washed with ethanol and pure water, dried with nitrogen gas, then injected with a PBS solution of 0.1 M N-Hydroxysuccinimide (NHS) and 0.4 M 1-(3-dimethylaminopropyl)-3-ethylcarbodiimide hydrochloride (EDC) by using Biacore 3000, and incubated for 10 minutes at 25°C. The resulting substrate (with its surface reformed with a carboxylic acid active ester group as a binding group) was injected with 400 μg/mL of MIP-NGs and incubated for 10 minutes at 25°C to obtain a substrate with MIP-NGs immobilized thereon.

10-5. Synthesis of functional molecule

A functional molecule (following compound (3)) having a function group comprising a group for binging a group that interacts with a biological material and a signal substance binding group used in the following section 10-6 was synthesized in accordance with the following scheme.

[Chemical Formula 20]

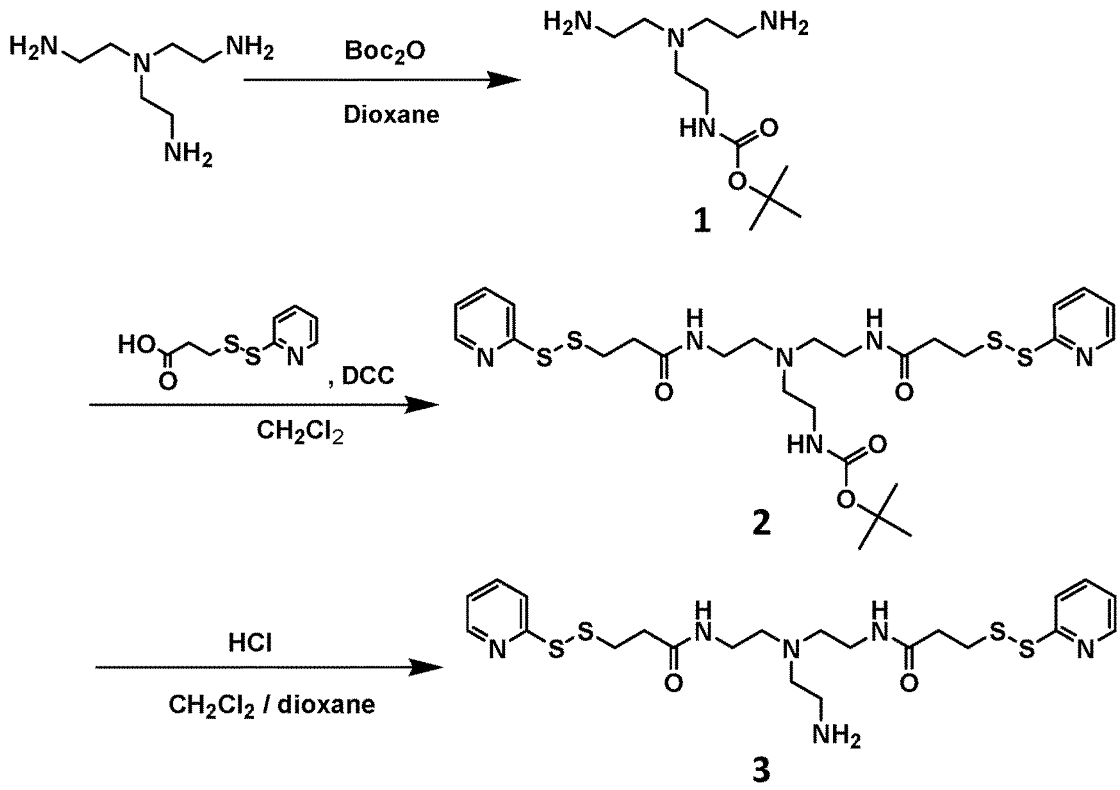


1

2

3

1800 μL (12 mmol) of Tris(2-aminoethyl)amine was dissolved in dioxane (4 mL) and stirred while cooling with ice. Dioxane (10 mL) with 440 mg (2.0 mmol) of Boc$_2$O dissolved therein was slowly added dropwise thereto, and the mixture was stirred overnight (0°C→room temperature). The post-reaction solvent was subjected to vacuum evaporation. The residue was dissolved in Milli-Q water and extracted (x4) with $CH_2Cl_2$ (15 mL) . The organic layer was dried with $Na_2SO_4$ and the solvent was subjected to vacuum evaporation with a rotary evaporator and vacuum dried to obtain a transparent and oily compound of interest (yield: 364 mg, 74 %)

$^1$H-NMR (300 MHz, $CDCl_3$) δ = 5.29 (br, 1H, carbamide), 3.18 (t, 2H, -$CH_2$-NH-Boc), 2.75 (t, 4H, $H_2N$-$CH_2$-), 2.53 (m, 6H, - $CH_2$-) , 1.45 (s, 9H, Boc)

323 mg (1.5 mmol) of (3-Pyridyldithio)propionic acid and 330 mg (1.6 mmol) of DCC were dissolved in $CH_2Cl_2$ (10 mL) and stirred while cooling with ice. 130 mg (0.53 mmol) of compound (1) was added thereto, and the mixture was stirred for another hour. After the reaction, the precipitate was filtered and removed, and the solvent was subjected to vacuum evaporation with a rotary evaporator. The residue was purified by silica gel chromatography (EA/Hx = 45/55→100/0, EA/MeOH = 99/1→80/20) to obtain compound (2) (yield: 230 mg, 68 %)

$^1$H-NMR (300 MHz, $CDCl_3$) δ= 8.44 (d, 2H, pyridyl), 7.70-7.60 (m, 4H, pyridyl), 7.18 (br, 2H, amide), 7.12-7.07 (m, 2H, pyridyl), 5.01 (br, 1H, carbamide), 3.34-3.29 (m, 4H, $H_2N$-$CH_2$-), 3.14-3.07 (m, 6H, -$CH_2$-NH-Boc, -$CH_2$-), 2.68 (t, 4H, -$CH_2$-), 2.61-2.51 (m, 6H, -$CH_2$-), 1.43 (s, 9H, Boc)

230 mg (0.36 mmol) of compound (2) was dissolved in $CH_2Cl_2$ (5 mL). 0.5 mL of 4 N HCl in dioxane was added thereto, and the mixture was stirred while cooling with ice. After stirring overnight (0°C→room temperature), an excessive amount of diethyl ether was added, and the precipitate was separated by decantation. The resulting solid was washed with diethyl ether and vacuum dried to obtain the compound of interest (compound (3)) (yield: 255 mg, quant.).

$^1$H-NMR (300 MHz, $D_2O$) δ = 8.51 (d, 2H, pyridyl), 8.14 (t, 2H, pyridyl), 8.01 (d, 2H, pyridyl), 7.56 (m, 2H, pyridyl), 3.67-3.3.56 (m, 6H, HN-$CH_2$-), 3.43 (t, 6H, -$CH_2$-N) , 3.09 (t, 4H, -$CH_2$-), 2.74 (t, 4H, -$CH_2$-)

10-6. Introduction of a functional group (step 14)

A functional molecule was introduced in a binging group of a molecularly imprinted recess via a disulfide bond (reversible linking group) in the following manner.

The substrate with particulate MIP-NGs of molecularly imprinted polymer immobilized thereon obtained in 10-4 was immersed in 900 μg/mL PBS solution of the functional molecule (compound (3)) obtained in 10-5 and reacted for 2 hours at room temperature to convert a pyridyl disulfide group of the functional molecule and a thiol group of the molecularly imprinted polymer into a disulfide bond (reversible linking group) .

10-7. Introduction of affibody and fluorescent substance

An anti-HER2 affibody molecule (molecule providing a group that interacts with a biological material) and a fluorescent substance (signal substance) were introduced to the substrate obtained in 10-6 in the following manner.
10 μL of 1 mg/mL solution (10 mM PB, 137 mM NaCl, 0.05 vol% Tween20 (pH 7.4); PBST) of Anti-Her2 Affibody(R) Molecule (anti-Her2 affibody molecule, Abcam) was added to 40 μL of 20 mM DTT solution (50 mM PB, 137 mM NaCl (pH7.5)) and reacted for 2 hours at 25°C. The mixture was then subjected to ultrafiltration (4°C, 14000 × g, 15 minutes) three times by using Amicon Ultra-0.5 (MWCO: 10 kDa) and purified with PBST, whereby a molecule providing an anti-Her2 affibody was obtained.
10 μg/mL PBS solution of an anti-Her2 affibody molecule (molecule providing a group that interacts with a biological material) was reacted with the substrate obtained in 10-6 (room temperature, 2 hours) to introduce an anti-Her2 affibody group (group that interacts with a biological material) to a pyridyl disulfide group (group for binding a group that interacts with a biological material). 100 pM of 2-mercaptoethanol solution was then reacted to cap a remaining pyridyl disulfide group. The substrate was then reacted with an Atto647N-NHS solution (room temperature, 2 hours) to introduce Atto647N (signal group) to an amino group (signal substance binding group), whereby a substrate for sensing use, which has a molecularly imprinted recess using HER2 as a template and has a functional group having an anti-HER2 affibody that interacts with HER2 and a signal substance ATTO647N immobilized in the molecularly imprinted recess.

10-8. Analysis of target of detection by measurement of fluorescence

An exosome capture test utilizing a fluorescence microscope equipped with an automatic SIC dispenser was conducted by using the substrate for sensing use obtained in 10-7.

10-8-1. Experimental procedure

An exosome solution was prepared by dissolving an exosome expressed by SK-BR-3 cells in PBS (10 mM phosphate, 140 mM NaCl, pH 7.4) at a concentration of 0 to $1.0 \times 10^{-13}$ M. The prepared exosome solution was added dropwise to a sensor for analyzing a target of detection obtained in 10-7.
The fluorescence microscope measurement conditions were measurement points: 3 points on the substrate; filter: Cy5; objective lens: 5×; exposure time: 0.1 seconds; quantity of light: 100%; and light source: mercury lamp.

10-8-2. Results

The relative fluorescence intensity was calculated to study the relationship between the relative fluorescence intensity and exosome concentration. Figure **20** shows the results.
As shown in Figure **20,** an exosome concentration dependent increase in the degree of quenching was confirmed for the substrate for sensing use obtained in 10-7. Specifically, an ability to recognize exosome expressed by SK-BR-3 cells was demonstrated. As a result of calculating dissociation constant $K_d$ by curve fitting, the dissociation constant $K_d$ was $1.0 \times 10^{-17}$ (M).
Another substrate for sensing use was obtained in the same manner as above, except for using an anti-HSA affibody in place of an anti-HER2 affibody. Figure **21** shows the change in relative fluorescence intensity when an exosome ($5 \times 10^{-17}$ M) expressed by HER2 overexpressing breast cancer cell line SK-BR-3 cells was added to a substrate for sensing use introduced with an anti-HER2 affibody and another substrate for sensing use introduced with an anti-HSA affibody.
As shown in Figure **21,** an exosome expressed by SK-BR-3 cells was specifically detected by an HER2 capture mediated exosome substrate for sensing use with an anti-HER2 affibody introduced thereto.

[Reference Signs List]

| | |
|---|---|
| **1, 1a** | substrate for sensing use |
| **10, 10a** | nanoparticle for sensing use |
| **20** | molecularly imprinted polymer |
| **21** | molecularly imprinted cavity |
| **$R^1$** | group that interacts with a biological material |
| **$R^2$** | linking group comprising a signal substance binding group |

| | |
|---|---|
| **22** | functional group |
| **F22** | functional monomer |
| **F23** | acrylamide |
| **F24** | biocompatible monomer |
| **30** | signal substance |
| **31** | signal group |
| **40** | substrate |
| **90** | biological material (targeted for sensing use) |
| **90'** | biological material (template) |

**Claims**

1. A nanoparticle for sensing use comprising a molecularly imprinted polymer having a molecularly imprinted cavity for a biological material,

   wherein the molecularly imprinted polymer comprises a constituent unit derived from a functional monomer having a functional group comprising a group that interacts with the biological material and a signal substance binding group that is different from the group that interacts with the biological material, and
   wherein the functional groups are present on a surface of the molecularly imprinted cavity.

2. The nanoparticle for sensing use of claim 1, wherein the functional group is represented by formula (1):

   [Chemical Formula 1] $\mathbf{-R^2-L^1-R^1}$ **(1)**

   wherein $R^1$ represents the group that interacts with the biological material, $R^2$ represents a linking group comprising the signal substance binding group, and $L^1$ represents a direct bond or a linking group.

3. The nanoparticle for sensing use of claim 1 or 2, wherein the group that interacts with the biological material is selected from the group consisting of a substituted or unsubstituted amino group, a substituted or unsubstituted aromatic group, an amidino group, a guanidino group, a carboxyl group, a sulfo group, a boronyl group, and a ligand of the biomolecule.

4. The nanoparticle for sensing use of any of claims 1 to 3, wherein the signal substance binding group is selected from the group consisting of an amino group, a carboxyl group, a thiol group, a disulfide group-containing group, an aminooxy group, an aldehyde group, and a hydroxyl group.

5. The nanoparticle for sensing use of any of claims 1 to 4, wherein the functional group is represented by formula (11) or (12):

   [Chemical Formula 2]

   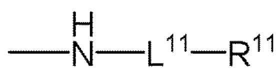

   (11)

   wherein $R^{11}$ represents a carboxylaryl group or a sulfoaryl group, and $L^{11}$ represents an alkylene group with 1 to 4 carbons, or

   [Chemical Formula 3]

   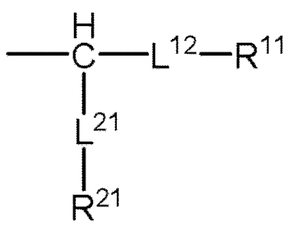

   (12)

wherein $R^{11}$ represents a carboxylaryl group or a sulfoaryl group, $L^{12}$ represents a divalent linking group, $R^{21}$ represents an amino group, a carboxyl group, a thiol group, a disulfide group-containing group, an aminooxy group, an aldehyde group, or a hydroxyl group, and $L^{21}$ represents a divalent linking group.

**6.** The nanoparticle for sensing use of any of claims 1 to 5, comprising a signal group bound to the signal substance binding group.

**7.** The nanoparticle for sensing use of any of claims 1 to 6, wherein the molecularly imprinted polymer comprises a constituent unit derived from an N-substituted or unsubstituted (meth)acrylamide and/or a biocompatible monomer.

**8.** The nanoparticle for sensing use of any of claims 1 to 7, wherein the biological material is selected from the group consisting of a protein, a sugar chain, a lipid, and a composite molecule of two or more thereof.

**9.** The nanoparticle for sensing use of any of claims 1 to 8, wherein the biological material is albumin.

**10.** The nanoparticle for sensing use of any of claims 1 to 9, which is used for the detection of the biological material.

**11.** The nanoparticle for sensing use of any of claims 1 to 9, wherein the biological material is a substance adhering to or bound to a biological membrane of a membrane structure, a molecule corresponding to an exposed partial structure of the biological membrane, or a fragment of a specific biomolecule, and is used for the detection of the membrane structure or the specific biomolecule via capture of the biomolecule by the group that interacts with the biological material.

**12.** The nanoparticle for sensing use of claim 11, wherein the membrane structure is a cell, a virus, an extracellular vesicle, or an organelle, and the specific biomolecule is an antibody.

**13.** The nanoparticle for sensing use of any of claims 1 to 12, wherein the biological material is a biological material derived from an animal or plant, which falls under haram, and is used to verify halal.

**14.** A substrate for sensing use, comprising a substrate, and the nanoparticle for sensing use of any of claims 1 to 13 immobilized on the substrate.

**15.** A reagent for sensing use, comprising the nanoparticle for sensing use of any of claims 1 to 13.

**16.** A method of manufacturing a nanoparticle for sensing use, comprising:

step 1 of synthesizing a molecularly imprinted polymer by polymerizing monomer components comprising a functional monomer having a functional group comprising a group that interacts with a biological material and a signal substance binding group that is different from the group that interacts with the biological material by using the biological material as a template; and
step 2 of removing the template from the molecularly imprinted polymer.

**17.** The method of manufacturing a nanoparticle for sensing use of claim 16, comprising, after step 2, step 3 of binding a signal substance to the signal substance binding group in the molecularly imprinted polymer.

FIG.1

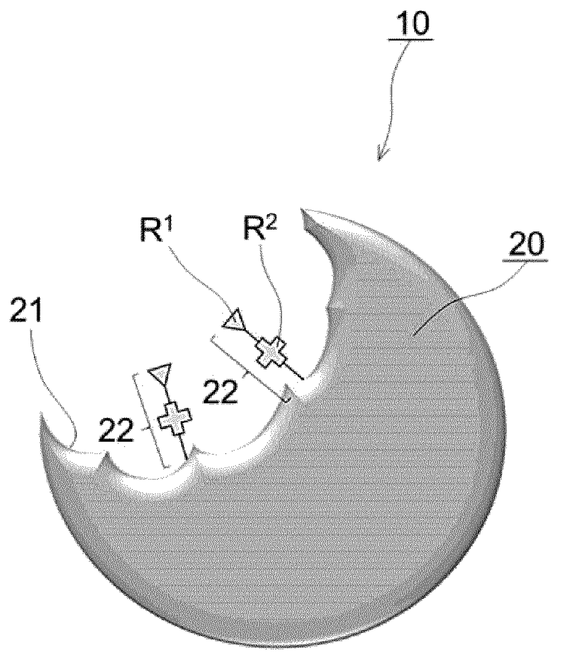


FIG.2

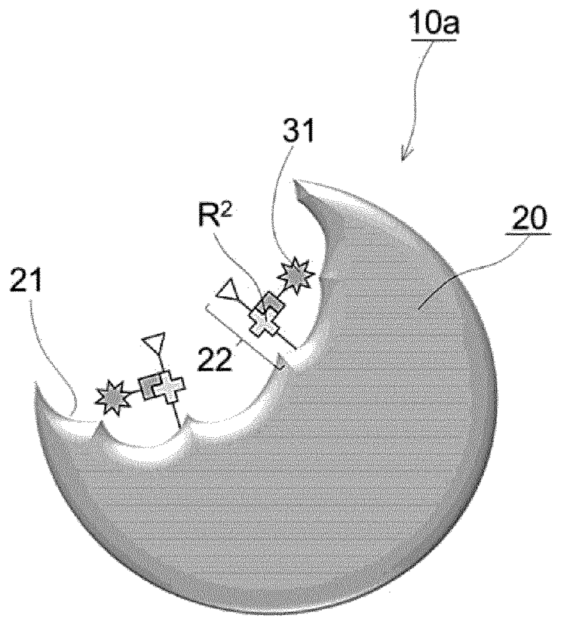

FIG.3

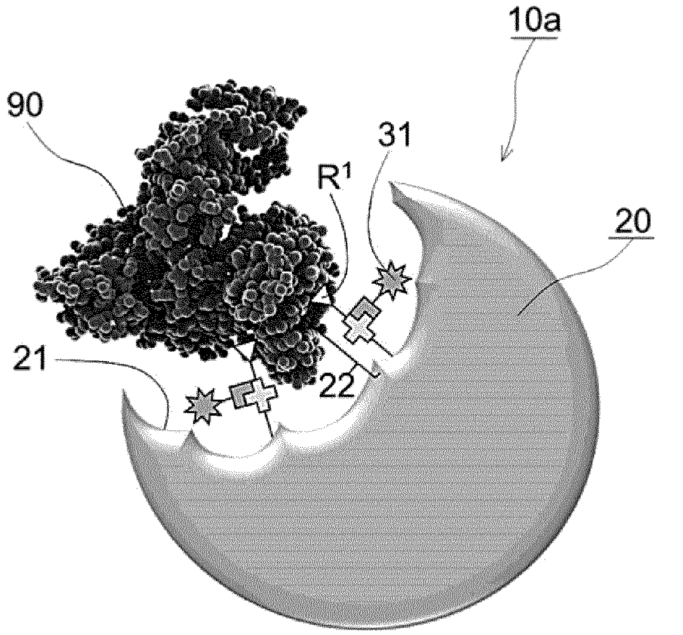


FIG.4

<Step 1>

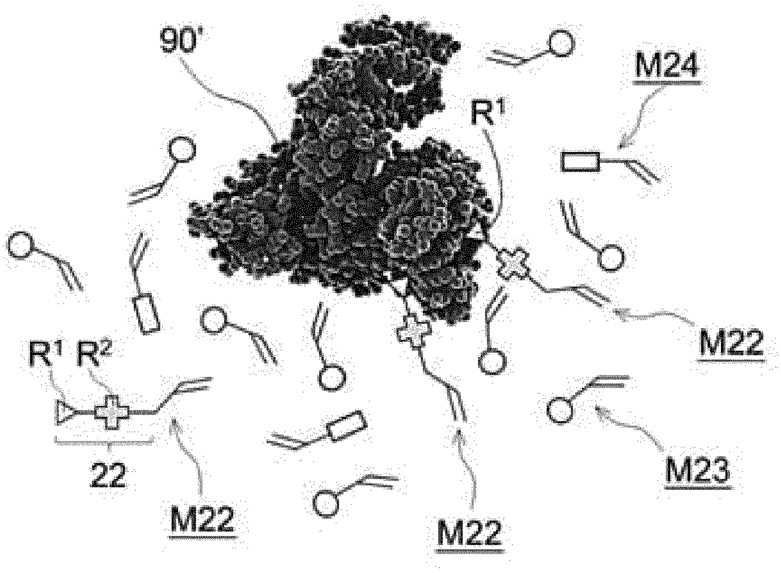

FIG.5

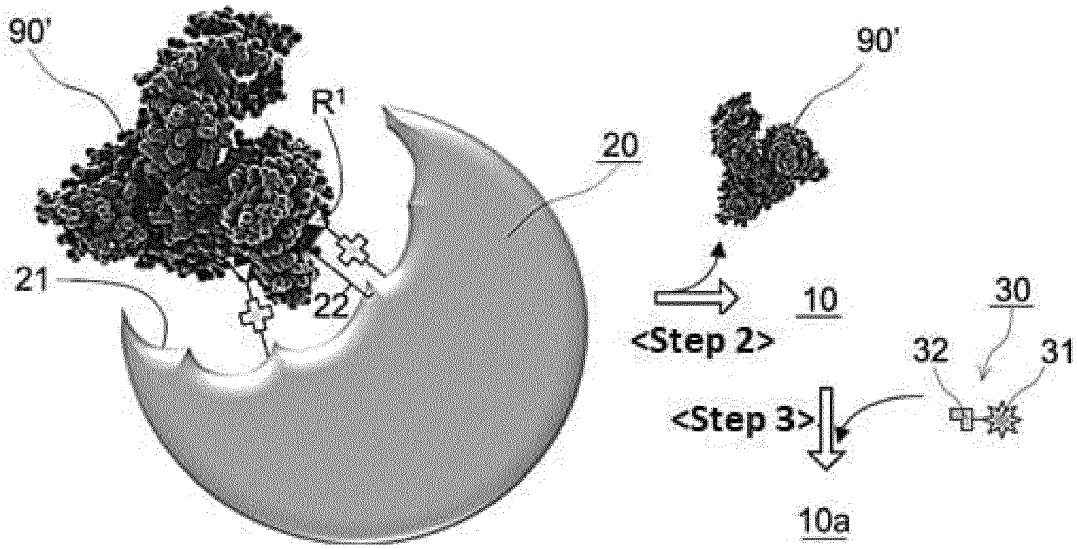
90'
R1
20
90'
21
22
10
<Step 2>
30
32
31
<Step 3>
10a

FIG.6

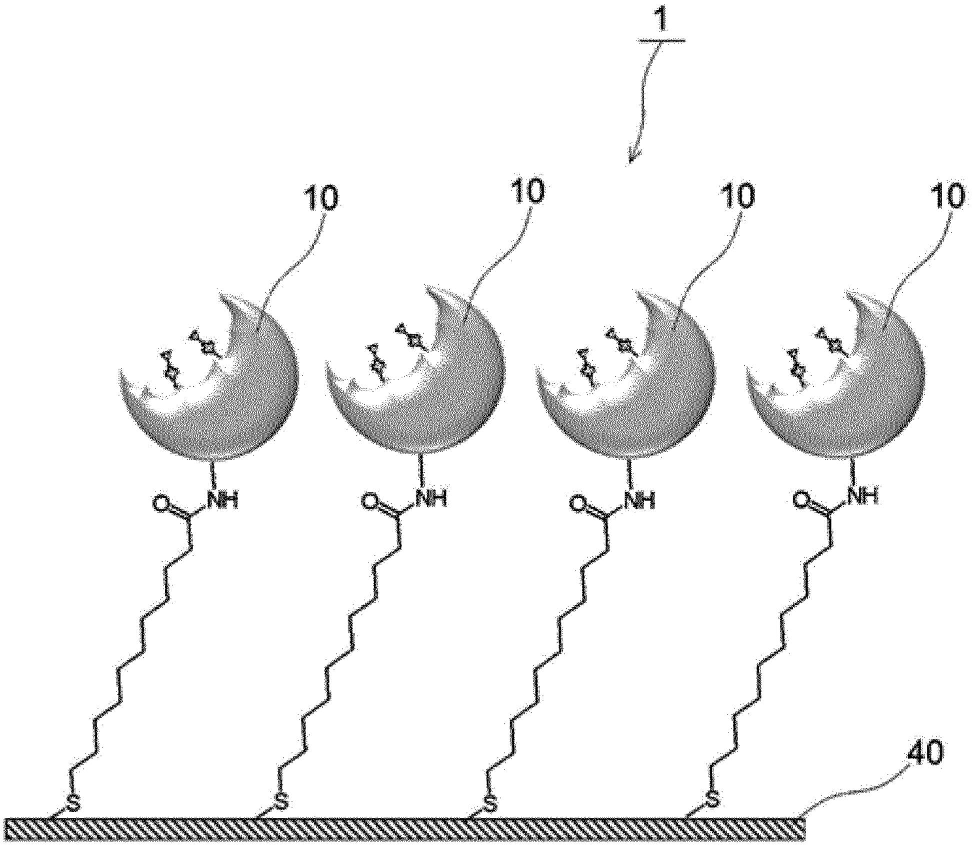
1
10
10
10
10
NH
NH
NH
NH
S
S
S
S
40

FIG.7

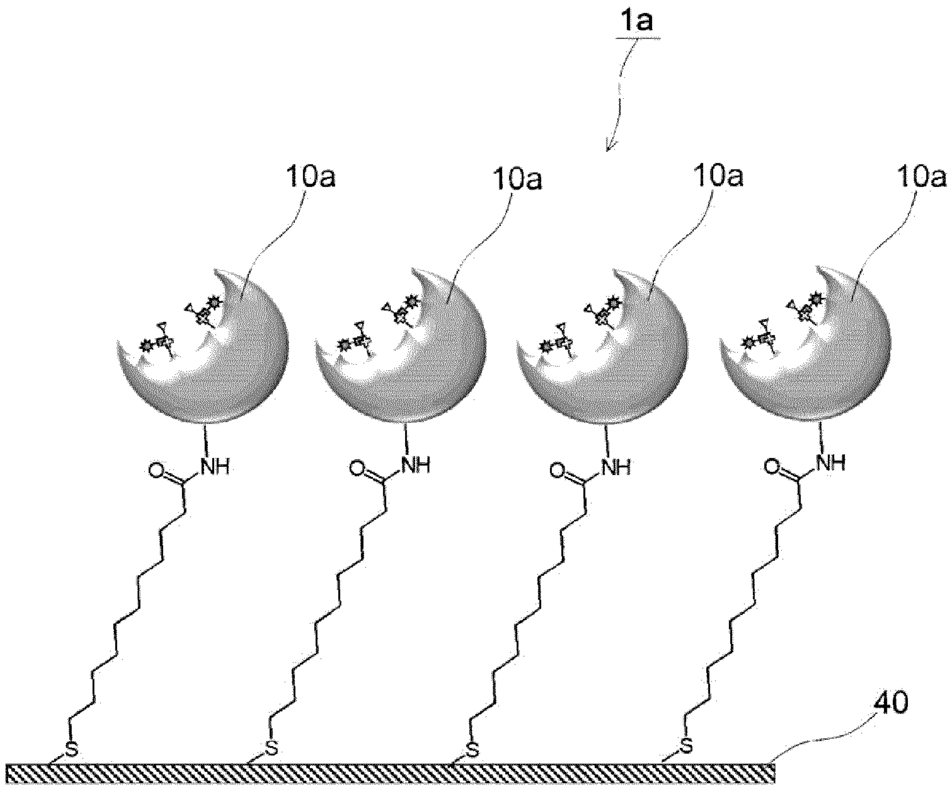
1a
10a
10a
10a
10a
NH
NH
NH
NH
O
O
O
O
S
S
S
S
40

FIG.8

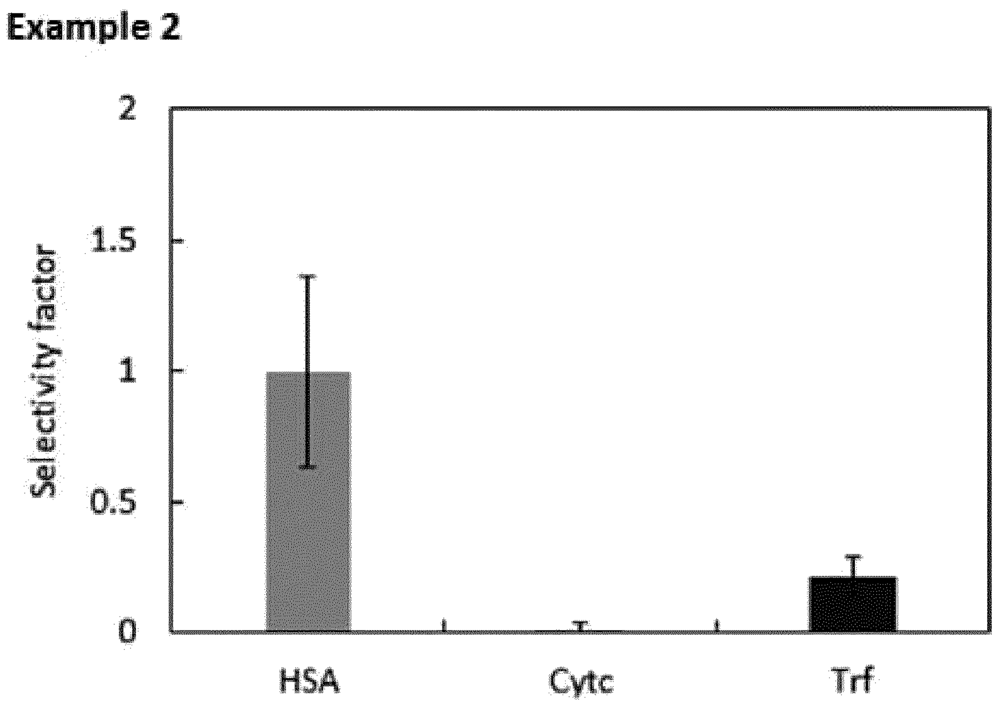
Example 2
2
1.5
1
0.5
0
Selectivity factor
HSA
Cytc
Trf

FIG.9

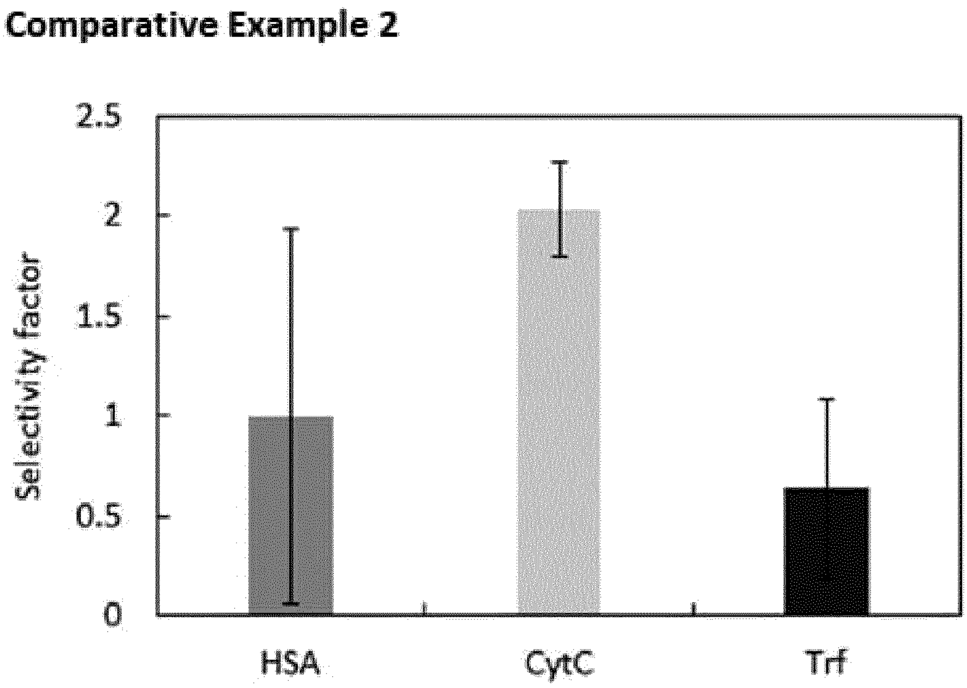

Comparative Example 2
Selectivity factor
2.5
2
1.5
1
0.5
0
HSA
CytC
Trf


FIG.10

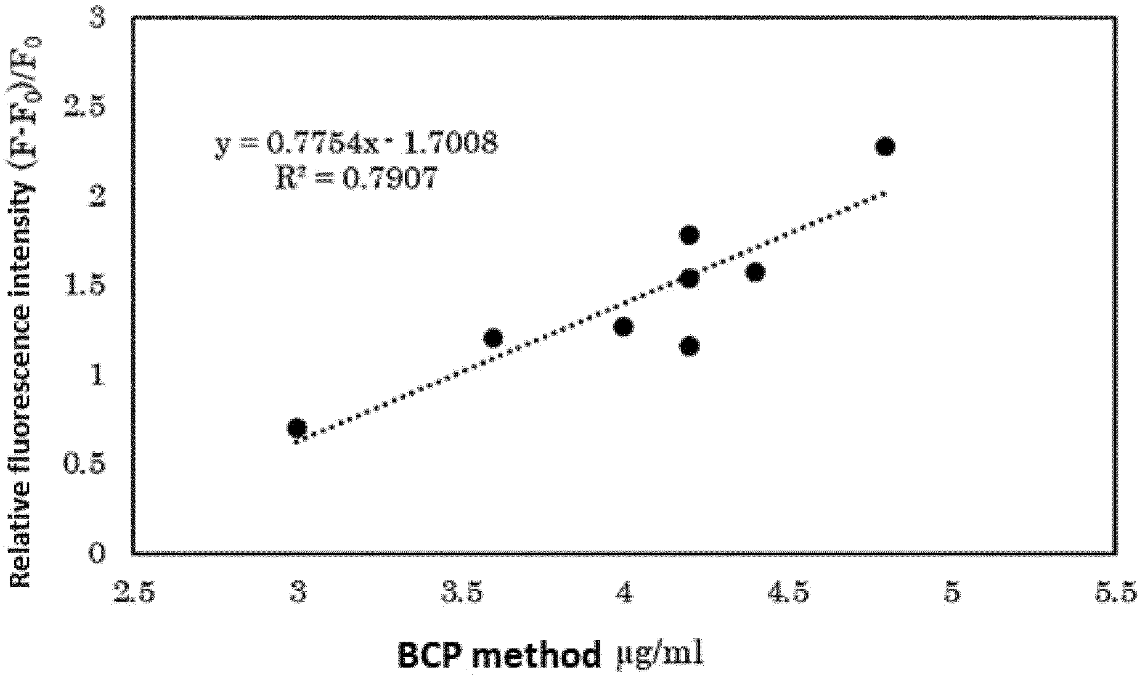

y = 0.7754x - 1.7008
R² = 0.7907
Relative fluorescence intensity (F-F0)/F0
3
2.5
2
1.5
1
0.5
0
2.5
3
3.5
4
4.5
5
5.5
BCP method μg/ml

FIG.11

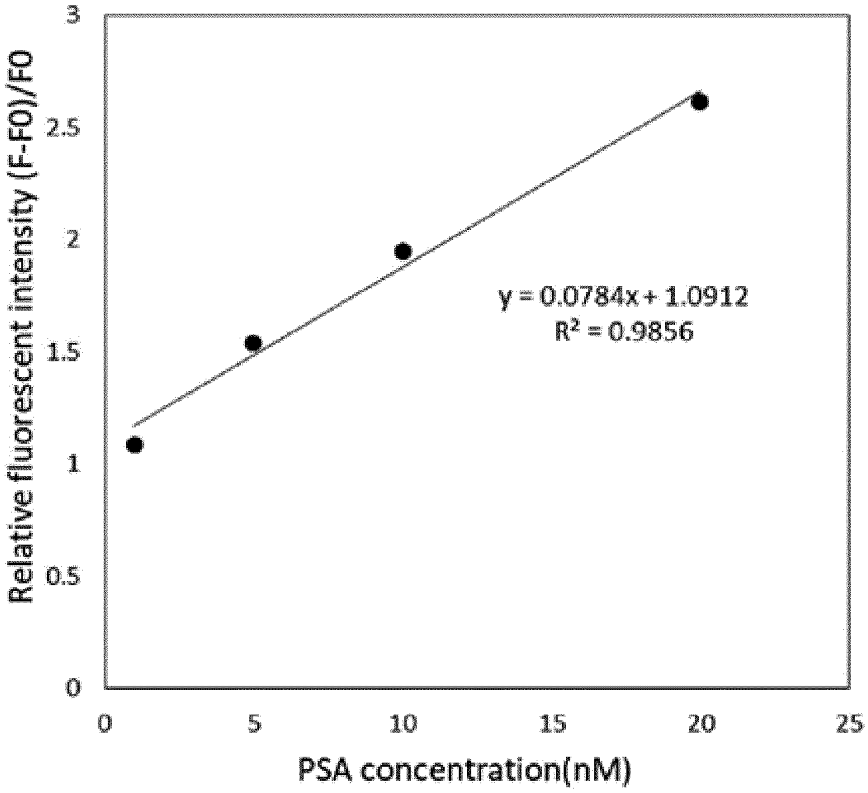
3
2.5
2
1.5
1
0.5
0
Relative fluorescent intensity (F-F0)/F0
y = 0.0784x + 1.0912
R² = 0.9856
0
5
10
15
20
25
PSA concentration(nM)

FIG.12

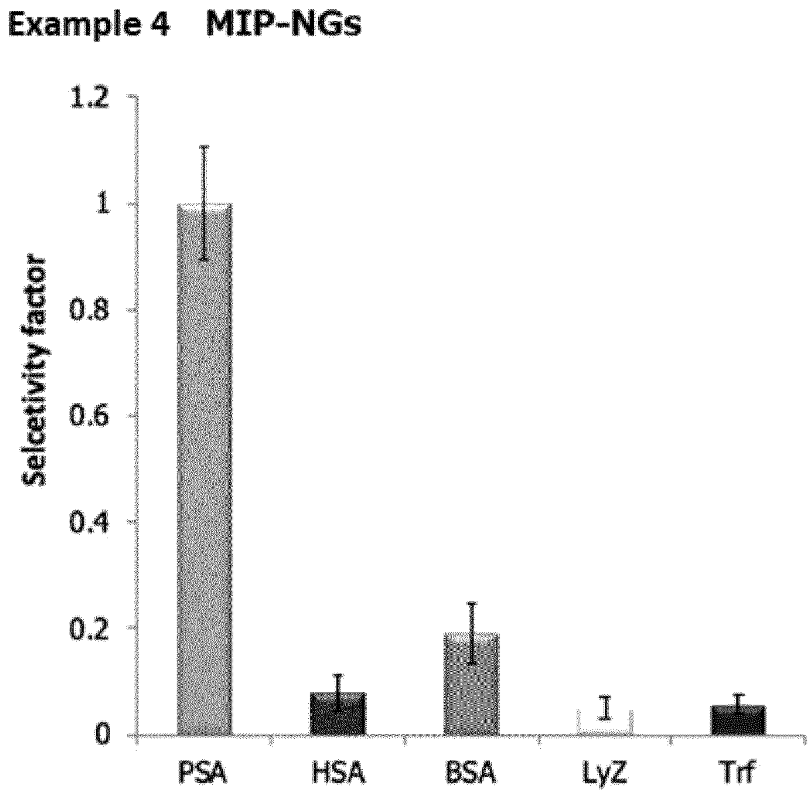
Example 4 MIP-NGs
1.2
1
0.8
0.6
0.4
0.2
0
Selcetivity factor
PSA
HSA
BSA
LyZ
Trf

FIG.13

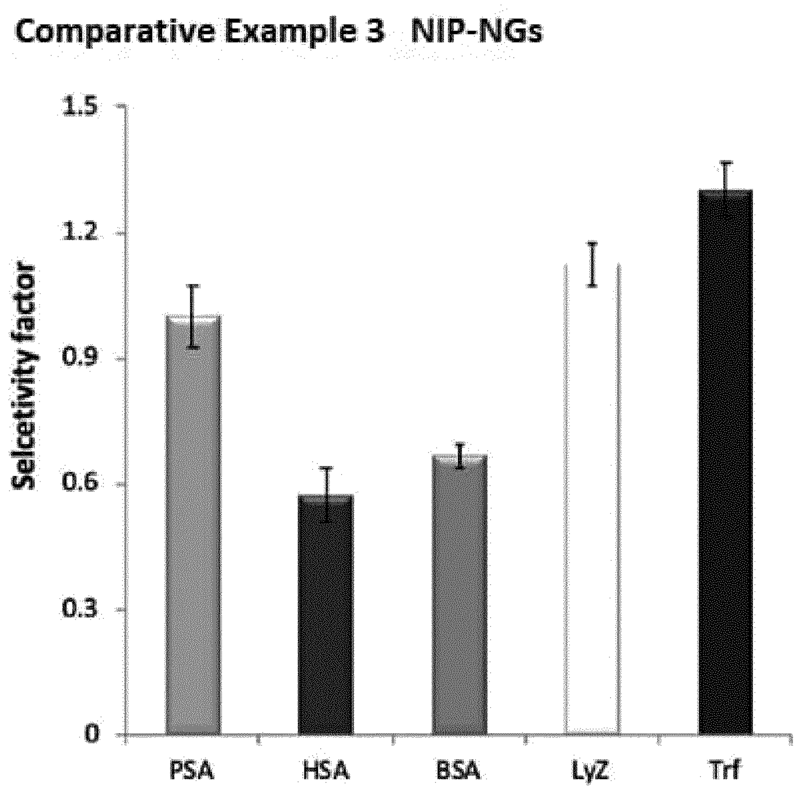
Comparative Example 3 NIP-NGs
Selcetivity factor
1.5
1.2
0.9
0.6
0.3
0
PSA
HSA
BSA
LyZ
Trf

FIG.14

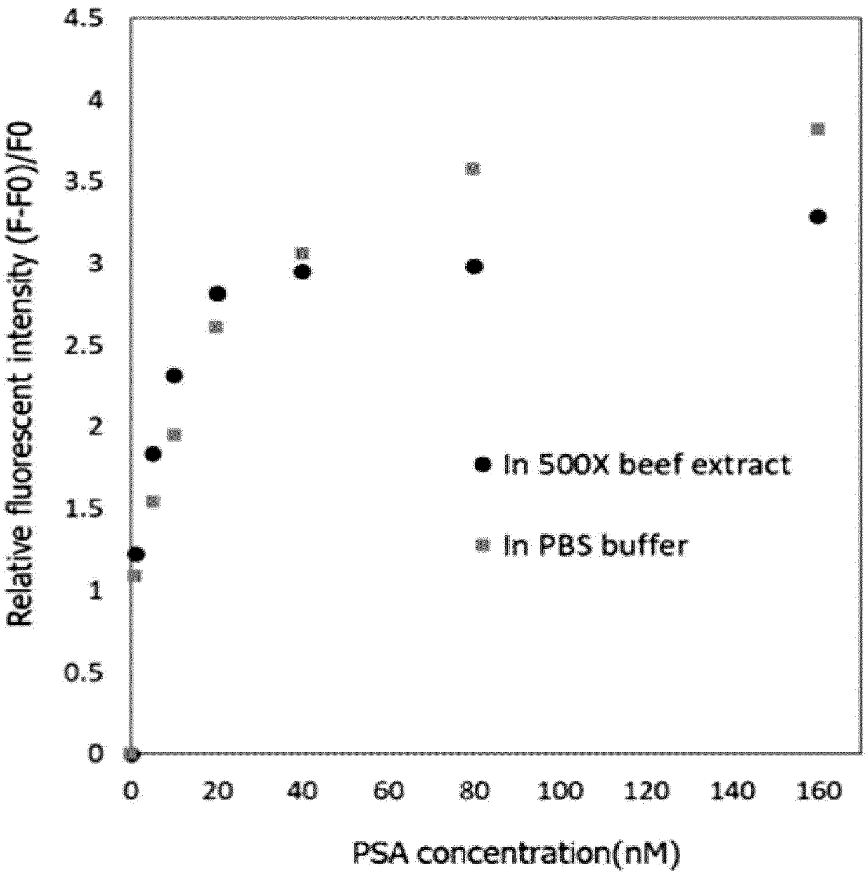
Relative fluorescent intensity (F-F0)/F0
4.5
4
3.5
3
2.5
2
1.5
1
0.5
0
0
20
40
60
80
100
120
140
160
PSA concentration(nM)
In 500X beef extract
In PBS buffer

FIG.15

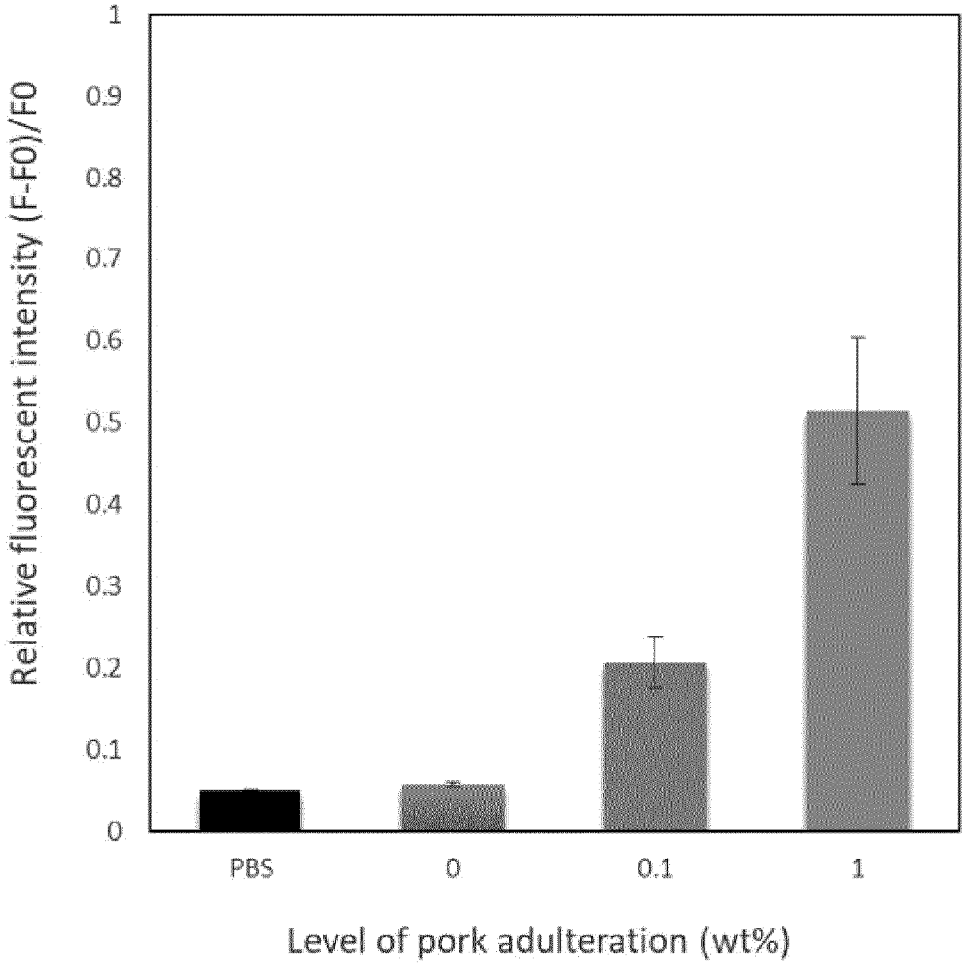
Relative fluorescent intensity (F-F0)/F0
1
0.9
0.8
0.7
0.6
0.5
0.4
0.3
0.2
0.1
0
PBS
0
0.1
1
Level of pork adulteration (wt%)

FIG.16

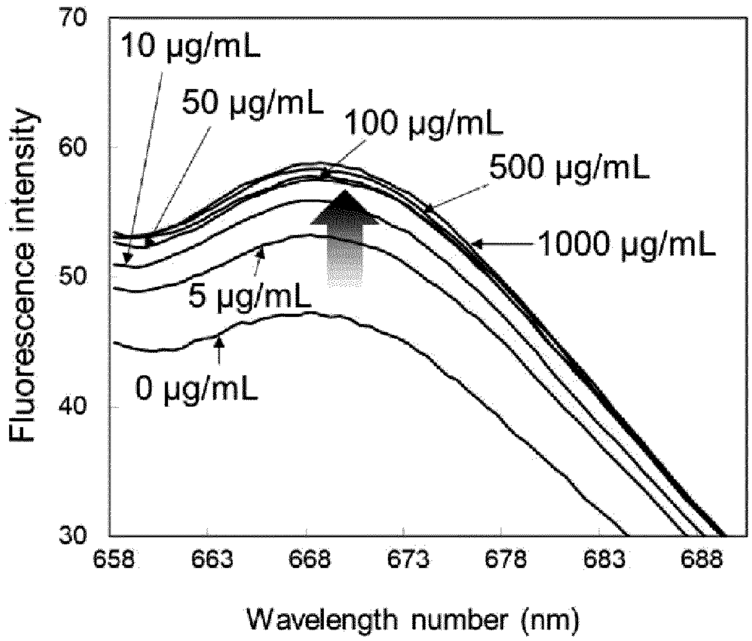
Fluorescence intensity
70
60
50
40
30
10 μg/mL
50 μg/mL
100 μg/mL
500 μg/mL
1000 μg/mL
5 μg/mL
0 μg/mL
658
663
668
673
678
683
688
Wavelength number (nm)

FIG.17

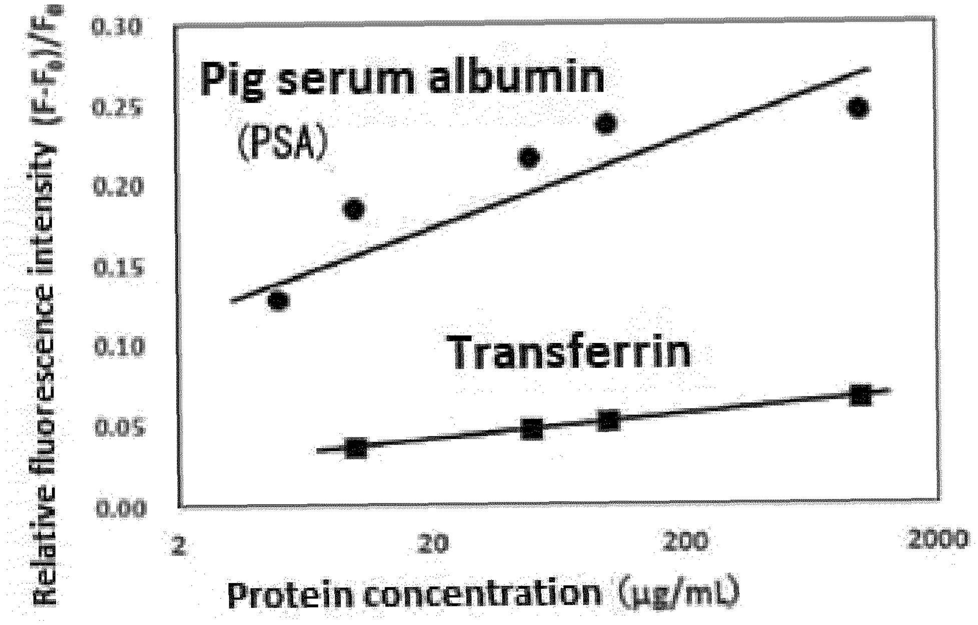
Pig serum albumin
(PSA)
Transferrin
Relative fluorescence intensity (F-F0)/F0
0.30
0.25
0.20
0.15
0.10
0.05
0.00
2
20
200
2000
Protein concentration (μg/mL)

FIG.18

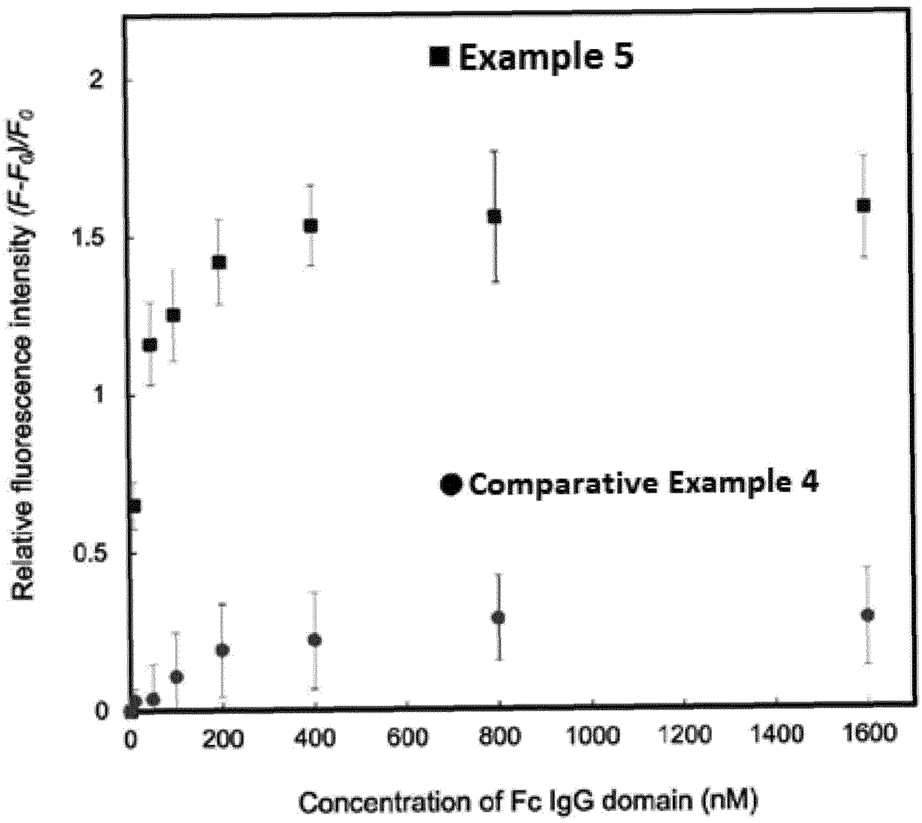
Example 5
Comparative Example 4
Relative fluorescence intensity (F-F0)/F0
2
1.5
1
0.5
0
0
200
400
600
800
1000
1200
1400
1600
Concentration of Fc IgG domain (nM)

FIG.19

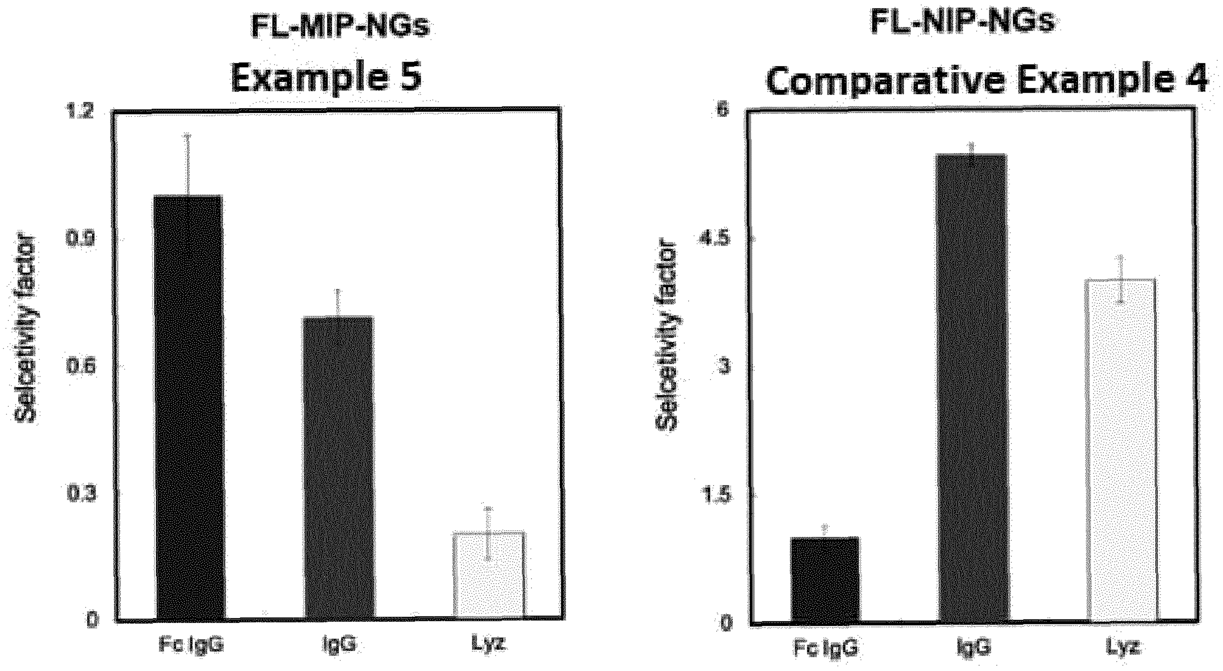
FL-MIP-NGs
Example 5
Selcetivity factor
1.2
0.9
0.6
0.3
0
Fc IgG
IgG
Lyz
FL-NIP-NGs
Comparative Example 4
Selcetivity factor
6
4.5
3
1.5
0
Fc IgG
IgG
Lyz

FIG.20

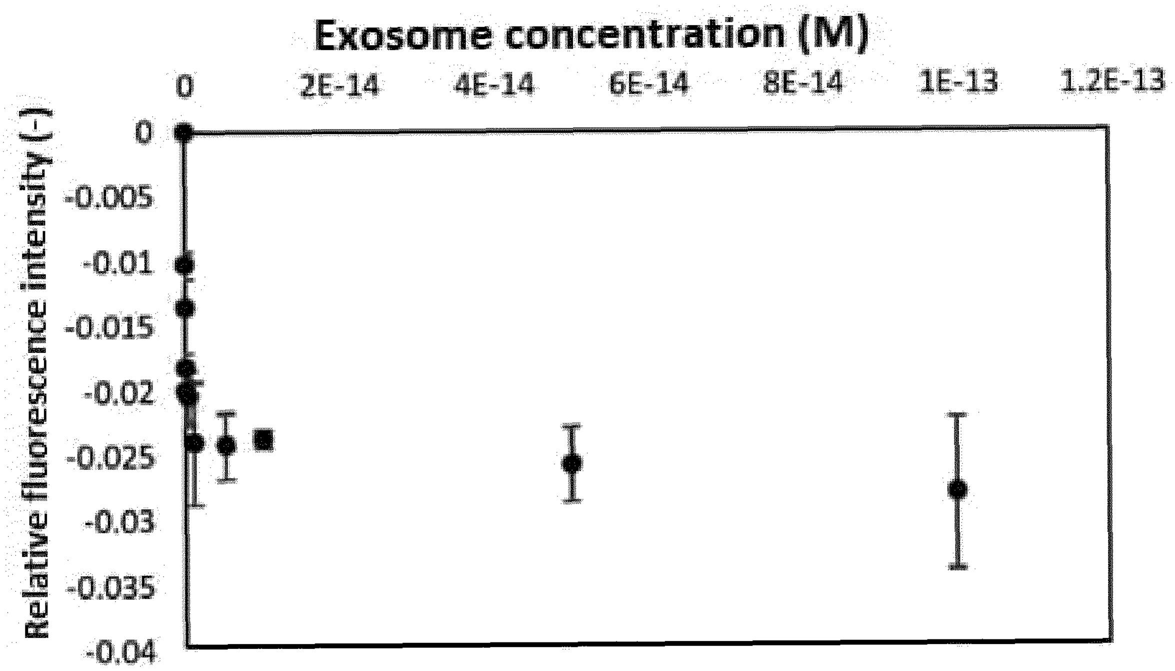
Exosome concentration (M)
0
2E-14
4E-14
6E-14
8E-14
1E-13
1.2E-13
Relative fluorescence intensity (-)
0
-0.005
-0.01
-0.015
-0.02
-0.025
-0.03
-0.035
-0.04

FIG.21

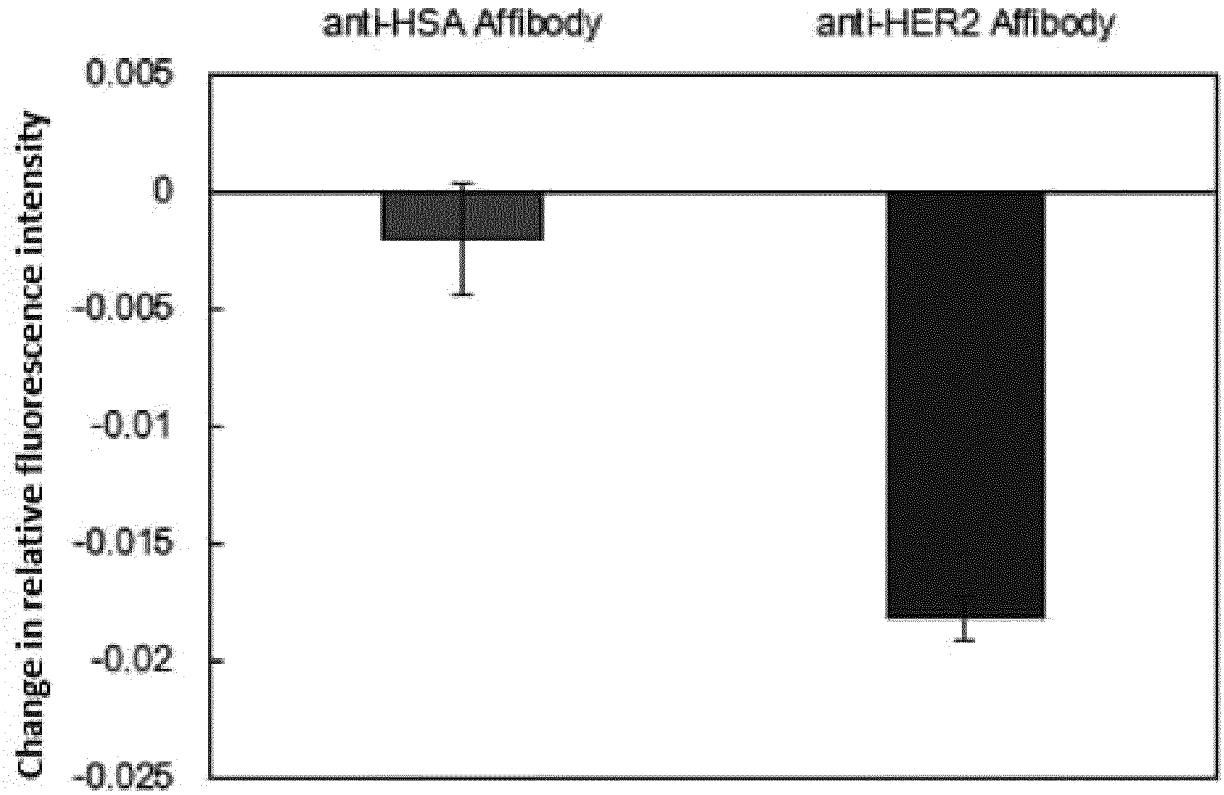
anti-HSA Affibody
anti-HER2 Affibody
Change in relative fluorescence intensity
0.005
0
-0.005
-0.01
-0.015
-0.02
-0.025

## INTERNATIONAL SEARCH REPORT

International application No.
PCT/JP2021/007726

A. CLASSIFICATION OF SUBJECT MATTER
G01N 33/53(2006.01)i; G01N 33/566(2006.01)i
FI: G01N33/566; G01N33/53 D

According to International Patent Classification (IPC) or to both national classification and IPC

B. FIELDS SEARCHED

Minimum documentation searched (classification system followed by classification symbols)
G01N33/53; G01N33/566

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2018-132527 A (SHIBAURA INSTITUTE OF TECHNOLOGY) 23 August 2018 (2018-08-23) entire text, all drawings, in particular, see claims, paragraphs [0004], [0021]-[0034], etc. | 1-3, 5-6, 8, 15-17<br>4, 7, 9-14 |
| Y | WO 2018/221271 A1 (KOBE UNIVERSITY) 06 December 2018 (2018-12-06) entire text, all drawings, in particular, see claims, paragraphs [0019]-[0032], [0044]-[0070], etc. | 4, 7, 9-14 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 April 2021 (30.04.2021) | 18 May 2021 (18.05.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/007726

C (Continuation). DOCUMENTS CONSIDERED TO BE RELEVANT

| Category* | Citation of document, with indication, where appropriate, of the relevant passages | Relevant to claim No. |
|---|---|---|
| Y | 松本 大樹、外 4 名，ポストインプリンティング修飾分子インプリントポリマーナノ薄膜による前立腺特異抗原センシング，2017，第 78 回応用物理学会秋季学術講演会［講演予稿集］ The 78th JSAF Autumn Meeting, 2017 [extended abstracts]，公益社団法人応用物理学会 The Japan Society of Applied Physics, see entire text, etc., non-official translation (MATSUMOTO, Hiroki et al., "Sensing of prostate-specific antigen with post-Imprinting modifications and nano-film of molecularly imprinted polymers") | 4, 7, 9-14 |
| Y | 松浦 亮、外 5 名，分子インプリントポリマー修飾プラズモニックチップによるヒト血清アルブミンの高感度プラズモニックセンシング "Highly Sensitive Plasmonic Sensing for Human Serum Albumin by Molecularly Imprinted Polymer-Coated Plasmonic Chips", <第 63 回>応用物理学会春季学術講演会講演予稿集, see entire text, etc., (MATSUURA, Akira et al., Lecture preprints of the 63rd JSAP Spring Meeting, 2016) | 9-14 |
| Y | JP 2016-194443 A (MORINAGA INSTITUTE OF BIOLOGICAL SCIENCE, INC.) 17 November 2016 (2016-11-17) entire text, in particular, see claims, paragraph [0092], etc. | 13 |
| A | JP 2017-150815 A (HITACHI, LTD.) 31 August 2017 (2017-08-31) see entire text, all drawings, etc. | 1-17 |

Form PCT/ISA/210 (continuation of second sheet) (January 2015)

**INTERNATIONAL SEARCH REPORT**
Information on patent family members

International application No.
PCT/JP2021/007726

| Patent Documents referred in the Report | Publication Date | Patent Family | Publication Date |
|---|---|---|---|
| JP 2018-132527 A | 23 Aug. 2018 | (Family: none) | |
| WO 2018/221271 A1 | 06 Dec. 2018 | US 2020/0110084 A1 entire text, all drawings, in particular, see claims, paragraphs [0073]-[0088], [0104]-[0143], etc. EP 3647786 A1 | |
| JP 2016-194443 A | 17 Nov. 2016 | (Family: none) | |
| JP 2017-150815 A | 31 Aug. 2017 | WO 2015/182296 A1 see entire text, all drawings, etc. | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Journal of Visualized Experiments,* 2018, vol. 132, e57208
- *Angewandte Chemie International Edition.,* 2016, vol. 55, 13023-13027